# EUROPEAN PATENT APPLICATION

(11) **EP 1 473 370 A2**
(43) Date of publication of application: **03.11.2004**
(21) Application number: 04252422.3
(22) Date of filing: 26.04.2004
(51) Int. Cl.: C12Q 1/68

(54) **Genus, group, species and/or strain specific 16S rDNA Sequences**

(30) Priority: 24.04.2003 US 464955 P
(71) Applicant: BioMerieux, Inc., Durham, NC 27712 (US)
(72) Inventor: Chatellier, Sonia, 01500 Amberieu (FR); Lacroix, Bruno, 69230 Genis-Laval (FR); Zeng, Qiandong, Belmont, MA 02478 (US); Childress, Darrell, Auburn Alabama 36831 (US); Moir, Donald T., Lexington, MA 02173 (US)
(74) Representative: Froud, Clive

(57) **Abstract**

Materials and methods for identifying unique sites in bacterial 16S and 23S rDNA are provided, as well as specific unique sequences of 16S rDNA in select bacteria. The distinguishing moieties will enable rapid differentiation between families, genera, groups, species, strains, subspecies, and isolates of microorganisms. Such differentiation can be performed by using rapid screening kits in combination with *in silico* analysis for diagnostic, prognastic, epidemiologic, phylogenetic, and other purposes.

## Description

**APPENDICES:** Sequence Listing is submitted in triplicate on CD-ROM and is herein incorporated by reference in its entirety. Five tables (14, 15, 19, 20, and 21) submitted on CD-ROM are also incorporated into the specification by reference in their entirety. The files bmx_2003_seq_list.txt, Table 14.txt, Table 15.txt, Table 19.txt, Table 20.txt, and Table 21.txt were saved on April 22, 2004, and are respectively 5138, 3150, 8006, 4097, 1345, and 3516 kilobytes.

### BACKGROUND OF THE INVENTION

Microorganisms are classically identified by their ability to utilize different substrates as a source of carbon and nitrogen through the use of biochemical tests such as the API20E™ system (bioMerieux). For susceptibility testing, clinical microbiology laboratories use methods including disk diffusion, agar dilution and broth microdilution. Although identifications based on biochemical testing and antibacterial susceptibility tests are cost-effective, generally two days are required to obtain preliminary results due to the necessity of two successive overnight incubations to identify the bacteria from clinical specimens as well as to determine their susceptibility to antimicrobial agents. There are some commercially available automated systems *(i.e.,* the MicroScan™ system from Dade Behring and the Vitek™ system from bioMérieux) which use sophisticated and expensive apparatus for faster microbial identification and susceptibility testing (Stager *et al.,* 1992, *Clin. Microbiol. Rev.* 5: 302-327). These systems require shorter incubations periods, thereby allowing most bacterial identifications and susceptibility testing to be performed in less than 6 hours. Nevertheless, these faster systems always require the primary isolation of the bacteria or fungi as a pure culture, a process which takes at least 18 hours for a pure culture or 2 days for a mixed culture.

Most clinical samples are in the form of blood or urine samples. The remaining samples are in the form of such biological fluids as sputum, pus, cerebrospinal fluid, synovial fluid and the like. The biochemical and susceptibility testing for a urine sample typically requires 18-24 hours of incubation and for blood upwards of 6 to 7 days.

Thus there exists an obvious need for rapid and accurate diagnostic tests for the detection and identification of pathogens. DNA tests are preferred because these tests can be performed more rapidly and accurately than the standard biochemical and susceptibility tests. Thus new DNA tests capable of discriminating between microorganisms are needed.

Bacterial ribosomes contain at least three distinct RNA molecules: 5S, 16S and 23S rRNAs. Historically, these names were chosen with reference to their sedimentation rate, which is reflective of the size of the molecule. However, the true size of the ribosomes from one organism to another varies substantially. Nevertheless, the terminology of 5S , 16S and 23S rRNA is used to describe the ribosomes of all bacteria.

A genetic comparison of the 16S subunits of various bacterial species has shown that there are highly conserved regions intercalated with regions of average and low homology, even in cases of related species. In fact, 16S RNA genes have been used for analyzing the evolutionary relationship between microorganisms. Research groups have used differentially hybridizing DNA probes in order to identify unknown microorganisms based on the hybridization patterns of ribosomal RNA. However, nucleic acid hybridization is an imprecise technique and is ill suited for distinguishing between closely related species and strains of organisms.

Methods of distinguishing between genera and strains for purposes of identification and classification differ and there is no set method. Classification at the genus or species level may be based on DNA/DNA hybridization, whereas identification of the subject organism may be based on a phenotypic character of the organism. Serological reactions, which have only limited value in classification, have enormous value for identification of a particular organism. Serological methods include slide agglutination tests, fluorescent antibody techniques and other serological methods. Although these methods can be performed simply and rapidly, their specificity is frequently not absolute and additional confirmation by physiological or biochemical tests is usually required.

Recently, an effort has been made to identify probes which will differentiate genera, groups, species and strains based on the genetic make-up of the organism. The ability to find probes that distinguish between related species and strains is further complicated by the fact that public databases, such as GenBank, possess accuracy and completeness problems. These problems arise at least because of DNA sequencing errors, and because many bacteria have two or more 16S ribosomal RNA loci in their genomes. Sequence variations may occur between the different copies of the gene present in the same genome, *i.e.* polymorphisms.

In view of these issues, a great need remains for methods and reagents which can be used to differentiate bacteria based on genus, species and strain for diagnostic, prognostic, environmental, agricultural, and research purposes. Herein are provided reagents and methods for systematically using such reagents to identify bacteria based on genus, species and strain.

### SUMMARY OF THE INVENTION

One aspect contemplates a plurality of 16S polynucleotides immobilized to a solid support, wherein the plurality of 16S polynucleotides are subsequences of 16S rDNA and each 16S polynucleotide individually comprises at least one distinguishing moiety, which differentiates between microorganisms by genus, group, species, strain and/or isolate. The polynucleotide is preferably an oligomer of about 11 to about 45 nucleotides, and more preferably between 15-30 nucleotides. The plurality can include 10-100 or 5 to 1 x 10⁶ or more polynucleotides, and any number inbetween.

Another aspect contemplates a method of detecting the presence of a microorganism and determining an isolate, a strain, a species, a group, or a genus of a microorganism in a sample suspected of containing the microorganism comprising the steps of: (A) selecting at least one primer pair to amplify at least a portion of a 16S rDNA of the sample; (B) amplifying the 16S rDNA of the sample with the at least one primer pair; (C) contacting the amplified rDNA with at least one isolated nucleic acid comprising at least one distinguishing moiety; (D) incubating the amplified rDNA and the isolated nucleic acid under hybridizing conditions which allow hybridization in a sequence-specific manner between the sample and the at least one isolated nucleic acid to form a hybridization product; (E) detecting presence of the hybridization product and thereby one or more distinguishing moieties of the microorganism; and (F) determining the isolate, strain, species, group, and/or genus of the microorganism by the presence of the one or more distinguishing moieties.

In yet another aspect, a kit is contemplated. The kit is for the detection and identification of at least one microorganism by genus, group, species, strain and/or isolate in a sample and comprises: (A) at least one primer pair for amplification of at least a portion of a 16S rRNA of the microorganism; (B) two or more nucleic acids comprising at least two critical residues of a 16S rDNA which distinguish the microorganism by genus, group, species, strain or isolate; (C) a hybridization buffer to allow sequence-specific hybridization between the probes and the nucleic acids present in the sample, or to allow sequence-specific hybridization between the probes and the nucleic acids of amplified products of the sample; and (D) a detection moiety.

Another embodiment is a composition comprising a plurality of probes of Table 14 and/or Table 15, (or Table 20 and/or Table 21) , wherein each probe comprises at least one distinguishing moiety and wherein the plurality of probes are immobilized on a substrate. The substrate can be a bead, plate, slide, microtube, in the form of an affinity column, and the like. Preferably, the plurality of probes comprises probes that are about 15 to about 45 and more preferably 20 to about 30 nucleotides in length.

Another aspect of the invention contemplates a method of diagnosing a subject and determining the microorganism causing an infection in the subject comprising the steps of: (A) obtaining a sample from the subject; (B) screening the sample for the microorganism using a kit described herein.

Yet a further aspect of the invention contemplates a method of identifying distinguishing moieties in a 16S bacterial rRNA or rDNA comprising the steps of: (A) obtaining a nucleotide sequence of a genetic locus shared by two or more different bacterial strains, species, or genera; (B) dividing the nucleotide sequence into a set of oligomers of length "n" which overlap by "x" nucleotides, wherein "x" is at least one nucleotide less than "n" and wherein said overlapping oligomers span the length of the sequence of the genetic locus; (C) comparing an oligomer using a comparative algorithm against at least one database of nucleotide sequences for that locus from a plurality of bacterial strains, species, or genera, wherein the nucleotide sequences are stored in at least one database; and (D) determining whether the oligomer has a nucleotide sequence which matches, or has no more than one mismatch with, a portion of all available nucleotide sequences for the locus of the strain, species, or genus of origin, or whether the nucleotide sequence has at least two mismatches when aligned with any other strain, species, or genus, wherein the at least two mismatches when aligned correspond to distinguishing moieties which differentiate between strain, species or genus.

This invention provides new methods for identifying genera, group, species, strain, and isolate specific markers based on bacterial rRNA and rDNA sequences. Preferred bacterial rRNA or rDNA is 16S rRNA or rDNA, however in all instances when discussing 16S rRNA or rDNA, unless otherwise noted, 23S rRNA or rDNA is also contemplated.

It is a further object of the invention to provide a method a method of identifying a distinguishing moiety in a 16S or a 23S bacterial rRNA or rDNA comprising:
(A) obtaining a nucleotide sequence of a genetic locus shared by two or more different bacterial strains, species, or genera; (B) computationally dividing the nucleotide sequence into a set of short oligomers of length "n" which overlap by "x" nucleotides, wherein "x" is at least one nucleotide less than "n" and wherein said overlapping short oligomers span the length of the sequence of the genetic locus; (C) analyzing the set of short oligomers using a comparative algorithm against at least one database of nucleotide sequences for that locus from a plurality of bacterial strains, species, or genera; and (D) identifying one or more short oligomers of the set of short oligomers having a nucleotide sequence which matches, or has no more than one mismatch with, a portion of all available nucleotide sequences for the locus of the strain, species, or genus of origin and having at least two mismatches when aligned with any other strain, species, or genus. The locus is preferably that of a bacterial 16S or 23S ribosomal locus.

In a preferred embodiment of the invention "n" is 30 nucleotides and "x" is 15 nucleotides or alternatively "n" is 20 nucleotides and "x" is 19 nucleotides. However "x" can be 9 to 19 nucleotides and "n" is 10 to 30 nucleotides.

The method further comprises analyzing the short oligomers by identifying short oligomers that require the largest number of nucleotide changes to match a different strain, species, or genus than that from which the oligomer derived. The mismatch is preferably one or more. The sequences thus identified can be genus, group, species, strain or isolate specific.

Another embodiment of the invention includes nucleic acids identified by the above method which are isolate, strain, species, group or genus specific. Some of these nucleic acids may be in the forms of probes of sufficient length to bind in a sequence-specific manner to a polynucleotide in a sample or to a polynucleotide amplified from a sample. Optimally such probes will comprise a detectable label and range from 15 to about 60 nucleotides or any number in between.

Another aspect of the invention comprises a method of detecting the presence of and determining the strain, species, group, or genus identity of a microorganism in a sample suspected of containing said microorganism. Such a method can comprise the following steps: (i) optionally selecting and using at least one primer pair to amplify at least a portion of a 16S rDNA of the sample; (ii) contacting the amplified or unamplified sample DNA with at least one of the isolated nucleic acid discussed above; (iii) incubating the amplified or unamplified sample DNA and the isolated nucleic acid under hybridizing conditions which allow hybridization in a sequence-specific manner between the sample and said at least one isolated nucleic acid to form a hybridization product; and (iv) detecting presence of the hybridization product as an indication of the identity of said microorganism. The microorganisms are preferentially from Table 1, but can be any human bacterial pathogen. Alternatively, the sample can be obtained from food or is a biological sample taken from a subject, an environmental sample, or a plant.

Another aspect of the invention contemplates a kit for the detection and identification of at least one microorganism from a set of microorganisms of Table 1 in a sample. The kit comprises comprising: (i) at least one primer pair for amplification of at least a portion of a 16S or a 23S rRNA of a microorganism of Table 1; (ii) a composition comprising at least one probe having at least one distinguishing moiety; (iii) a hybridization buffer to allow sequence-specific hybridization between the probes in said composition and the nucleic acid present in the sample or amplified products of the sample nucleic acid; and (iv) a detection moiety. In a preferred examble, the microorganisms are from Table 1.

A further aspect of the invention contemplates a method of using a probe of Tables 14, 15, 20, or 21 (all of which are attached to the specification as separate documents) comprising at least one 16S rRNA distinguishing moiety to detect the presence of a bacterium in a sample. The sample can be an environmental sample, food sample, or a biological sample obtained from an animal. Alternatively, the probes can be used in a method to determine a treatment protocol in a subject believed to suffer from a bacterial infection.

Yet another aspect of the invention includes a database comprising nucleic acid sequences with distinguishing moieties which distinguish a microorganism based on genus, species, or strain and wherein the database comprises at least one sequence from Tables 2, 14, 15, 20, or 21 (all of which are attached to the specification as separate documents). Another database contemplated is a reference database which comprises Table 14 and/or Table 15 or Table 20 and/or Table 21 (in a relational form with a means for querying said reference database).

In another embodiment of the invention, a computerized storage and retrieval system of biological information, comprising: a data entry means; a display means; a programmable central processing unit; and a data storage means having oligomers comprising 16S rRNA sequences with distinguishing moieties and annotated information on attributes of the rRNA sequences electronically stored in a relational database.

Another object of the invention provides for a computerized storage and retrieval system of biological information, comprising: a data entry means; a display means; a programmable central processing unit; and a data storage means having oligomers comprising 16S rRNA sequences with distinguishing moieties and annotated information on attributes of the rRNA sequences electronically stored in a relational database.

### BRIEF DESCRIPTION OF THE DRAWINGS

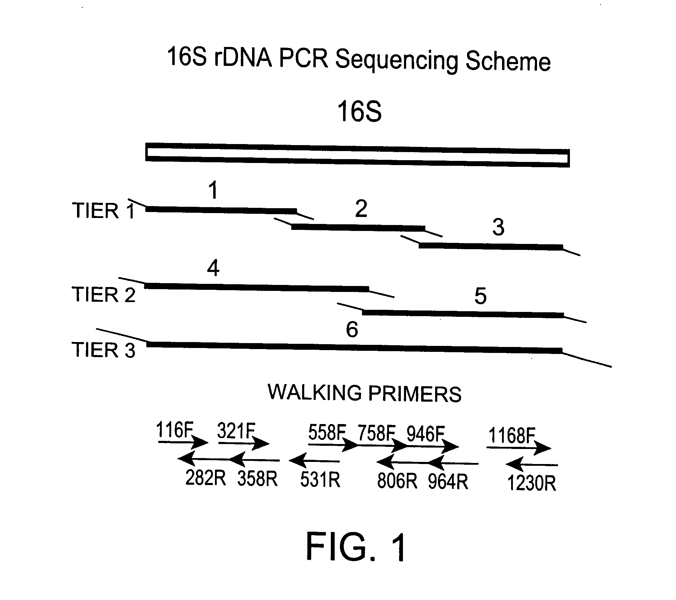

**FIG.1.** 16S PCR Sequencing Procedure. In order to determine the DNA sequence of the 16S rDNA locus of bacterial samples, a total of six PCR products were generated for use as sequencing templates. Three PCR products comprised a first set of minimally-tiled fragments that cover the entire length of 16S, and two PCR products (Tier 2, Nos. 4-5; see Tables 4-6) comprise a backup tier that also covers the full length of 16S. The third tier covers the entire 16S sequence with a single amplicon. Thirteen 16S specific sequencing primers (walking primers in Table 6) were generated and used on the full-length amplicon in addition to the M 13 based end reads. In the high throughput schema, all 6 PCR products were amplified from each bacterial genomic DNA sample, and were sequenced from both ends. These PCR primers were tailed with M13 primer binding sites to provide for robust sequencing of resulting products with standard M13 sequencing primers. Twelve of the thirteen walking primers, excluding 16-514F, were used to generate reads from the full-length amplicon at this time. Primer 16-514F was only used on assemblies remaining incomplete after initial sequencing attempts. All sequence reads were clipped to remove untemplated extraneous bases and assembled by polyphred (Univ. Washington) into a single assembly group for each sample. Sequences were only termed "final" and ready for release if they met the minimum length *(i.e.,* 1380 nt for 16S) and coverage requirements *(i.e.,* 3.5-fold average coverage with bases at ≥Phred 20 quality). In some cases, even more sequence reads will be required to fill all sequence gaps and to provide adequate coverage. Primers chosen to fill gaps and provide coverage for some 16S loci may not anneal or prime in other species due to unpredictable sequence variations. Thus, these primers may have to be re-designed as needed for specific samples based on the sequences obtained for those samples.

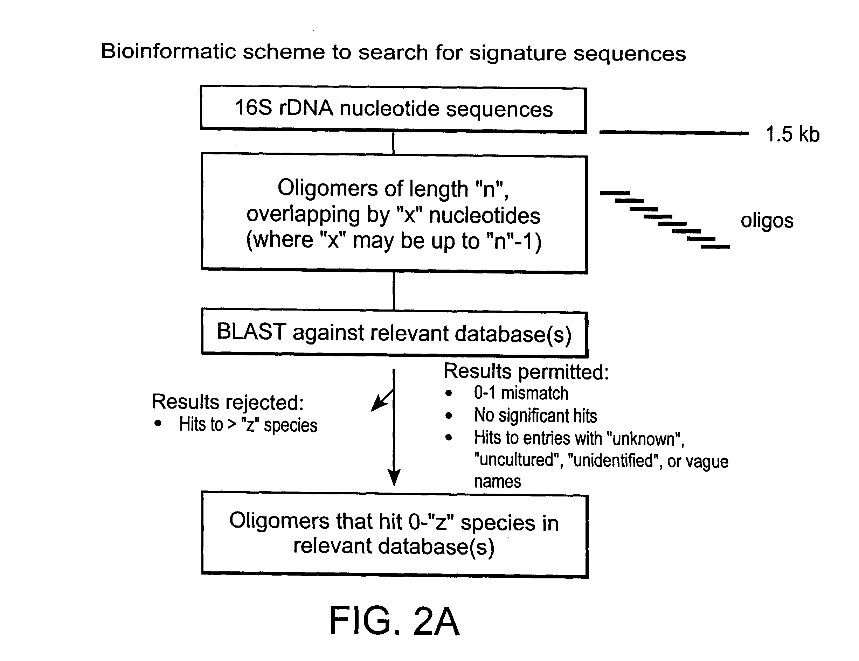

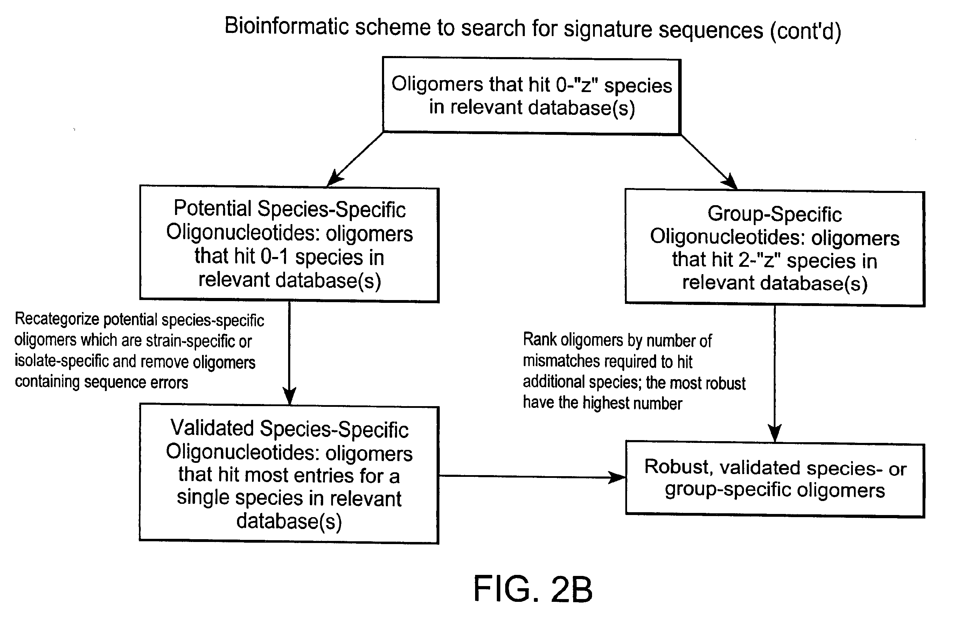

**FIG. 2.** Signature Sequence Analysis Model of 16S rDNA using overlapping oligonucleotides. The depicted embodiment uses 30-mers with 15 nucleotide (nt) overlaps. For a 1.5 kb region, one uses approximately 100 30-mer oligonucleotides. The 30-mers are then analyzed against a database using BLAST, *e.g.*, GenBank, RDP, or an internal database. Alternative lengths of oligomers are also contemplated herein.

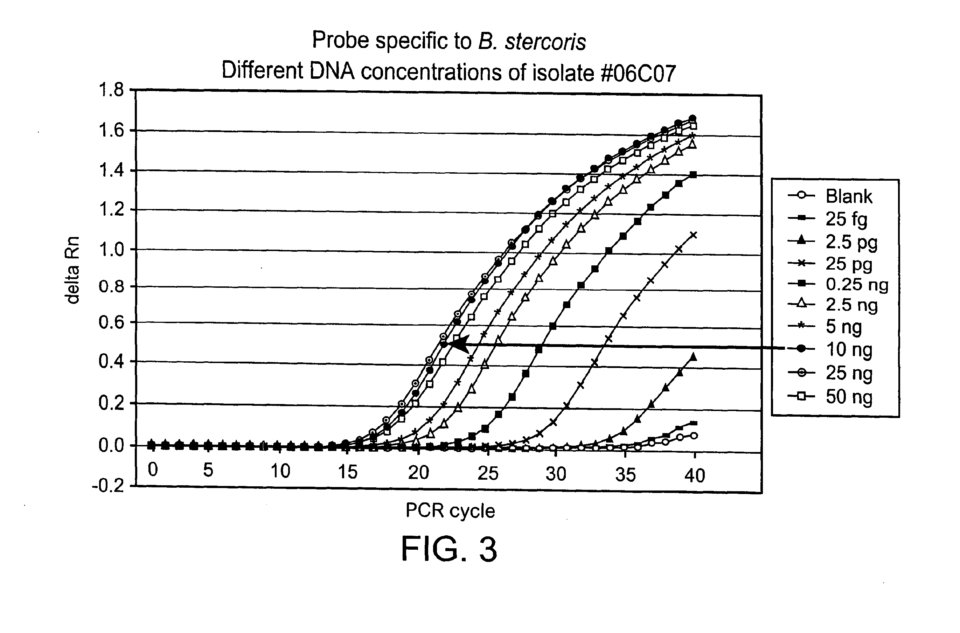

**FIG 3.** TaqMan® Assay Results at Different Template DNA Concentrations. The diagram shows the average of a triplicate of experiments for each point. Serial dilutions of genomic DNA from one *Bacteroides stercoris* isolate were used as templates for real-time PCR. Fig. 3 represents the relative fluorescence obtained for each PCR cycle and for each DNA concentration tested. The threshold cycle (Ct) is defined as the PCR cycle at which a fluorescence signal passes a preset value (threshold). The fastest Ct was obtained with the highest DNA amount used (50 ng). However, a concentration of 10 ng is a suitable minimum amount, since the Ct had a similar value (around 18) to the Ct obtained with DNA tested at 25 and 50 ng. Furthermore, the slope of the curves was also similar. In the studies described herein, all DNA samples were tested at 10 ng per reaction.

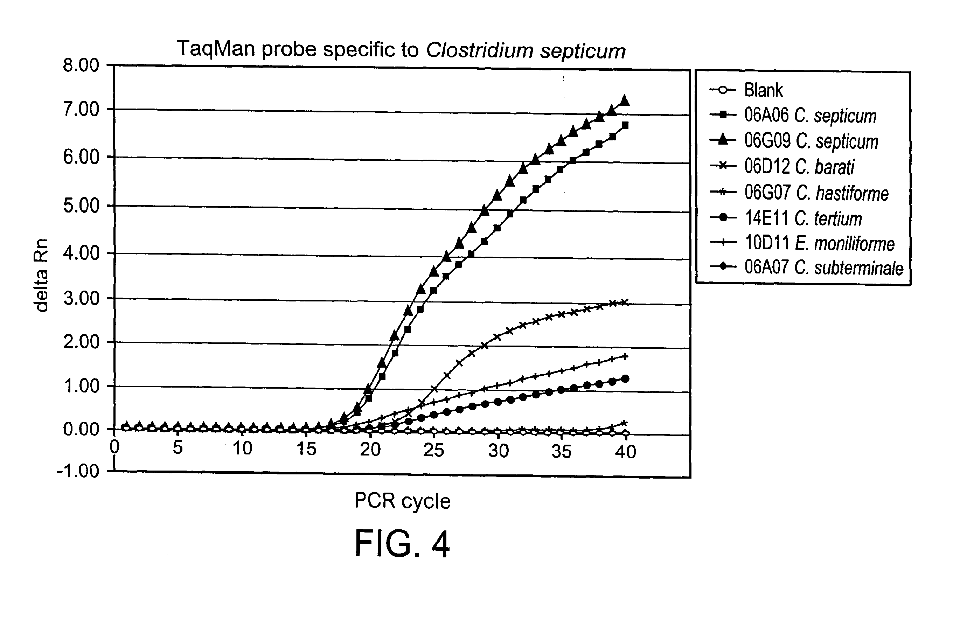

**FIG. 4.** TaqMan® Probe Specific to *C. septicum.* Fig. 4 illustrates results from a TaqMan® real-time PCR test of seven strains -- two strains identified as *C. septicum* by phenotypic testing and 5 strains identified as different, but related species. The TaqMan® test results showed that both of the strains phenotypically identified as *C. septicum* had a positive amplification signal (Ct value of about 15 cycles) clearly distinguishable from the response of the 5 other strains belonging to other species. Two strains belonging to 2 other species did not have an amplification signal and the 3 remaining samples that harbor a 16S rDNA sequence with only two mismatches with the probe sequence had a delayed response.

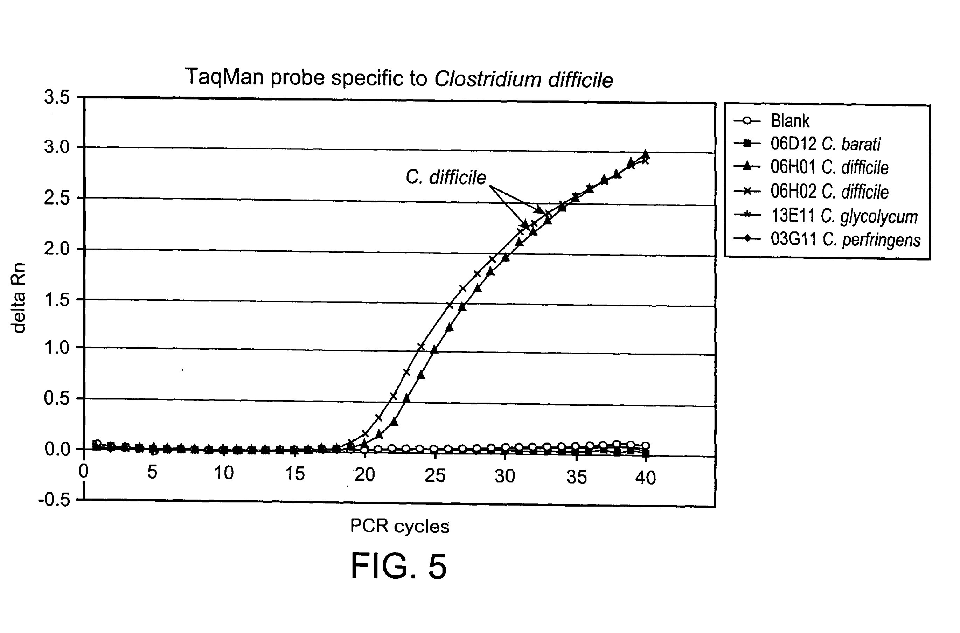

**FIG. 5.** TaqMan® Probe Specific to *Clostridium difficile.* Fig. 5 illustrates results from a TaqMan® real-time PCR test of five bacterial strains, two *C. difficile* strains and three strains representing other species from the *Clostridium* genus. The test clearly distinguished between the *C. difficile* strains (#06H01 and 06H02) and the non *C. difficile* isolates, since only the isolates belonging to *C. difficile* species had a positive amplification signal detected after 18 PCR cycles (CT value of about 18 cycles).

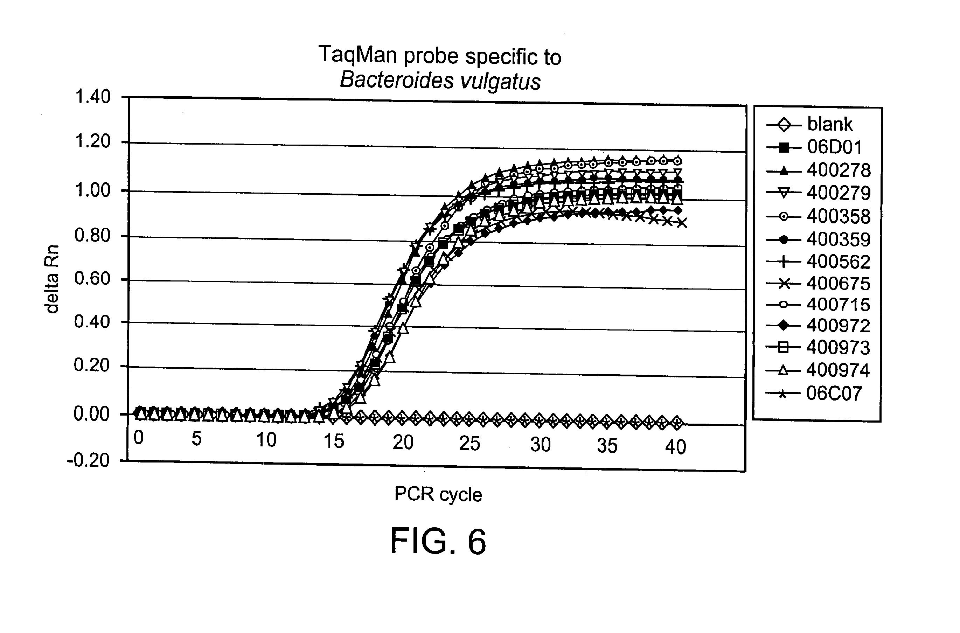

**FIG. 6.** TaqMan® Assay for *Bacteroides vulgatus.* Fig. 6 illustrates results from a TaqMan® real-time PCR test of 12 strains of *B. vulgatus.* All twelve *B. vulgatus* strains had similar Ct values of about 15 cycles, consistent with the fact that all twelve contain the probe SSO sequence within their 16S rDNA sequence.

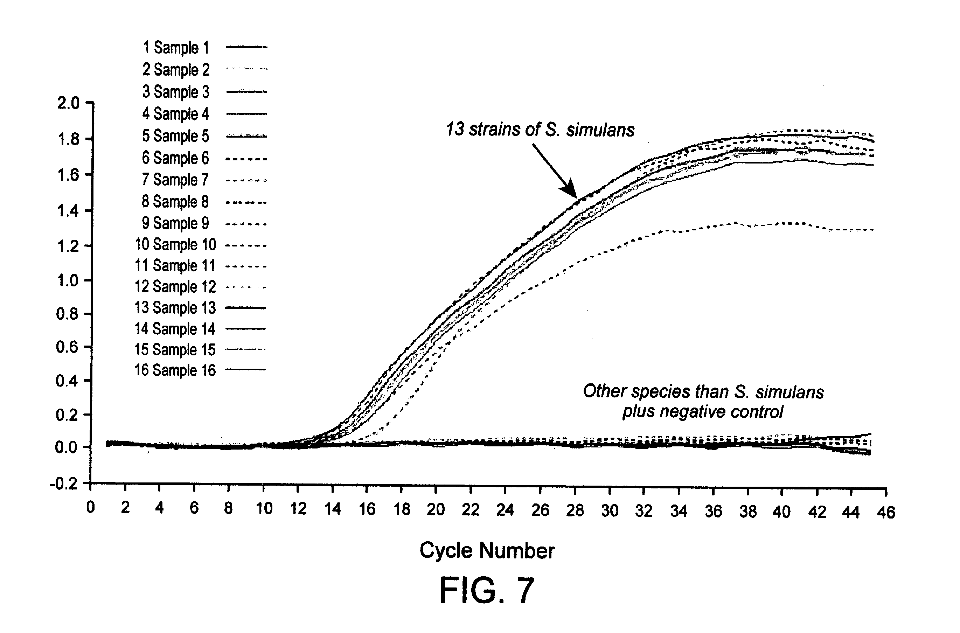

**FIG. 7.** Representative LightCycler assay for *Staphylococcus simulans.* Fig. 7 illustrates results from a LightCycler test of 15 strains (10 *Staphylococcus simulans,* 4 *Staphylococcus chromogenes,* 2 *Staphylococcus hyicus,* 2 *Staphylococcus schleiferi,* 2 *Staphylococcus intermedius,* 2 *Staphylococcus caprae,* and 1 *Staphylococcus cohnii)* and a negative control (sample 1). All thirteen strains of *Staphylococcus simulans* had a positive amplification signal represented by Ct values of about 16 cycles, consistent with the fact that all these strains harbor the probe sequences within their 16S rDNA sequence.

**FIG. 8.** Pyrosequencing assay for two *Enterococcus* species. Each peak represents the incorporation of the nucleotide mentioned on the X-axis during the sequencing reaction. The size of the peak determines the number of nucleotides incorporated. The software also gives a schematic representation of the pyrogram for each possibility of the single-nucleotide polymorphism also called SNP (C or T in this example). In this example, the strain of *Enterococcus faecalis* differs from that of *Enteroccocus durans* by a C (cytosine) instead of a T (thymidine) in the target sequence TG(C or T)AGGCGAGTTG.

### DETAILED DESCRIPTION OF THE INVENTION

The methods and reagents disclosed herein are for use in differentiating bacteria by genus, group, species and strain. Bacteria are described as single cells or simple associations of similar cells forming a group defined by cellular and not organismal properties. The nucleoplasm (genophore) of a bacterium is never separated from the cytoplasm by a unit-membrane system (nuclear membrane). The super kingdom of bacteria is classified in descending order by phylum, class, family, genus, group, species and strain.

There are four major identification categories of bacteria: (1) Gram-negative eubacteria that have cell walls, (2) Gram-positive eubacteria that have cell walls, (3) eubacteria lacking cell walls such as mycoplasmas, and (4) archaeobacteria. The preferred bacterial targets of the methods and reagents of this invention are 1 through 3.

Of the preferred categories, these can then be subdivided again into groups, any of which can be subjected to the methods and reagents derived therefrom using the methods of the application. For Category 1, the groups include (a) the spirochetes, (b) aerobic/microaerophilic, motile helical/vibroid gram-negative bacteria, (c) non-motile (or rarely motile), gram-negative curved bacteria, (d) gram-negative aerobic/microaerophilic rods and cocci, (e) facultatively anaerobic Gram-negative rods, (f) gram-negative anaerobic straight, curved and helical rods, (g) dissimilatory sulfate- or sulfur-reducing bacteria, (h) anaerobic gram-negative cocci, (i) the rickettsias and chlamydias, (j) anoxygenic phototrophic bacteria, (k) oxygenic phototrophic bacteria, (l) aerobic chemolithotrophic bacteria and associated organisms, (m) budding and/or appendaged bacteria, (n) sheathed bacteria, (o) nonphotosynthetic, non-fruiting gliding bacteria, and (p) fruiting gliding bacteria: the myxobacteria. Of these groups, the preferred groups that include, among others, bacteria of medical importance, are: (a), (b), (c), (d), (e), (f), and (h).

Category 2 can be divided into 6 groups as follows: (a) gram-positive cocci, (b) endospore-forming gram-positive rods and cocci, (c) regular, non-sporing gram-positive rods, (d) irregular, non-sporing gram-positive rods, (e) the mycobacteria, and (f) actinomycetes. Of these groups, the preferred groups that include, among others, bacteria of medical importance are (a), (b), (c), and (d). Category 3 consists of mycoplasmas. For a breakdown and further description of the groups and the members of the groups and descriptions of each, *see* Bergey's Manual of Determinative Bacteriology (9^{th} ed., John G. Holt et al., eds. Philadelphia, 1994). More preferred organisms are presented *infra.*

### 1. Definitions

The "sample" may be any biological material taken either directly from the infected human being (or animal), or after culturing (enrichment), a sample taken from food or feed, an environmental sample comprising a mixture of bacteria, or a plant sample. Biological material may be, *e.g.*, expectorations of any kind, broncheolavages, blood, skin tissue, biopsies, lymphocyte blood culture material, urine, fecal samples, sputum, and the like. Said samples may be prepared or extracted according to any of the techniques known in the art. Sample is also meant to include samples from food, environmental samples or plant samples.

By "subject" is meant to include any vertebrate or invertebrate capable of being infected by a bacterium. Preferred subjects include agricultural animals (*e.g.*, birds, pigs, cattle, sheep, goats, bison, horses and the like), and mammals (*e.g.*, dogs, cats, etc.) including primates (humans).

By "distinguishing moiety" is meant a nucleic acid(s) sequence difference in the sequence of mature 16S rRNA, or in the portion of rDNA which encodes it, that differentiates a bacterium's 16S rRNA or rDNA from another bacterium's 16S rRNA. The distinguishing moiety may be family or genus specific. More preferably the distinguishing moiety is group, species, subspecies or strain specific.

The probes of the invention preferably distinguish between genus, species and strains of bacteria. However, the probes may also be used to distinguish between families and classes of bacteria for classification purposes. By "family specific" is meant a characteristic, preferably at the nucleic acid level, which distinguishes families of bacteria based on their ribosomal DNA or RNA.

By "genus-specific" is meant to include a distinguishing characteristic of a genus which allows differentiation between genera of bacterium based on their ribosomal DNA or RNA. It would be understood that such a genus-specific moiety would also identify the family of a bacterium of that particular genus. Such genus specific moieties may or may not be capable of identifying all members in that family.

By "group specific" is meant an oligonucleotide sequence which is specific towards two or more members of a species. "Group specific" may include species from different genera. By "microorganism group" is meant at least two members of a genus *(e.g., Bacteroides)* or even members of at least two different genera *(e.g., Bacteroides* and *Prevotella),* provided that the species involved are clinically and therapeutically similar. For example, probes, which identify members of a group consisting of *Streptococcus pneumoniae, Haemophilus influenzae,* and *Moraxella catarrhalis,* would be useful, because these members of the group all exhibit a similar clinical outcome (namely, pneumonia), which is treated similarly. Thus, knowledge that a bacterium is a member of this group would provide clinicians with useful information. Probes, which identify a microorganism group, will typically hybridize to rRNA or rDNA of a plurality of microorganism species tested.

By "species-specific" is meant to include a distinguishing characteristic of a genus, which allows differentiation between species of bacterium based on their ribosomal DNA or RNA *(e.g., Cedecea davisaes, Cedecea lapagei* and *Cedecea neteri).* It would be understood that such a species-specific moiety would also identify the genus of a bacterium of that particular species, but may not necessarily be capable of identifying all members of that genus.

By "strain specific" is meant to include distinguishing characteristics of a bacterium which allows differentiation between strains of bacterium. It would be understood that such a strain-specific moieties would also identify the species of that particular strain, but may not necessarily be capable of identifying all members of that species.

By "rRNA" and "ribosomal RNA" is meant the structural RNA components of the ribosome. Prokaryotes such as bacteria have 5S rRNA and 23S rRNA species in the large subunit and a 16S rRNA species in the small subunit. The large subunit in, for example, *E. coli* is known to sediment at 50S due to the presence of 23S rRNA, 5S rRNA and 31 proteins. The small subunit, in *E. coli,* is 30S due to the presence of the 16S rRNA and 21 ribosomal proteins. In *E. coli* the 16S rRNA is 1,542 nucleotides. This rRNA is synthesized like mRNA using a RNA polymerase that uses DNA as a template.

By "rDNA" is meant typically to refer to the DNA coding for ribosomal RNA. However, in certain instances "rDNA" may refer to recombinant DNA.

By "r-proteins" and "ribosomal proteins" is meant the proteins that associate with the large and small ribosomal subunits.

As used throughout the application and claims, the term "probe" will refer to synthetic or biologically produced nucleic acids, between 10 and 250 bases in length, which by design or selection, contain specific nucleotide sequences that allow specific and preferential hybridization under predetermined conditions to target nucleic acid sequences, and optionally contain a moiety for detection or for enhancing assay performance. A minimum of ten nucleotides is generally necessary in order to statistically obtain specificity and form stable hybridization products, and a maximum of 250 nucleotides generally represents an upper limit for sequences in which reaction parameters can be adjusted to determine mismatched sequences and preferential hybridization. Therefore, in general, a preferred length of a probe will be between 10 and 250 nucleotides. A more preferred length of probe is between 15 and 60 oligonucleotides (*i.e.*, 15-mers and 60-mers and any range in between). Probes may optionally contain certain constituents that pertain to their proper or optimal functioning under certain assay conditions. For example, probes may be modified to improve their resistance to nuclease degradation (*e.g.*, such as by end-capping), to carry detection ligands (*e.g.*, such as fluorescein, ³²P, biotin, and like labeling) or to facilitate their capture onto a solid support (*e.g.*, poly-deoxyadenosine "tails").

"Preferential hybridization" or "hybridizing preferentially" means that hybridization with the intended target nucleic acid results in a hybridization reaction product which is more stable than any hybridization reaction products resulting from hybridization with a non-target nucleic acid under identical conditions. It is well within the skill of the ordinary artisan to compare stability of hybridization reaction products and evaluate which one is more stable, *i.e.*, determine which one has bound "preferentially". By "specific hybridization" is meant that a nucleotide sequence will hybridize to a predetermined target sequence and will not substantially hybridize to a non-target sequence.

"Specifically discriminate" means that a probe will substantially hybridize to a predetermined target sequence and will not substantially hybridize to a non-target sequence.

"Hybridization" is a process by which, under predetermined reaction conditions, two partially or completely complementary strands of nucleic acids are allowed to come together in an antiparallel fashion to form a double stranded nucleic acid with specific and stable hydrogen bonds, following explicit rules pertaining to which nucleic acids bases may pair with one another. "Substantial hybridization" means that the amount of hybridization observed will be such that one observing the results would consider the result positive in a clinical setting. Data which is considered "background noise" is not substantial hybridization.

"Stringent hybridization conditions" means approximately 35°C to 65°C in a salt solution of approximately 0.9 M NaCl. Stringency may also be governed by such reaction parameters as the concentration and type of ionic species present in the hybridization solution, the types and concentrations of denaturing agents present, and the temperature of hybridization. Generally as hybridization conditions become more stringent, probes of greater length and/or containing fewer mismatched nucleotides are preferred if stable hybrids are to be formed. As a rule, the stringency of the conditions under which a hybridization is to take place will dictate certain characteristics of the preferred probes to be employed. Such relationships are well understood and can be readily manipulated by those skilled in the art.

As used herein, the terms "homology" and "homologous to are meant to refer to the degree of similarity between two or more nucleic acid sequences, and is not meant to imply any taxonomic relatedness between organisms. The degree of similarity is expressed as a percentage, *i.e.,* 90% homology between two sequences will mean that 90% of the bases of the first sequence are identically matched to the bases of the second sequence.

"Target" or "target molecule" as used herein in the diagnostic sense, refers to a molecule of interest, *i.e.*, the molecule whose presence one wishes to determine. In a therapeutic sense, the term "target" or "target molecule" refers to a molecule associated with a disease or with an organism causing a disease.

"Biological binding pair" as used herein refers to any pair of molecules, which exhibit mutual affinity or binding capacity. A biological binding pair is capable of forming a complex under binding conditions. For the purposes of the present application, the term "ligand" will refer to one molecule of the biological binding pair; and the terms "antiligand" or "receptor" will refer to the opposite molecule of the biological binding pair. For example, without limitation, embodiments of the present invention have application in nucleic acid hybridization assays where the biological binding pair includes two complementary nucleic acids. One of the nucleic acids is designated the ligand and the other nucleic acid is designated the antiligand or receptor. One of the nucleic acids may also be a target molecule. The designation of ligand or antiligand is a matter of arbitrary convenience. The biological binding pair may include antigens and antibodies, drugs and drug receptor sites, and enzymes and enzyme substrates, to name a few.

The term "label" refers to a chemical moiety which is capable of detection including, by way of example, without limitation, radioactive isotopes (*e.g.*, ³²P), enzymes *(e.g.,* horseradish peroxidase), luminescent agents (*e.g.*, luciferase), precipitating agents, and dyes (*e.g.* , FAM which is derived from fluoresceine and TAMRA which is derived from rhodamine). The term "agent" is used in a broad sense, including any chemical moiety, which participates in reactions that lead to a detectable response. The term "cofactor" is used broadly to include any chemical moiety, which participates in reactions with the label.

The term "amplify" is used in the broad sense to mean creating an amplification product, which may include by way of example, additional target molecules, or target-like molecules, capable of functioning in a manner like the target molecule, or a molecule subject to detection steps in place of the target molecule, which molecules are created by virtue of the presence of the target molecule in the sample. In the situation where the target is a polynucleotide, additional target, or target-like molecules, or molecules subject to detection can be made enzymatically with DNA or RNA polymerase.

The term "support" when used alone, includes conventional supports such as filters, dipsticks, microarrays, beads and membranes as well as retrievable supports.

### 2. Method of Identifying Group, Genus, Species and Strain Specific Polymorphisms

*2.1* *In Silico* Methods. A systematic method is described herein for identifying regions of a 16S sequence, for any bacterial organism, as being specific to particular genera or species, and in some instance, specific to a particular strain or isolate. As illustrated in Figs. 2A and 2B, this method involves dividing each of one or more full-length 16S rRNA gene sequences into a number of nucleotide fragments (*i.e.*, oligomers). Each full-length sequence comprises, for example, 1500 nucleotides, where each nucleotide fragment has a predefined length of "n" nucleotides (*e.g.*, between 10 and 30 nucleotides) and a nucleotide overlap with an adjacent fragment of "x" nucleotides, where "x" is greater than or equal to zero and less than or equal to n-1 (see *e.g.*, Fig. 1).

As illustrated in Fig. 2A, each nucleotide fragment is then compared using an algorithm, such as BLAST, against other 16S sequences of length "n" stored in one or more databases, possibly including any one of a number of publically accessible databases, such as GenBank, the Ribosomal Database Project database or other like databases. The algorithm ultimately generates one or more reports which contain data reflecting the results of each comparison. The resultant data is then processed using additional algorithms to identify those oligomers exhibiting one or fewer (0-1) nucleotide mismatches when compared to nucleotide fragments associated with zero through Z (0-z) number of species. Oligomers exhibiting 0-1 nucleotide mismatches when compared to nucleotide sequences associated with greater than z number of species may be ignored for purposes of this exemplary method.

Oligomers exhibiting 0-1 nucleotide mismatches when compared to nucleotide sequences associated with 0-z number of species are categorized as either species-specific oligomers or group-specific oligomers, as shown in Fig. 2B. Species-specific oligomers are those exhibiting 0-1 nucleotide mismatches when aligned with 0-1 species, whereas group-specific oligomers are those exhibiting 0-1 nucleotide mismatches when aligned with 2-z number of species. Species-specific oligomers may be further validated by recategorizing certain ones of these oligomers as being strain or isolate specific, and by removing certain ones that containing sequence errors. In turn, the group-specific oligomers may be ranked in order of robustness, as indicated in Fig. 2B (see *e.g.*, Tables 14, 19, and 20).

More specific examples of the aforementioned method are described below (see Example 2 and Example 3). Example 2 specifically provides an example where n equals 30 and x equals 15. Example 3 specifically provides an example where n equals 20 and x equals 19.

2.2 Experimental verification. Experimental verification can then be performed using, for example, real time PCR (TaqMan®), spot format assays *(e.g.,* a single hybridization test on a membrane) or low-density DNA microarray format *(e.g.,* multiple probes spotted on a chip). The TaqMan® technique was developed by Perkin-Elmer Applied-Biosystems and relies on fluorescence resonance energy transfer (FRET). It requires the use of two primers and a fluorescent probe. The TaqMan® probes possess a fluorescent reporter at the 5' end and a fluorescent quencher at the 3' end. When irradiated, the excited fluorescent dye transfers energy to the nearby quenching dye molecule rather than fluorescing, resulting in a non-fluorescent substrate. When the probe hybridizes to its complementary sequence on the target DNA, the *Taq* DNA polymerase starts to digest the probe separating the reporter and the quencher so that no FRET occurs and the fluorescent signal of the reporter can be detected and measured. The more PCR product formed, the higher the fluorescent signal observed.

To accurately test the specificity of a TaqMan® probe, the PCR primers used in the TaqMan® assay must represent sequences that are present in all species to be tested. In order to verify the presence of conserved sequences upstream and downstream from the 30-mer oligonucleotide sequence from which to select PCR primers, each 30-mer was extended by 60 bases on each side to generate a 150-mer oligonucleotide. A multiple sequence alignment using CLUSTALW was generated using the particular 150-mer oligonucleotide to be tested and the 20-40 closest sequences in the selected database(s). CLUSTALW is the more recent version of CLUSTAL, with the W standing for weighting to represent the ability of the program to provide weights to the sequence and program parameters (Higgins et al., 1996, *Meth. Enz.* 266: 383-402). Another program that can be used is for multiple sequence alignment is PILEUP, and the like.

### 3. Organisms

The methods described *infra* can be utilized with any bacteria, such as those discussed in John G. Holt et al., Bergey's Manual of Determinative Bacteriology (Philadelphia, PA, 9^{th} ed., 1994). The approximately 600 species and about 80 genera, whose rDNA sequences were analyzed to discover the markers of this invention, are provided in Table 7. Preferred genera include *Actinobacillus* spp., *Actinomyces* spp., *Bacteroides* spp., *Campylobacter* spp., *Cardiobacterium* spp., *Clostridium* spp., *Eikenella* spp., *Enterobacter* spp., *Enterococcus* spp., *Escherichia* spp., *Fusobacterium* spp., *Haemophilus* spp., *Kingella* spp., *Klebsiella* spp., *Moraxella* spp., *Neisseria* spp., *Oligella* spp., *Prevotella* spp., *Propionibacterium* spp., *Pseudomonas* spp., *Staphylococcus* spp., *Streptococcus* and *Wolinella* spp. Any of the species within these individual groups of species can be analyzed using the methods, reagents, and the like described herein. Preferred species, include the following:

**TABLE 1:**

| Most common species found in blood cultures, plus anaerobesand fastidious species | |
|---|---|
| *Actinobacillus* spp. | *Kingella* spp. |
| *A. actinomycetemcomitans* | *K. denitrificans* |
| *A. ureae* | *K. kingae* |

| *Actinomyces* spp. | *Klebsiella* spp. |
|---|---|
| *A. bovis* | *K. pneumoniae* |
| *A. israelii* | *Moraxella* spp. |
| *A. meyeri* | *M. catarrhalis* |
| *A. naeslundii* | *M. lacunata* |
| | *M. osloensis* |
| | *M. nonliquefaciens* |

| *Bacteroides* spp. | *Neisseria* spp. |
|---|---|
| *B. fragilis* | *N. gonorrhoeae* |
| *B. thetaiotaomicron* | *N. meningitidis* |
| *B. vulgatus* | *Oligella* spp. |

| *Campylobacter* spp. | *O. urethralis* |
|---|---|
| *C. fetus* | *Pantoea* spp. |
| *C. jejuni* | P. agglomerans |

| *Cardiobacterium* spp. | P. dispersa |
|---|---|
| *C. hominis* | *Prevotella* spp. |
| | *P. bivia* |

| *Cedecea* spp. | *Propionibacterium* spp. |
|---|---|
| *C. davisae* | *P. acnes* |
| *C. lapagei* | |
| *C. neteri* | *Proteus* spp. |

| *Clostridium* spp. | *P. mirabilis* |
|---|---|
| *C. botulinum* | *P. myxofaciens* |
| *C. difficile* | *P. penneri* |
| *C. perfringens* | *P. vulgaris* |
| *C. septicum* | *Providencia* spp. |
| *C. sordellii* | *P. alcalifaciens* |
| *C. tetani* | *P. heimbachae* |

| *Eikenella* spp. | *P. rettgeri* |
|---|---|
| *E. corrodens* | *P. rustigianii* |
| | *P. stuartii* |

| *Enterobacter* spp. | *Pseudomonas* spp. |
|---|---|
| *E. cloacae* | *P. aeruginosa* |

| *Enterococcus* spp. | *Staphylococcus* spp. |
|---|---|
| *E. faecalis* | *S. aureus* |

| *Escherichia* spp. | *S. haemolyticus* |
|---|---|
| *E. coli* | *S. epidermidis* |
| | *S. saprophyticus* |

| *Fusobacterium* spp. | *Streptococcus* spp. |
|---|---|
| *F. moriferum* | *S. bovis* |
| *F. necrogenes* | *S. intermedius* |
| *F. necrophorum* | *Wolinella* spp. |
| *F. varium* | *W. curva* |

| *Haemophilus* spp. | *W. recta* |
|---|---|
| *H. aegyptius* | *W. succinogenes* |
| *H. aphrophilus* | *Yokinella* spp. |
| *H. ducreyi* | *Y. regensburgei* |
| *H. haemolyticus* | |
| *H. influenzae* | |
| *H. parahaemolyticus* | |
| *H. parainfluenzae* | |

*Actinomyces* species occur mainly in the oral cavity and on mucous membranes of warm-blooded vertebrates. *Actinomyces* spp. commonly cause pyogenic infections in association with other concomitant bacteria. Differentiation of the genus *Actinomyces* is difficult because of variable test reactions and because some species are best differentiated by protein gel electrophoresis, which requires culture and thus slows analysis.

*Bacteroides* are isolated from a wide range of anaerobic habitats including gingival crevices, the intestinal tract, sewage sludge and infective and purulent conditions in humans and animals.

*Campylobacter* is found in the reproductive organs, intestinal tract and oral cavity of humans and animals.

*Cardiobacterium* are associated with endocarditis in humans.

*Cedecea* were first thought to be intermediate between typical *Serratia* species and *S. fonticola.* They also are similar to *Ewingella* and thus can be hard to distinguish by traditional phenotypic methods.

*Clostridum* species are widespread in the environment. Many species produce potent endotoxins and some are pathogenic for animals, because of either wound infections or the absorption of toxins. There are over 100 species in this genus and differentiation between the species needs to be carried out only with well trained personnel because of the exacting growth conditions and test procedures required for distinguishing the species.

*Eikenella* can be opportunistic pathogens that cause infections in the human mouth and intestine.

*Enterobacter* species are widely distributed in nature, occurring in fresh water, soil, sewage, plants, vegetables and animal and human feces. Several species, most notably *E. cloacae, E. sakazakii, E. aerogenes, E. agglomerans, and E. gergoviae* are opportunistic pathogens causing urinary tract infections and occasionally septicemia and meningitis.

*Enterococcus* species occur widely in the environment, particularly in feces of vertebrates. *Enterococcus* species are also sometimes the cause of pyogenic infections.

Although *Escherichia* species are part of the normal intestinal flora of warm-blooded animals and in the case of *E. blattae,* of cockroaches, *Escherichia* are also responsible for diarrheal disease, and are major contributors to urinary tract infections and nosocomial infections, including septicemia and meningitis.

Fusobacterium is found principally in the gingival sulcus and in the intestinal and genital tracts. Species have also been isolated from blood cultures and various purulent lesions in humans and animals and from tropical ulcers. Because the typical fusiform or spindle-shaped appearance of these organisms is not shown by all fusobacteria, it causes difficulty in distinguishing some species from strains of *Bacteroides, Clostridium* and *Eubacterium* where the cells are easily decolorized. Differentiation of the species belonging to the *Fusobacterium* genus is also difficult because of the general lack of reactivity in conventional tests. This is especially true for subspecies of *F. nucleatum:* subsp. *nucleatum,* subsp. *polymorphym* and subsp. *vincentii.*

The genus *Haemophilus* includes *H. influenzae,* which is the leading cause of meningitis in children. It also is known to cause septicemic conditions, otitis media, sinusitis, and chronic bronchitis. *H. influenzae biovar aegyptius* is mainly responsible for conjunctivitis, a highly transmissible eye infection. *H. ducreyi* is the causative agent of the venereal disease soft chancre or chancroid.

*Kingella* species are Gram-negative cells, but because they have tendency to resist decolorization, they are difficult to characterize clinically. These organisms occur in human mucous membranes of the upper respiratory tract and are known to be susceptible to penicillin. Consequently, rapid identification of *Kingella* spp. would allow clinicians to prescribe drugs such as penicillin over more pharmacologically toxic antibiotics.

*Klebsiella* species can be found in human feces, clinical specimens, soil, water, grain, fruits and vegetables. *K. pneumoniae* and *K. oxytoca* are opportunistic pathogens that can cause bacteremia, pneumonia and urinary tract and other human infections. *Klebsiella* species are also known for causing nosocomial infections in urological, neonatal, intensive care and geriatric patients.

Species of *Moraxella* are Gram-negative, but as with other microorganisms often have a tendency to resist decolorization of the dye, thus making a clinical diagnosis difficult. Diagnosis of a *Moraxella* spp. infection is useful, as they are usually highly sensitive to penicillin. These organisms are responsible for parasitic infections of mucous membranes of humans and other warm-blooded animals.

Species of *Neisseria* are also Gram-negative, and also have a tendency to resist decolorization of the dye, thus making a clinical diagnosis difficult. These organisms typically are inhabitants of the mucous membranes of mammals. Some species are primary pathogens for humans.

The *Oligella* genus was not created until 1987 (Rossaue *et al.,* 1987, *Int. J. Syst. Bacteriol.* 37: 198-210) and contains two species: *O*. *urethralis* and *O*. *ureolytica.* These bacterium are isolated mainly from the genitourinary tract of humans.

*Pantoea* is a genus that was not created until 1989 by Gavini *et al. (Int. J. Syst. Bacteriol.* 39: 337-45). It included two species at that time, *Pantoea agglomerans* (also known as *Enterobacter agglomerans, Erwinia herbicola,* and *Erwinia milletiae)* and *Pantoea dispersa.* Since its creation, the *Pantoa* genus has grown to include at least seven species. The species of *Pantoea* are isolated from plant surfaces, seeds, soil, and water, as well as from animals and human wounds, blood and urine. These microorganisms are also considered opportunistic human pathogens.

The genus *Prevotella* was created in 1990 by Shah and Collins *(Int. J. Syst. Bacteriol.* 40: 205-208) with the species *P. melaninogenica* (formerly *Bacteroides melaninogenicus).* At least fifteen other species have been added to the *Prevotella* genus from the genus *Bacteroides.* Consequently, discrimination between at least these two genera is difficult. Additional classification based on species is required and would be aid by species specific probes.

*Propionibacterium* species are found mainly in cheese and dairy food products and on the human skin. *Propionibacterium* are readily confused with some species of *Cornebacterium* or *Clostridium.*

Proteus is a Gram-negative genus of organisms that occur in intestines of humans and a wide variety of animals. Species are also found in manure, soil and polluted waters. *P. myxofaciens* has been isolated from gypsy moth larvae. Species, which are human pathogens, cause urinary tract infections. Certain species also act as secondary invaders causing septic lesions often in burn patients. The species, *Proteus penneri,* was first created in 1982 by Hickman *et al., J. Clin. Microbiol.* 15: 1097-1102 and *Int. J. Syst. Bacteriol.* 33: 438-440.

The genus, Providencia, consists of microorganisms that have been isolated from human diarrhetic stools, urinary tract infections, wounds, burns and bacteremias, and from penguins. It is thus considered to include human pathogen species. The species *Providencia heimbachae* was created in 1986 by Muller *et al., (Int. J. Syst. Bacteriol.* 36: 252-6) and *Providencia rustigianii* (formerly known as biogroup 3 of *Providencia alcalifaciens)* was created in 1983 by Hickman-Brenner *et al., (J. Clin. Microbiol.* 17: 1057-60; and *Int. J. Syst. Bacteriol.* 33: 672-4).

*Pseudomonas* spp. are widely distributed in nature. Some species are pathogenic for humans, animals or plants.

*Staphylococcus* species are mainly associated with the skin and mucous membranes of warm-blooded vertebrates but are often isolated from food products, dust and water. Some species are opportunistic pathogens in humans and animals or are known to produce extracellular toxins. Consequently, subspecies and strain determination is important for clinical diagnosis, prognosis and treatment of *Staphylococcus* infections.

*Streptococcus* is a complex genus. The genus in Bergey's Manual of Systematic Bacteriology included microorganisms that were members of the *Enterococcus, Lactococcus* and *Streptococcus* genera. These genera broadly encompass enterococci, the lactic streptococci and the pyrogenic, oral and anaerobic streptococci. Additionally, the genus *Melissococcus (q.v.)* contains an organism previously known as *Streptococcus pluton.* Additionally, most of the anaerobic streptococci (whose speciation is currently confused) will be transferred to genera such as *Peptostreptococcus,* but they are currently included in the *Streptococcus* genus. Streptococci are parasites of vertebrates that mainly inhabit the mouth and upper respiratory tract. Some species are pathogenic for humans and animals. Various antigens associated with Lancefield serological groups are characteristic of some of the species and are required for accurate identification of *Streptococcus* species. There is difficulty in differentiating the strains that belong to the pyrogenic, oral and anaerobic groups of *Streptococcus* especially. For example, many species especially in the oral group are undergoing active study, with consequent rearrangement of taxonomy and continuing emendation of description so that a number of areas are still not very clear. *See* John G. Holt *et al.,* Bergey's Manual of Determinative Bacteriology (9^{th} ed., Lippincott Williams & Wilkins 1994).

*Wolinella* species are isolated from the bovine rumen, human gingival sulcus, dental root canal infections and other clinical material.

The genus *Yokenella* was not included in Bergey's Manual of Systematic Bacteriology. The genus was created in 1984 by Kosako *et al., (Japan. J. Med. Sci. Biol.* 37: 117-24; *Int. J. Med. Sci. Biol.* 35: 223-5, 1985). It included one species, *Y. regensburgei.* The genus *Koserella* with its single species, *K. trabulsii,* was created in 1985 by Hickman-*Brenner et al., (J. Clin. Microbiol.* 21: 39-42; *Int. J. Syst. Bacteriol.* 35: 223-5, 1985). *Y regensburgei* and *K. trabulsii* were shown to be subject synonyms in 1987 by Kosako *et al., (Int. J. Syst. Bacteriol.* 37: 127-9). Other synonyms for this organism are *Hafnia* hybridization group 3, Enteric Group 45 (CDC), and NIH biogroup 9 (Japan). Today, the predominant name is *J. regensburgei.* The organism is isolated from human wounds, urine, sputum and stool and insect intestine, although its clinical significance is unknown. However, there is difficulty characterizing the microorganism. Both human and insect strains of *Yokenella* were first thought to be *Hafnia alvei* or a *Hafnia*-like species. *Yokenella* is also somewhat similar to species in the genera *Citrobacter* and *Escherichia.* Finally, it is further difficult to characterize *Yokenella* because several strains frequently give delayed *(i.e.* 3-7 days) positive reactions for several biochemical tests. Thus, a method of positively identifying this organism will be helpful clinically.

Novel sequences have been identified for the following species using the methods described herein.

**TABLE 2**

| **Species** | **Other Names For Species** | **SEQ ID NOS** |
|---|---|---|
| *Cedecea lapagei* | | 48667,48668 |
| *Citrobacter youngae* | | 48671,48672 |
| *Moellerella wisconsensis* | | 48680, 48682 |
| *Pantoea dispersa* | | 48681, 48683 |
| *Proteus penneri* | | 48675, 48678 |
| *Providencia rettgeri* | *Proteus rettgeri* | 48677 |
| *Providencia rustigianii* | *Providencia* | 48684, 48685 |
| | *friedericiana* | |
| *Streptococcus urinalis* | | 48688 |
| *Yokenella regensburgei* | *Koserella trabulsii* | 48686, 48687 |

### 4. Amplification

Amplification of genes (or DNA and RNA) can be performed using polymerase chain reaction (PCR) and other rapid amplification procedures known in the art, such as ligase chain reaction (LCR), transcription-mediated amplification (TMA), self-sustained sequence replication transcription, self-sustained sequence replication (3SR), nucleic acid sequence-based amplification (NASBA), strand displacement amplification (SDA), branched DNA (bDNA), cycling probe technology (CPR), solid phase amplification (SPA), rolling circle amplification technology (RCA), solid phase RCA, anchored SDA and nuclease dependent signal amplification (DNSA) *(Lee et al.,* 1997, Nucleic Acid Amplification Technologies: Application to Disease Diagnosis, Eaton Publishing, Boston, MA; Persing *et al.,* 1993 Diagnostic Molecular Microbiology: Principles and Applications, Amer. Soc. Microbiol., Washington, D.C.; *Westin et al.,* 2000, *Nat. Biotechnol.* 18:199-204). In the instance of amplifying rRNA, the transcripts can first be reverse-transcribed using RT-PCR and then any of the above cited amplification methods can be used to amplify the DNA transcripts obtained from RT-PCR. Any of these methods of rapid amplification can be used according to the basic concept of identifying sequence specific changes which discriminate microorganisms bases on genus, species, group, family and the like.

For DNA amplification by the widely used PCR (polymerase chain reaction) method, primer pairs are derived either from the DNA sequences surrounding the probe (*e.g.*, TaqMan® primer design) or from data bank sequences. Prior to synthesis, the potential primer pairs can be analyzed by using the program Oligo™ 4.0 (National Biosciences, Plymouth, MN) to verify that they are likely candidates for PCR amplifications.

During DNA amplification by PCR, two oligonucleotide primers binding respectively to each strand of the denatured double-stranded target DNA from the bacterial genome are used to amplify exponentially *in vitro* the target DNA by successive thermal cycles allowing denaturation of the DNA, annealing of the primers and synthesis of two new DNA strands of the target DNA at each cycle (Persing et al, 1993, Diagnostic Molecular Microbiology: Principles and Applications, American Society for Microbiology, Washington, D.C.). Briefly, the PCR protocols may be as follows. Clinical specimens or bacterial colonies or extracted genomic DNA were added directly to the 50 uL PCR reaction mixtures containing 50 mM KCl, 10 mM Tris-HCl pH 8.3,1.5 mM MgCl₂, 0.4 uM of each of the two primers, 200 uM of each of the four dNTPs and 1.25 Units of *Taq* DNA polymerase (Perkin Elmer).

PCR reactions can then subjected to thermal cycling (*e.g.*, 3 min at 95°C followed by 30 cycles of 30 sec at 95°C, 30 sec at 55°C and 45 sec at 72°C) using, for example, a Perkin Elmer Gene Amp System 2400 thermal cycler. Amplified DNA products can then be analyzed by standard ethidium bromide-stained agarose gel electrophoresis. It is clear that other methods for the detection of specific amplification products, which may be faster and more practical for routine diagnosis, may be used. Such methods may be based on the detection of fluorescence signal after amplification (*e.g.*, TaqMan® system from Perkin Elmer or Amplisensor™ from Biotronics Technology Corp., Lowell, MA) or liquid hybridization with an oligonucleotide probe binding to internal sequences of the specific amplification product. These novel probes can be generated preferably from genus-specific or species-specific fragment probes. Methods based on the detection of fluorescence are particularly useful for utilization in routine diagnosis as they are, very rapid and quantitative and can be automated.

To assure PCR efficiency, glycerol or dimethyl sulfoxide (DMSO) or other related solvents, can be used to increase the sensitivity of the PCR and to overcome problems associated with the amplification of target with a high GC content or with strong secondary structures. The concentration ranges for glycerol and DMSO are about 5-10% (v/v) and about 3-10% (v/v), respectively. For the PCR reaction mixture, the concentration ranges for the amplification primers and the MgCl₂ are about 0.1-1.0 µM and about 1.5-3.5 mM, respectively. Modifications of the standard PCR protocol using external and nested primers (*i.e.*, nested PCR) or using more than one primer pair (*i.e.*, multiplex PCR) may also be used (see *Persing et al.,* 1993, Diagnostic Molecular Microbiology: Principles and Applications, American Society for Microbiology, Washington, D.C.).

The person skilled in the art of DNA amplification knows the existence of other rapid amplification procedures such as ligase chain reaction (LCR), transcription-based amplification systems (TAS), self-sustained sequence replication (3SR), nucleic acid sequence-based amplification (NASBA), strand displacement amplification (SDA) and branched DNA (bDNA) (Persing *et al.,* 1993). The scope of this invention is not limited to the use of amplification by PCR, but rather includes the use of any rapid nucleic acid amplification methods or any other procedures, which may be used to increase rapidity and sensitivity of the tests. Any oligonucleotides suitable for nucleic acid amplification can also use approaches other than PCR and are contemplated for use herein.

Amplification products are classically detected by standard ethidium bromide-stained agarose gel electrophoresis. It is clear that other methods for the detection of specific amplification products, which may be faster and more practical, can be used. Such methods may be based on detection of fluorescence after or during amplification. One simple method for monitoring amplified DNA is to measure its rate of formation by measuring the increase in fluorescence of intercalating agents such as ethidium bromide or SYBR® Green I (Molecular Probes). If more specific detection is required, fluorescence-based technologies can monitor the appearance of a specific product during the reaction. The use of dual-labeled fluorogenic probes such as in the TaqMan® system (Applied Biosystems), which utilizes the 5' to 3' exonuclease activity of the *Taq* polymerase, is a good example (Livak *et al.,* 1995, *PCR Methods Appl.* 4: 357-62). TaqMan® can be performed during amplification and this "real time" detection can be done in a single closed tube, which eliminates post-PCR sample handling and consequently prevents the risk of amplicon carryover. Several other fluorescence-based detection methods can be used in real-time.

Fluorescence resonance energy transfer (FRET) is the principle behind the use of adjacent hybridization probes (Wittwer *et al.,* 1997 *BioTechniques* 22: 130-1, 138-8) and molecular beacons (Tyagi *et al.,* 1996, *Nature Biotech.* 14: 303-8). Adjacent hybridization probes are designed to be internal to the amplification primers. The 3' end of one probe is labeled with a donor fluorophore while the 5' end of an adjacent probe is labeled with an acceptor fluorophore. When the two probes are specifically hybridized in close proximity (spaced by 1 to 5 nucleotides) the donor fluorophores, which has been excited by an external light source emits light that is absorbed by a second acceptor that emit more fluorescence and yields a FRET signal.

Molecular beacons possess a stem-and-loop structure where the loop is the probe and at the bottom of the stem a fluorescent moiety is at one end while a quenching moiety is at the other end. The beacons undergo a fluorogenic conformation change when they hybridize their targets hence separating the fluorochrome from its quencher. The FRET principle is also used in an air thermal cycler with a built-in fluorometer (Wittwer *et al.,* 1997). The amplification and detection are extremely rapid as reactions are performed in capillaries; it takes only 18 min. to complete 45 cycles. Those techniques are suitable especially in the case where few pathogens are searched for. Boehringer-Roche Inc. sell the LightCycler™, and Cepheid makes the SmartCycler. These two apparatus are capable of rapid cycle PCR combined with fluorescent SYBR® Green I or FRET detection.

Microbial pathogen detection and identification may also be performed by solid support or liquid hybridization using, *e.g.*, genus-, species-, and/or strain-specific internal DNA probes, which hybridize to an amplification product. Such probes may be generated from any sequence discussed herein and designed to specifically hybridize to DNA amplification products that are objects of the present invention. The oligonucleotide probes may be labeled with biotin or with digoxigenin or with any other suitable reporter molecule. Hybridization on a solid support is amenable to miniaturization.

Preferred methods include oligonucleotide microarray technology. Currently, low to medium density arrays are available and can be used to capture fluorescent labeled amplicons (Heller *et al.,* "An integrated microelectronics hybridization system for genomic research and diagnostic applications," in Harrison *et al.,* 1998, Micrototal Analysis Systems '98 (Kluwer Academic Publisher, Dordrecht). Detection methods of hybridization are not limited to fluorescence. Potentiometry, colorimetry and plasmon resonance are some examples of alternative detection methods. In addition to detection by hybridization, nucleic acid microarrays could be used to perform rapid sequencing by hybridization.

4.1 Amplification Primers. Amplification primers need to be designed in order to amplify the DNA regions that contain probes (*i.e.*, species-specific oligonucleotide sequences) of interest. Multiple sequence alignments are made using the 16S (or 23S) rDNA sequence of the target species and 16S sequences of other species phylogenetically related to the target species, as well as species phylogenetically distant from the target species.

A typical PCR amplification requires the use of two primers, one called a Forward primer (typically designated by "F") and the other one called a Reverse primer (typically designated by "R"). The Forward primer is located upstream of the probe sequence, the Reverse primer is located downstream of the probe sequence.

Suitable primers strongly depend on the type of applications/technologies used for probe testing. If the species-specificity exclusively relies on the probe sequence, regions suitable for amplification primers should be as conserved as possible among all species included in the multiple sequence alignment. If, on the other hand, the user wishes to use the locus amplification process to achieve some of the specificity of the diagnostic test, then the regions suitable for amplification primers should be conserved for the target species and different for all the other species from which the target species should be differentiated.

Once the regions for amplification primers are selected, several characteristics need to be checked to ensure the formation of a good PCR product. These characteristics include one or more of the following:
- Primers should contain at least 18-20 nucleotides.
- Primers with long runs of a single base (*e.g.*, a run of A's) should generally be avoided. It is especially important to avoid 3 or more G's or C's in a row.
- The percentage of Guanine (G) and Cytosine (C) *(i. e.* GC%) in the primer sequence should be around 50%. G's and C's at 3' end of the primers should be avoided as they increase the chance of forming primer dimers.
- The melting temperature of primers (Tₘ) should be close to 70°C.
- The annealing temperature (Tₐ) used during PCR amplification should be about 5°C below the lowest Tₘ of the pair of primers to be used.
- Primers should not contain palindromic sequences, because palindromic sequences may lead to the formation of hairpins that reduce the efficiency of priming of the primer to the DNA to amplify.

All these primer design guidelines can be easily evaluated for a particular pair of primer using standard computer software for primer design, such as but not limited to GCG (Genetics Computer Group, Madison, Wisconsin) or Oligo (Molecular Biology Insights, Inc, Cascade, USA).

### 5. Hybridization

Probes are classified as primers and/or probes for use in amplifying and/or isolating genus-, species- and strain-specific moieties. Then there are the probes used for diagnostic, prognostic, and research purposes that allow the categorization of the bacterium based on phylum, family, genus, species and strain. The latter probes will contain a distinguishing moiety that will allow the probe to distinguish between families, genera, species and/or strains of bacteria. However, regardless of the category of probe, most will share similar characteristics and will follow the similar guidelines for their preparation and selection.

In general, "primers" as used herein are oligonucleotides that are used for amplifying the regions containing the differentiation moieties. "Probes" are oligonucleotides that are used to distinguish between genera, groups, species, and strains of bacteria. For the instant invention, primers can range in size from 15 nucleotides to 50 nucleotides, and more preferably from 17 nucleotides to 30 nucleotides, and most preferably from 20 nucleotides to 25 nucleotides. Probe size can range in size from 10 to 100 nucleotides, and more preferably from 15 nucleotides to 30 nucleotides, and most preferably from 20 nucleotides to 25 nucleotides.

Because the extent and specificity of hybridization reactions such as those described herein are affected by a number of factors, manipulation of one or more of those factors will determine the exact sensitivity and specificity of a particular probe, whether perfectly complementary (*i.e.*, an oligonucleotide chain in which all of the bases are able to form base pairs with a sequence of bases in another polynucleotide chain) to its target or not. The importance and effect of various assay conditions, explained further herein, are known to those skilled in the art.

First, the stability of the [probe:target] nucleic acid hybrid should be chosen to be compatible with the assay conditions. This may be accomplished by avoiding long A and T rich sequences, by terminating the hybrids with G:C base pairs, and by designing the probe with an appropriate Tₘ. The beginning and end points of the probe should be chosen so that the length and % GC result in a Tₘ about 2-10°C higher than the temperature at which the final assay will be performed. The base composition of the probe is significant because G-C base pairs exhibit greater thermal stability as compared to A-T base pairs due to additional hydrogen bonding. Thus, hybridization involving complementary nucleic acids of higher G-C content will be stable at higher temperatures. Probe selection for use in the *in silico* method is dependent on the overlapping nature of the oligomers (*e.g.*, 30-mer) used. Once the distinguishing moiety is identified, then suitable diagnostic, prognostic and research probes can be prepared, according to these guidelines and what is generally known in the art, which comprise the distinguishing moiety (*e.g.*, one or more nucleotides which distinguish, for example, different species of bacteria).

Conditions such as ionic strength and incubation temperature under which a probe will be used should also be taken into account in constructing a probe. It is known that hybridization will increase as the ionic strength of the reaction mixture increases, and that the thermal stability of the hybrids will increase with increasing ionic strength. On the other hand, chemical reagents, such as formamide, urea, DMSO and alcohols, which disrupt hydrogen bonds, will increase the stringency of hybridization. Destabilization of the hydrogen bonds by such reagents can greatly reduce the Tₘ. In general, optimal hybridization for synthetic oligonucleotide probes of about 10-50 bases in length occurs approximately 5°C below the melting temperature (Tₘ) for a given duplex. Incubation at temperatures below the optimum may allow mismatched base sequences to hybridize and can therefore result in reduced specificity.

It is desirable to have probes that hybridize only under conditions of high stringency. Under high stringency conditions, only highly complementary nucleic acid hybrids will form; hybrids without a sufficient degree of complementarity will not form. Accordingly, the stringency of the assay conditions determines the amount of complementarity needed between two nucleic acid strands forming a hybrid. Stringency is chosen to maximize the difference in stability between the hybrid formed with the target and the non-target nucleic acid. In some examples of the current invention, it may be necessary to detect single base pair changes. In those instances, conditions of very high stringency are needed.

Second, probes should be positioned so as to minimize the stability of the [probe:non-target] nucleic acid hybrid. This may be accomplished by minimizing the length of perfect complementarity to non-target organisms, avoiding GC rich regions of homology to non-target sequences, and by positioning the probe to span as many destabilizing mismatches as possible. Whether a probe sequence is useful to detect only a specific type of organism depends largely on the thermal stability difference between [probe:target] hybrids and [probe:non-target] hybrids. In designing probes, the differences in these Tₘ values should be as large as possible (*e.g.*, at least about 2°C and preferably about 5°C).

The length of the target nucleic acid sequence and, accordingly, the length of the probe sequence can also be important. In some cases, there may be several sequences from a particular region, varying in location and length, which will yield probes with the desired hybridization characteristics. In other cases, one sequence may be significantly better than another which differs merely by a single base. While it is possible for nucleic acids that are not perfectly complementary to hybridize, the longest stretch of perfectly complementary base sequence will normally primarily determine hybrid stability. While oligonucleotide probes of different lengths and base composition may be used, oligonucleotide probes preferred in this invention are between about 10 to about 50 bases in length, and most preferred 20-30 bases in length, and are sufficiently homologous to the target nucleic acid.

Third, regions in the target DNA or RNA that are known to form strong internal structures inhibitory to hybridization are less preferred. In the preferred embodiment, the probe will hybridize to an rDNA. However, hybridization between probes and rRNA, DNA prepared from rDNA, and cDNA prepared from rRNA are also contemplated. Likewise, probes with extensive self-complementarity should be avoided. As explained above, hybridization is the association of two single strands of complementary nucleic acids to form a hydrogen bonded double strand. It is implicit that if one of the two strands is wholly or partially involved in a hybrid that it will be less able to participate in formation of a new hybrid. There can be intramolecular and intermolecular hybrids formed within the molecules of one type of probe if there is sufficient self-complementarity. Such structures can be avoided through careful probe design. By designing a probe so that a substantial portion of the sequence of interest is single stranded, the rate and extent of hybridization may be greatly increased. Computer programs are available to search for this type of interaction. However, in certain instances, it may not be possible to avoid this type of interaction.

The probes of the present invention are designed for attaining optimal performance under the same hybridization conditions so that they can be used in sets for simultaneous hybridization; this highly increases the usability of these probes and results in a significant gain in time and labor. Evidently, when other hybridization conditions should be preferred, all probes should be adapted accordingly by adding or deleting a number of nucleotides at their extremities. It should be understood that these concomitant adaptations should give rise to essentially the same result, namely that the respective probes still hybridize specifically with the defined target. Such adaptations might also be necessary if the amplified material should be RNA (*e.g.*, rRNA) in nature and not DNA.

The hybridization conditions can be monitored relying upon several parameters, such as the nature and concentration of the components of the media, and the temperatures under which the hybrids are formed and washed.

The hybridization and wash temperature is limited in upper value depending on the sequence of the probe (*i.e.*, its nucleic acid composition, kind and length). The maximum hybridization or wash temperature of the probes described in the present invention ranges from about 40°C to about 60°C, more preferably from about 45°C to about 55°C, in the specific hybridization and wash media as described in the Examples section. At higher temperatures, duplexing *(i.e.,* formation of the hybrids) competes with the dissociation *(i.e.,* or denaturation) of the hybrid formed between the probe and the target.

In a preferred hybridization medium of the invention, containing 3X SSC and 20% formamide, hybridization temperatures can range from about 45°C to about 55°C, with a preferred hybridization temperature of 50°C. A preferred wash medium contains 3X SSC and 20% formamide, and preferred wash temperatures are the same as the preferred hybridization temperatures, *i.e.*, preferably between 45°C and 55°C, and most preferably 50°C.

However, when modifications are introduced, be it either in the probes or in the media, the temperatures at which the probes can be used to obtain the required specificity should be changed according to known relationships, such as those described in the following reference: B. Hames and S. Higgins (eds.), Nucleic Acid Hybridization ― A Practical Approach, IRL Press, Oxford, U.K., 1985.

The selected nucleic acid probes derived from the 16S rRNA using *the in silico* methods and provided herein include SEQ ID NOS: 1- 48664. As described in the examples section, some of these probes show a better sensitivity and/or specificity than others, as reflected in the overall quality score for each probe *(see* Tables 14 and Table 20 which are attached to the specification as a separate document) which incorporates the estimated robustness of the probe and its predicted ability to detect most strains of a species. The better probes are therefore preferentially used in methods to detect the organism of interest in a biological sample. However, it is possible that for certain applications (*e.g.*, epidemiology, substrain typing, and groups which comprise two or more species and potentially even spanning genera) a set of probes including the less specific and/or less sensitive probes may be very informative.

Preferably said probes share at least 70% or 80% identity with the complementary sequence. More preferably, the probes share at least 90% identity (*e.g.*, 3 mismatches in a 30 base probe). More preferably the probes are at least about 95%, 96%, 97%, 98%, or 99% identital to the exact complement of the target sequence to be detected. Most preferred, the probe is homologous to the target sequence (*i.e.* has 100% sequence identity). Preferably, the target sequences are either ribosomal RNA or ribosomal DNA or amplified versions thereof. A probe with an apparent mismatch at one nucleotide position in its matching species rDNA sequence is still considered specific for the purposes of this invention because (1) available sequence databases contain occasional sequence errors (*i.e.*, the apparent mismatch may not be an actual mismatch), and (2) in practice the hybridization conditions and the method determine the actual specificity of a probe. Therefore, it is important to maintain some flexibility in selecting probes for potential use, and to qualify them practically by testing them under actual hybridization conditions.

Preferably, these probes are about 5 to 60 nucleotides long, more preferably from about 10 to 30 nucleotides. The nucleotides as used in the present invention may be ribonucleotides, deoxyribonucleotides and modified nucleotides such as inosine or nucleotides containing modified groups, which do not essentially alter their hybridization characteristics. Also contemplated is the use of peptide nucleic acid probes (PNA). Moreover, it is obvious to one skilled in the art that any of the below-specified probes can be used as such, or in their complementary form, or in their RNA form (wherein T is replaced by U).

The probes according to the invention can be formed by cloning of recombinant plasmids containing inserts including the corresponding nucleotide sequences, if need be by cleaving the latter out from the cloned plasmids upon using the requisite nucleases and recovering them, *e.g.*, by fractionation according to molecular weight. The probes according to the present invention can also be synthesized chemically, for instance by the conventional phosphotriester method.

The term "polynucleic acid" as used herein corresponds to either double-stranded or single-stranded DNA, rDNA, cDNA or genomic DNA or RNA, containing at least 5, 10, 15, 20, 25, 30, 40, 45 or 50 contiguous nucleotides or any length in between. A polynucleic acid that is smaller than 100 nucleotides in length is often also referred to as an oligonucleotide. Thus, in some instances polynucleotides greater than 100 bases are contemplated. Additionally the terms oligonucleotide and polynucleotide may be used interchangeably. Single stranded polynucleic acid sequences are always represented in the current invention from the 5' end to the 3' end.

The term "sensitivity" refers to the number of false negatives: *i.e.* if 1 of 100 strains to be detected is not detected, the test shows a sensitivity of [(100-1)/100]% = 99%.

The term "specificity" refers to the number of false positives: *i.e.* if out of 100 strains detected, 2 actually belong to species for which the test is not designed, the specificity of the test is [(100-2)/100]% = 98%.

The probes selected as being "preferential" show a sensitivity and specificity of more than 80%, preferably more than 90% and most preferably more than 95%. The higher the robustness number (*i.e.*, the number of nucleotide changes required for a species-specific oligonucleotide sequence to match a second species), the more likely the probe will have a high specificity score. The precise relationship between robustness number and specificity depends on the method used to apply the species-specific oligonucleotide sequences of this invention. In general, probes with a robustness number of 2 or more are useful in hybridization-based methods that can distinguish 1-2 nucleotide differences such as the Affymetrix Gene Chip System. However, use of probes with higher robustness numbers increases the likelihood of achieving high specificity in a diagnostic test.

The term "primer" refers to a single stranded DNA oligonucleotide sequence capable of acting as a point of initiation for synthesis of a primer extension product which is complementary to the nucleic acid strand to be copied. The length and the sequence of the primer must be such that they allow to prime the synthesis of the extension products. Preferably the primer is about 5-50 nucleotides long. Specific length and sequence will depend on the complexity of the required DNA or RNA targets, as well as on the conditions of primer use such as temperature and ionic strength. The fact that amplification primers do not have to match exactly with the corresponding template sequence to warrant proper amplification is amply documented in the literature. Methods of amplifying products using primers are known in the art.

The oligonucleotides used as primers or probes may also comprise nucleotide analogues such as phosphorothioates, alkylphosphorothioates or peptide nucleic acids or may contain intercalating agents.

As most other variations or modifications introduced into the nucleic acid sequences of the invention, these variations may necessitate adaptions with respect to the conditions under which the oligonucleotide should be used to obtain the required specificity and sensitivity. However, the eventual results of hybridization will be essentially the same as those obtained with the unmodified oligonucleotides.

The introduction of these modifications may be advantageous in order to positively influence characteristics such as hybridization kinetics, reversibility of the hybrid-formation, biological stability of the oligonucleotide molecules, etc.

The term "solid support" can refer to any substrate to which an oligonucleotide probe can be coupled, provided that it retains its hybridization characteristics and provided that the background level of hybridization remains low. Usually the solid substrate will be a microtiter plate, a membrane (*e.g.* nylon or nitrocellulose), slide or microarray, or a microsphere (bead). Prior to application to the membrane or fixation it may be convenient to modify the nucleic acid probe in order to facilitate fixation or improve the hybridization efficiency. Such modifications may encompass homopolymer tailing, coupling with different reactive groups such as aliphatic groups, -NH₂ groups, -SH groups, carboxylic groups, or coupling with biotin, haptens or proteins.

The term "labeled" refers to the use of labeled nucleic acids. Labeling may be carried out by the use of labeled nucleotides incorporated during the polymerase step of the amplification or by the use of labeled primers, or by any other method known to the person skilled in the art. The nature of the label may be isotopic (*e.g.*, ^{32p}, ³⁵S, etc.) or non-isotopic (*e.g.*, biotin, digoxigenin, etc.).

### 6. Preparation of Samples

In other embodiments of the invention, the sequences identified by these methods can be used in the form of probes and in kits for the identification of a specific family, genus, group, species or strain of a bacterium in a sample.

Typically, detection of the microorganism is performed by first isolating DNA from the sample. The sample can be, for example, from food, a mixed environmental sample or a biological sample from a subject (*e.g.*, urine or blood). The cells from a sample are typically pelleted and resuspended in a lysis buffer (*e.g.*, 10 mM Tris-HCl, 10 mM NaCl, 50 mM EDTA at pH 8.0). The cells are subjected to an enzymatic digest which can comprise, *e.g.*, 25 U Lysostaphin, 30 µg N-Acetylmuramidase, 400 µg Achromopeptidase, and 600 µg lysozyme in a final volume of 306 µL of lysis buffer. The digestion is carried out for 30-45 minutes at 37°C. Lysates are extracted with chloroform/phenol, then ethanol precipitated. The DNA is redissolved in a Tris/EDTA buffer and the final concentration is determined by spectrophotometric measurement. Variations of this method would by known to the skilled artisan.

6.1 Amplification and Labeling of DNA. An amplification reaction is generally described in PCR PROTOCOLS: A GUIDE TO METHODS AND APPLICATIONS (Michael Innis *et al.,* ed., 1990); PCR STRATEGIES, (David H. *Gelfand et al.,* eds., 1998); *Ausubel et al.,* CURRENT PROTOCOLS IN MOLECULAR BIOLOGY, (Greene Publishing Co., NY, 1995), and Schweitzer and Kingsmore, "Combining nucleic acid amplification and detection" (Current Opinion in Biotechnology, 2001; 12: 21-27).

Alternatively, rRNA from a sample can be used by first processing it using RT-PCR to produce DNA, or probes can be allowed to directly hybridize to the rRNA once it is isolated from the sample. Different labeling techniques for hybridization probe have been developed. In the past, labeling of nucleic acids by the enzymatic incorporation of radioactive isotopes (³²P, ³³P, ³⁵S, ³H, and ¹²⁵I) was the method of choice. However, in an effort to move away from the drawbacks associated to the use of radioactive products, DNA labeling techniques with non-radioactive products have been successfully developed. These non-isotopic labeling alternatives include among others the biotin-avidin/streptavidin system, the digoxigenin (DIG)/enzyme-labeled anti-DIG antibodies, the use of fluorescent dyes such as fluorescein, rhodamine, and cyanine dyes (Cy3 and Cy5). Nucleic acid labeling can be done by nick translation, random prime labeling, end-labeling reactions, and PCR. Several direct labeling kits are commercially available from different companies (Amersham Pharmacia Biotech Inc, Piscataway, NJ; Molecular Probes Inc, Eugene, OR; Vysis Inc, Downers Grove, IL) and make DNA labeling relatively easy. Labeled nucleic acid probes have been used in a wide range of applications in medicine, food industry, and environmental studies. For instance, probes labeled with fluorophore tags have been used in Fluorescent In Situ Hybridization (FISH) assay to detect bacteria in hemocultures (Oliveira et al., J Clin Microbiol. 2002, 40: 247-51).

To facilitate identification of a strain of a microorganism from a pattern of amplification products generated from the genome of that organism, the pattern should contain elements common to all members of the species in conjunction with elements which differentiate the unique strains within that species This only needs to be performed if it is necessary to distinguish strains. In the event that it is not necessary to distinguish between the strain of pathogen, this step can be omitted. This step is not necessary for the identification of many types of pathogens. In most cases, it will be preferable to include all strains in the species identification test.

This attribute makes it possible to identify a new strain of a species, which is not contained within the reference pattern database, providing that the common elements of that species pattern are contained within the reference database. If different strains of the same species each generate completely unique patterns with no common species elements, then the patterns themselves will not identify the species of an unknown strain. However, again typically there will not be a need to identify the strain. Additional methods of amplifying and labeling DNA are described *infra* and would be known to the artisan of ordinary skill.

6.2 Assays for Use with Plants Bacteria also cause problems in agriculture. For example, the bacterium, *Acidovorax avenae,* is a seed borne pathogen of several hosts including oats, corn, millet, wheat, sugarcane, rice and melons. It causes bacterial stripe of rice, leaf blight of oats, red stripe disease of sugarcane and millet and brown stripe of *Setaria italica.* It is an example of an organism which is hard to test for as it can be overgrown by other plant pathogens, such as the rice pathogens of *Pantoea (Erwinia) herbicola, Burkholdera (Pseudomonas) glumae, B. fuscovaginae,* or *P. Syringae pv. syringae.* Although these pathogens are known to produce distinct disease symptoms, field diagnosis remains difficult. Isolation of a pathogen is difficult in the presence of coexisting epiphytes. Thus, more sensitive testing methods are needed. Differentiation of microorganisms based on phyla, genus and species is important as well as testing of seed lots to determine whether seed batches are contaminated. Preferred bacteria include, but are not limited to, *Pseudomonas fluorescens, Pseudomonas syringae* species of the *Burkholderia cepacia* complex (Bcc), *Erwinia* species, *Xanthomonas* Species, *Agrobacterium* species and *Clavibacter* species.

DNA amplification and biological sample testing can be carried out generally as follows. In brief, the DNA amplification process is carried out by (a) providing a biological sample comprising bacterial cells or extracted DNA for standard PCR or cells amplified by growing on an agar medium for BIO-PCR; (b) amplifying a target sequence of the DNA to provide DNA amplification products carrying the selected target DNA sequence; and (c) detecting the presence of the DNA amplification products as an indication of the presence of, *e.g., A. avenae.*

The biological sample may either be bacteria cells or extracted genomic DNA. The biological sample may be a test sample suspected of containing bacterial cells, and thus the DNA of the bacterial cells, or a test sample containing extracted DNA.

The BIO-PCR method combines biological pre-amplification of the PCR target organism with enzymatic amplification of the PCR target. Briefly, the advantages of the BIO-PCR method, over those of the standard PCR assay, include the detection of live cells only, a 100-1000 fold increase in sensitivity, and elimination of PCR inhibitors associated with plant samples thereby eliminating false negatives. Sample processing can be further simplified by directly processing the samples comprising the expanded cells without further DNA extraction. However, even if a DNA extraction step is included, an advantage of the BIO-PCR methodology is that the DNA is extracted from a growing, viable population of cells or microorganisms. The enhanced sensitivity of the BIO-PCR method is particularly valuable, for example, in those screening situations where the monetary value of a particular seed type is high, and thus it is desirable to test the smallest quantity of seeds possible.

The preamplification step involves a plating step on an agar growth medium (or a liquid medium) prior to PCR analysis. A single cell per 0.1 ml can be detected because the single cell multiplies into a colony containing over 1000 cells on the agar medium.

Bacteria are recovered from suspect seeds of rice and watermelon by first soaking 1000-2000 seeds in 0.02% TWEEN 20 solution (ratio of 5 ml/g) for 4 hours at 40°C, pipetting aliquots of 0.1 ml of the bacterial extracts either onto plates of general purpose agar media, such as, KB or nutrient agar (NA), or onto a semi-selective medium such as EBB, described below, and then cloning. For BIO-PCR, each of five plates is washed three times with 1.0 ml of water and the resulting 14-15 ml of wash solution can be used for PCR-amplification with or without further DNA extraction or sample processing. Similarly, for standard PCR, either the DNA can be extracted or intact cells can be used. Since only pinpoint-size colonies are needed, incubation time ranges from only 10-15 hr for fast growing bacteria to 24-48 hrs for most plant pathogenic bacteria, depending on the media. Since the incubation time is short, few other bacterial colonies are present.

EBB medium is a preferred, semi-selective medium to use for the preamplification step. The protocol for preparing the EBB Medium is as follows:
(1) Mix, per liter: 1.0 g NH₄PO₄, 0.2 g MgSO₄·7H₂O, 0.2 g KCl, 0.3 g Yeast Extract, 0.5 g boric acid, 1.0 mL Brilliant blue R (10 mg/ml Stock), 0.6 ml Bromcreosol purple (15 mg/ml Stock), and 16.0 g agar;
(2) Autoclave and add, per liter: 10.0 ml 95% ethanol, 1.0 ml cycloheximide (200 mg/ml 95% ethanol Stock);
(3) Adjust pH carefully to 5.2 using 0.2 M HCl just before autoclaving. (If the pH falls below 5.0, do not use NaOH to readjust to a pH of 5.2 as NaOH will neutralize brilliant blue R).

In the preferred method, the enzymatic amplification of the DNA sequence is by polymerase chain reaction (PCR), as described herein.

For the binding and amplification, the biological sample (bacterial cells or extracted DNA) is provided in an aqueous buffer formulated with an effective amount of a divalent cation, which is preferable MgCl₂, preferably at a concentration of about 0.05-5 mM; an effective amount of DNA polymerase with *Taq* DNA polymerase being preferred in the form of native purified enzyme or a synthesized form such as AMPLITAQ® (Perkin-Elmer), an effective amount of dNTPs as a nucleotide source, including, dATP, dCTP, dGTP, and dTTP, preferably in a saturating concentration, preferably about 200 FM per dNTP; and an effective amount of one or a pair of oligonucleotide primers. The reaction mixture containing the annealed primer(s) is reacted with a DNA polymerase in a thermocycler. Each PCR cycle begins with a DNA denaturation step of 94°C for 30 s, followed by a primer annealing step at 60°C for 30 s, and a DNA elongation step at 72°C for 45 s.

If designed properly, a single product results. This product is preferably about 450-550 kb in size, whose termini are defined by the oligonucleotide primer(s), and whose length is defined by the distance between the two primers or the length of time of the amplification reaction. The gene sequence then serves as a template for the next amplification cycle.

The amplified DNA product is optionally separated from the reaction mixture and then analyzed. The amplified gene sequence may be visualized, for example, by electrophoresis in an agarose or polyacrylamide gel or by other like techniques, known and used in the art.

The amplified gene sequence may be directly or indirectly labeled by incorporation of an appropriate visualizing label, as for example, a radioactive, calorimetric, fluorometric or luminescent signal, or the like. In addition, the gel may be stained during or after electrophoresis with a visualizing dye such as ethidium bromide or silver stain, wherein the resulting bands by be visualized under ultraviolet light. The amplification of the gene sequence can be performed using PCR as described herein, or as would be known in the art.

To prove the identity of the amplified DNA product, a Southern blot assay should be conducted. The amplified products are separated by electrophoresis on a polyacrylamide or agarose gel, transferred to a membrane such as a nitrocellulose or nylon membrane, reacted with an oligonucleotide probe, and stained as above. The amplified products may also be detected by reverse blotting hybridization (dot blot) in which an oligonucleotide probe specific to the gene sequence is adhered to a nitrocellulose or polyvinylchloride (PVC) support, such as a multi-well plate, and then the sample containing labeled amplified product is added, reacted, washed to remove unbound substance, and a labeled amplified product attached to the probe or the gene sequence imaged by standard methods. A major advantage of TaqMan® PCR is that the technology is based on hybridization; therefore, a Southern blot assay is not needed.

The detection of amplified gene product in the sample is evidence of the presence of the bacterium, *e.g., A. avenae* subspecies, in the biological sample. When combined with BIO-PCR, the method is useful in diagnosing presence of viable cells of, *e.g., A. avenae.*

The primers and amplification method can further be useful for evaluating and monitoring the efficacy of any treatments utilized to eliminate the pathogen. In this method, biological samples are obtained from seeds, or other biological samples, prior to treatment and from seeds, or other biological samples that have undergone treatment with a treatment protocol. In addition, biological samples can be obtained from seeds at several time points during treatment. DNA amplification products of a target sequence of pathogen from all samples are analyzed for the presence of the pathogen. Results from samples obtained prior, during and after treatment are compared in order to determine efficacy of the treatment protocol.

### 7. Diagnostic, Prognostic and Classification Assays

It is also contemplated that the distinguishing family-, genus-, species- and/or strain-specific moieties be used in kits, such as a diagnostic kit. Additionally these probes can be used in kits helpful identifying a strain, which can be useful for classification or determining the appropriateness of a particular drug regimen to be administered to a subject. These kits may comprise any one or more of the following components:
(1) Unique components in accordance with the present invention:
   (a) An oligonucleotide complementary to rDNA or rRNA which comprises a distinguishing moiety (*e.g.*, a family-, genus-, group-, species- and/or strain specific nucleotide(s) or combination of nucleotides),
   (b) Oligonucleotide primers for use in amplification (*e.g.*, PCR) designed to amplify a sequence, where a first primer has a sequence 5' to a region comprising a distinguishing moiety, and a second primer that has a sequence 3' to the distinguishing moiety.
   (c) A negative control to confirm the identity of a sequenced test fragment. Preferably, the negative control is a species whose rDNA is mismatched with the positive control by varying numbers of nucleotides.
   (d) A positive control, such as a strain known to be a member of the family, genus, group, species being tested.
(2) Commercially available reagents:
   (a) Components of an amplification protocol, such as PCR.

Herein is provided a list of the genus-, group-, species- and strain- specific rRNA moieties thus identified *(see* Tables 14, 15, 20, and 21 which are attached to the specification as separate documents).

### 8. 16S Nucleic Acid Microarrays

8.1. Nucleic Acid Microarrays. The present invention provides for compositions comprising a plurality of polynucleotide probes which contain preferably two or more ribosomal moieties, *e.g.*, SEQ ID NOS: 1- 48664. The microarrays can be prepared for entire phylum, families, genera, species, groups, strains, or isolates of bacteria. Alternatively, diagnostic microarrays can be prepared based on infection phenotypes, *e.g.*, all bacteria that produce skin lesions or particular types of skin lesions.

In one embodiment, the arrays can be prepared based on the origin of the biological sample, which would indicate only a certain grouping of organisms. Thus, microarrays can be created for screening body fluids, bronchial aspirates, cerebrospinal fluid, genital swabs, nares swabs, wounds, sputum, stool *(e.g.,* for enteric pathogens, *Yersinia, Aeromonas* and *Pleisomonas* for example), throat samples, urine, miscellaneous sterile sites (*e.g.*, surgical specimens), and blood culture (*e.g.*, for aerobes, anaerobes, yeast, fungus, Mycobacteria). Arrays can also be created to screen cultures obtained from such biological samples. Preparations of such cultures and for obtaining such biological samples are known and routine in the art.

Biological samples can be obtained and screened based on clinical symptoms as determined by a health worker. For example, specific microarrays may be prepared based on inflammatory response, immune response or complement response, such as described in Table 3 below, which differentiate infectious diseases from conditions not linked to infection.

**TABLE 3**

| **Host Defect** | **Examples of diseases or therapies associated with defects** | **Common etiological agents of infection** |
|---|---|---|
| INFLAMMATORY RESPONSE | | |
| Neutropenia | Hematologic malignancies, cytotoxic chemotherapy, aplastic anemia | Gram-negative enteric bacilli, *Pseudomonas* spp., *Staphylococcus* spp., *Candida* spp. *Aspergillus* spp. |
| Chemotaxis | Chϑdiak-Higashi syndrome, Job's syndrome, protein-calorie malnutrition | *Staphylococcus aureus, Streptococcus pyogenes, Haemophilus influenzae,* Gram-negative bacilli |
| Phagocytosis (cellular) | Systemic lupus erythematosus, chronic myelogenous leukemia, megaloblastic anemia | *Streptococcus pneumoniae, Haemophilus influenzae* |
| Microbicidal defect | Chronic granulomatous disease | Catalase positive bacteria and fungi, staphylococci, *E. coli, Klebsiella* spp., *Pseudomonas aeruginosa, Aspergillus* spp., *Nocardia* spp. |
| | Chϑdiak-Higashi syndrome | *Staphylococcus aureus, Streptococcus pyogenes* |

| COMPLEMENT SYSTEM | | |
|---|---|---|
| C3 | Congenital liver disease, systemic lupus erythematosus | *Staphylococcus aureus, Streptococcus pneumoniae, Pseudomonas* spp., *Proteus* spp. |
| C5 | Congenital | *Neisseria* spp., Gramnegative rods |
| C7, C7, C8 | Congenital, systemic lupus erythematosus | *Neisseria meningitides, Neisseria gonorrhoeae* |
| Alternate pathway | Sickle cell disease | *Streptococcus pneumoniae, Salmonella* spp. |

Microarrays can also be prepared based on means by which an organism is introduced into a host (*e.g.*, bites, scratches, bums and environmental organisms), associated with surgery (*e.g.*, prosthetic devices, infectious complications of solid organ transplantation) or result from suppressed immunity (*e.g.* AIDS).

Additional microarrays can be prepared for organisms common for causing sepsis, which results in 300,000 to 500,000 incidences a year, and about 100,000 deaths per year in the United States alone. Such microarrays would typically comprise nucleic acids as discussed herein from Gram-positive cocci (10-20% of the cases), Gram-negative, bacteria and fungi. Treatment of sepsis must be rapid and specific in order to eradicate the organisms from the blood stream and thus there is a continued need to develop faster and more accurate diagnostic screening methods.

Arrays can be prepared which are specific towards infectious diseases of the upper respiratory tract. These arrays would comprise nucleic acids identified by the methods described herein of organisms which cause nasal infections (commonly caused by *Mycobacterium tuberculosis, M. leprae, Pseudomonus malleli, K. rhinoscleromatis, Rhinospordium seeberi, Actinomyces israelii, Cryptococcus neoformans, Blastomyces hominis),* paranasal sinus infections *(e.g.,* commonly caused by *S. pneumoniae, Haemophilus influenazae,* streptococci, and *Moraxella),* ear infections (commonly caused by *S. pneumoniae, H. influenzae, Branhamella catarrhalis, Streptococcus* and *S. aureus).* Similar arrays can be prepared to identify the microorganism causing an intraabdominal infection or abscess, acute infectious diarrheal disease and bacterial food poisoning (*e.g.*, common infectious causing agents include *Vibrio cholerae, E. coli, Clostridium perfringens, Bacillus cereus, Staphylococcus aureus, Aeromonas hydrophila, Plesiomonas shigelloides, Giardia lamblia, Cryptosporidum, Shigella* spp., *Salmonella enteritidis, Campylobacter jejuni, Vibrio parahemolyticus, Clostridium difficile, Entaemoeba histolytica, Salmonella typhi, Yersinia enterocolitica).* Additional arrays may be prepared that are directed towards screening for microorganisms responsible for a sexually transmitted infection (*e.g.*, commonly caused by *Neisseria gonorrhoeae, Chlamydia trachomatis, Treponema palidum, Calymmatobacterium granulomatis, Ureaplasma urealyticum, Mycoplasma hominis, Gardnerella vaginalis, Shigella* spp., and *Campylobacter* spp.), pelvic inflammatory disease, urinary tract infections (*e.g.*, commonly caused by Gram-negative bacilli) and pyelonephritis. Additionally, arrays may be prepared to screen for the microorganism responsible for infectious arthritis in a host (such as Gram-postive cocci induced arthritis, gonococcal arthritis, chronic monarticular arthritis, viral arthritis, and spirochetal arthritis).

Alternatively, microarrays can be prepared based on, for example, bacterial classification. For example, arrays can be prepared for diseases that are caused by Gram-postive bacteria. Such infectious diseases include pneumococcal infections *(e.g.,* caused by *Streptococcus pneumoniae,* and include such conditions as pneumococcal pneumonia, pneumococcal meningitis, pneumococcal peritonitis, and pneumococcal endocarditis), staphylococcal infections (*e.g.*, commonly caused *by S. saprophyticus, S. aureaus, S. epidermidis* and include conditions such as staphylococcal scalded-skin syndrome, toxic shock syndrome, bacteremia, endocarditis, osteomyelitis, pneumonia and urinary tract infections), streptococcal infections (*e.g.*, caused commonly by *S. pyogenes, S. agalactiae, S. faecalis, S. equi, S. bovis, S. canis, S. mutans, S. anguis, S. milleri* and include conditions such as pharyngitis, cellulitis, urinary tract infections, bacteremia, endocarditis, sinusitis, pneumonia, and meningitis), corynebacterial infections *(C diphtheriae* which causes diphtheria), anthrax *(Bacillus anthracis), Listeria monocytogenes* infections *(e.g.,* contributes to conditions including sepsis, central nervous system infections, and endocarditis).

Also contemplated are microarrays, which distinguish diseases caused by Gram-negative bacteria. Gram-negative bacterial infections include, but are not limited to, meningococcal infections *(e.g., Neisseria meningitides* and *N. lactamica),* gonococcal infections *(e.g., Neisseria gonorrhoeae),* Moraxella (also known as Branhamella) infections *(e.g., M. catarrhalis, M. osloensis, M. nonliquefaciens, M. osloensis* and *M lacunata), Haemophilus* infections *(e.g.,* including *H. influenzae,* and Hacek group infections which include infections by *H. phrophilis, H. paraphrophilis, H. parainfluenzae, Actinobacillus actinomycetemcomitans, Cardiobacterium hominis, Eikenella corrodens* and *Kingella kingae),* and Legionella infections *(e.g.,* Legionnaire's disease). Another embodiment contemplates microarrays that distinguish between diseases caused by Gram-negative enteric bacilli *(e.g., E. coli, Klebsiella, Enterobacter, Serratia, Proteus, Morganella, Providencia* and *Acinetobacter).*

Microarrays for distinguishing *Pseudomonas* species may also be prepared. The common *Pseudomonas* organisms that infect humans include *P. aeruginosa, P. cepacia, Xanthoomonas maltophilia, P. pseudomallei,* and *P. mallei.* These organisms are responsible for bacteremia, endocarditis, central nervous system infections, ear infections, eye infections, bone and joint infections, gastrointestinal infections, urinary tract infections, and skin/tissue infections.

Arrays for distinguishing *Salmonella* organisms are also contemplated, as these organisms are responsible for a large variety of human infections including typhoid (or enteric) fevers, focal systemic infections, septicemias, and gastroenteritis. *S. typhi, S. paratyphi A, S. paratyphi B, S. typhimurium, and S. enteritidis* are the most common species responsible for infection.

Also contemplated are arrays for distinguishing *Shigella,* which is responsible to Shigellosis and which is closely related to *E. coli* such that they cannot normally be distinguished by DNA hybridization methods. Common species involved in human disease include *S*. *flexneri* type 2A, *S. dysenteriae,* and *S. sonnei.*

Another embodiment contemplates an array that screens for mycobacterial diseases, which have become a problem, for example in patiesnts who are immune suppressed (*e.g.*, AIDS patients). Mycobacteria are also responsible for leprosy (also known as Hansen's disease; *M. leprae)* and tuberculosis (caused by *M. tuberculosis).* Other Mycobacteria responsible for diseases include *M. haemophilum, M. kansasii, M. marinum, M. scrofulaceum, M. szulgai, M. ulcerans, M. xenopi, M. asiaticum,* and *M. simiae.* Also contemplated are microarrays that distinguish between the various species and strains of mycobacteria.

Microarrays can also be prepared which can distinguish between different spirochetes. Spirochetes are responsible for a wide range of infectious diseases including syphilis (caused by *Treponema pallidum),* leptospirosis (caused by *Leptospira* and which encompass Weil's disease and canicola fever), relapsing fever (caused by *Borrelia),* and Lyme borreliosis. In the instance of leptospirosis, *Leptospira* has only one species, *L. interrogans,* but has two complexes, interrogans and biflexa. The interrogans complex contains the pathogenic strains while the biflexa complex includes saphrophytic strains. Thus microarrays are preferred which can distinguish between the two complexes and potentially also the strains found in each of those complexes.

For *Borrelia* induced infections, there are several organisms responsible for the infections that are introduced either by louses or ticks. The most common *Borrelia* organisms include *B. recurrentis, B. duttoni, B. hermsii, B. parkeri,* and *B. tuicatae. Borrelia* are also responsible for Lyme disease (specifically *B. burgdorferi).* Thus preferred microarrays may contain nucleic acids for both Lyme disease and relapsing fever inducing microorganisms.

Microarrays can be prepared for use in distinguishing members of the genus of *Chlamydia.* Although classifed as bacteria, *Chlamydia* has its own order, Chlamydiales. The *Chlamydia* genus includes three species: *C. psittaci, C. trachmatis* and *C. pneumoniae. C. psittaci* can be found in numerous avian and mammalian species, but only the avian strains have been shown to infect humans causing a condition known as psittacosis. *C. trachmatis* is exclusively a human pathogen and is recognized to cause trachoma (a contagious form of conjunctivitis). It is also known to be one of the most common bacterial sexually transmitted diseases in the United States with an estimated 3 to 4 million cases annually. Such genital infections are responsible for urethritis, proctits, epididymitis in men, and mucopurulent cervicitis, acute salpingitis, bartholinitis and Fitz-Hugh-Curtis syndrome in women. *C*. *pneumoniae* is a fastidious species that appears to be a frequent cause of upper respiratory tract infections and pneumonia primarily in children and young adults.

Other organisms contemplated for use in such microarrays are provided in Tables 2 and 7. Microarrays using any of these organisms would preferentially be prepared based on any of the above-described compilations (*e.g.*, distinguishing between strains and species, diagnostic screening based on origin of introduction, similar clinical presentation between infectious organisms, or similarity based on relative proximity on the phylogenic tree).

Preferably, the plurality of polynucleotide probes comprise at least a portion of one or more of the sequences of Tables 14, 15, 20, or 21 (attached to the specification as separate documents) or a fragment thereof.

A microarray can be used for large scale genetic or gene expression analysis of a large number of target polynucleotides. These microarrays can also be used in the diagnosis and/or prognosis of diseases and in the monitoring of treatments.

When the composition of the invention is employed as hybridizable array elements in a microarray, the array elements are organized in an ordered fashion so that each element is present at a specified location on the substrate. Because the array elements are at specified locations on the substrate, the hybridization patterns and intensities can be interpreted in terms of the presence or absence of particular nucleic acid sequences and can be correlated with, for example, a particular bacterial infection or for classification, a particular group or strain of bacterium.

The composition comprising a plurality of polynucleotide probes can also be used to purify a subpopulation of rRNAs or rDNAs, DNAs, genomic fragments and the like, in a sample. Typically, samples will include target polynucleotides of interest and other nucleic acids which may enhance the hybridization background. Therefore, it may be advantageous to remove these nucleic acids from the sample. One method for removing the additional nucleic acids is by hybridizing the sample containing target polynucleotides with immobilized polynucleotide probes under hybridizing conditions. Those nucleic acids that do not hybridize to the polynucleotide probes are washed away. At a later point, the immobilized target polynucleotide probes can be released in the form of purified target polynucleotides.

8.1.1. Method for Selecting Polynucleotide Probes. This section describes the selection of probe sequences for the plurality of polynucleotide probes. In one embodiment, the probe sequences are selected based on robustness and likelihood of being species-specific.

The resulting composition can comprise polynucleotide probes that are not redundant, *i.e.*, there is no more than one polynucleotide probe to represent a particular distinguishing moiety for any particular rRNA and/or rDNA. Alternatively, and preferably, the composition can contain polynucleotide probes that are redundant, *i.e.*, a rDNA is represented by more than one polynucleotide probe, because there are multiple combinations of distinguishing moieties known for that bacterium's rDNA.

The selected polynucleotide probes may be manipulated further to optimize their performance as hybridization probes. Probes that may not hybridize effectively under hybridization conditions due to secondary structure are avoided. To optimize probe selection, the sequences are examined using a computer algorithm to identify portions of genes without potential secondary structure. Such computer algorithms are well known in the art, such as Oligo® 4.06 software (National Biosciences) LASERGENE® software (DNASTAR®), and more preferably Primer 3 (Whitehead Institute and Howard Hughes Institute). These programs can search nucleotide sequences to identify stem-loop structures and tandem repeats and to analyze G+C content of the sequence; those sequences with a G+C content greater than 60% are preferably excluded from use in microarray compositions. Alternatively, the probes can be optimized by trial and error. Experiments can be performed to determine whether probes and complementary target polynucleotides hybridize optimally under experimental conditions.

8.1.2. Polynucleotide Probes. This section describes the polynucleotide probes for use in a nucleic acid microarray. The polynucleotide probes can be genomic DNA or amplified fragments of genomic DNA, which includes portions the bacterial genome responsible for producing rRNA, preferably rDNA, or rRNA, or DNA or cDNAs derived from rRNA, or complements of any thereof. The probes can also include peptide nucleic acids, branched DNAs and the like. The polynucleotide probes can be sense or antisense polynucleotide probes to the 16S rRNA or rDNA sequences of the subject bacterium. Where target polynucleotides are double stranded, the probes may be either sense or antisense strands. Where the target polynucleotides are single stranded, the nucleotide probes are complementary single strands. The preferred lengths of the probes will range between 12 and 60 nucleotides, more preferably between 12 and 50 nucleotides, more preferably between 15 and 35 nucleotides and most preferably between 20 and 30 nucleotides, and any range in between.

In another embodiment, the polynucleotide probes are plasmids. In this case, the size of the DNA sequence of interest, *i.e.*, the insert sequence excluding the vector DNA and its regulatory sequences, may vary from about 15 to 2,000 nucleotides, more preferably from about 15 to 150 nucleotides.

The polynucleotide probes can be prepared by a variety of synthetic or enzymatic schemes that are well known in the art. The probes can be synthesized, in whole or in part, using chemical methods well known in the art. Alternatively, the probes can be generated, in whole or in part, enzymatically.

Nucleotide analogues can be incorporated into the polynucleotide probes by methods well known in the art. The only requirement is that the incorporated nucleotide analogues must serve to base pair with target polynucleotide sequences. For example, certain guanine nucleotides can be substituted with hypoxanthine which base pairs with cytosine residues. However, these base pairs are less stable than those between guanine and cytosine. Alternatively, adenine nucleotides can be substituted with 2,6-diaminopurine which can form stronger base pairs than those between adenine and thymidine.

Additionally, the polynucleotide probes can include nucleotides that have been derivatized chemically or enzymatically. Typical chemical modifications include derivatization with acyl, alkyl, aryl or amino groups.

The polynucleotide probes can be immobilized on a substrate. Preferred substrates are any suitable rigid or semi-rigid support including membranes, filters, chips, slides, wafers, fibers, magnetic or nonmagnetic beads, glass or plastic beads, gels, tubing, microtubes, plates, polymers, microparticles and capillaries. The substrate can have a variety of surface forms, such as wells, trenches, pins, channels and pores, to which the polynucleotide probes are bound. Preferably, the substrates are optically transparent.

Probes can be synthesized, in whole or in part, on the surface of a substrate using a chemical coupling procedure and a piezoelectric printing apparatus, such as that described in PCT publication WO95/251116 (Baldeschweiler *et al.).* Mechanical coupling is also an option. See U.S. Pat. No. 5,143,854 and PCT Application Nos. WO 90/15070 and WO 92/10092. Alternatively, the probe can be synthesized on a substrate surface using a self-addressable electronic device that controls when reagents are added (Heller et al. U.S. Pat. No. 5,605,662).

The arrays can have any density of oligonucleotide probes. Arrays can have probes of greater than about 100, preferably greater than about 1,000, more preferably greater than about 15,000, and most preferably greater than about 25,000 different oligonucleotide probes. Arrays with 50,000, 65,000, 200,000 or even 1,000,000 or more different probes are also contemplated. Such arrays generally comprise a probe density of typically greater than about 60, more generally greater than about 100, most generally greater than about 600, often greater than about 1,000, more oftren greater than about 5,000, preferably more than about 10,000 probes per cm². Some arrays may be preferred wherein the density is about 100,000 to 400,000 probes per cm².

DNAs corresponding to rRNA or rDNA can be arranged and then immobilized on a substrate. The probes can be immobilized by covalent means such as by chemical bonding procedures or using ultraviolet (UV) light. In one such method, a DNA is bound to a glass surface, that has been modified to contain epoxide or aldehyde groups. In another case, a cDNA probe is placed on a polylysine coated surface and then UV cross-linked (Shalon *et al.,* PCT publication WO95/35505). In yet another method, a DNA is actively transported from a solution to a given position on a substrate by electrical means (Heller et al. U.S. Pat. No. 5,605,662). Alternatively, individual DNA clones can be gridded on a filter. Cells are lysed, proteins and cellular components degraded, and the DNA coupled to the filter by UV cross-linking or other method known in the art.

Furthermore, the probes do not have to be directly bound to the substrate, but rather can be bound to the substrate through a linker group. The linker groups are typically about 6 to 50 atoms long to provide exposure to the attached polynucleotide probe. Preferred linker groups include ethylene glycol oligomers, diamines, diacids and the like. Reactive groups on the substrate surface react with one of the terminal portions of the linker to bind the linker to the substrate. The other terminal portion of the linker is then functionalized for binding the polynucleotide probe.

The polynucleotide probes can be attached to a substrate by dispensing reagents for probe synthesis on the substrate surface or by dispensing preformed DNA fragments or clones on the substrate surface. Typical dispensers include a micropipette delivering solution to the substrate with a robotic system to control the position of the micropipette with respect to the substrate. There can be a multiplicity of dispensers so that reagents can be delivered to the reaction regions simultaneously.

### 8.2 Hybridization Array Design

One of skill in the art will appreciate the enormous number of array options that are suitable for use with the disclosed methods and compositions. An array, including high density arrays, will typically include a number of probes that specifically hybridize to the target nucleic acid of interest. Such arrays should also preferably include one or more control probes. Such probes include but are not limited to test probes, normalization controls, mismatch controls, and sample preparation/amplification controls.

Test probes are oligonucleotide probes having sequences complementary to particular microorganism sequences whose expression they are designed to detect. Such test probes are typically from 5 to about 50 nucleotides, more preferably from about 10 to about 40 nucleotides and most preferably from about 15 to about 40 nucleotides in length.

Normalization controls are oligonucleotide probes that are perfectly complementary to labeled reference oligonucleotides that are added to the nucleic acid sample. The signals obtained from the normalization controls after hybridization provide a control for variations in hybrization conditions, label intensity, reading efficiency and other factors that may cause the signal of the hybridization to vary between arrays. In one embodiement, signals (*e.g.*, fluorescence intensity) read from all other probes in the array are divided by the signal from the control probes thereby normalizing the measurements. Although any probe may be used as a normalization control, it is recognized that hybridization efficiencies vary with base composition and probe length. Thus, preferred normalization probes are selected to reflect the average length of the other probes present in the array. Normalization probes can be localized at any position in the array or at multiple positions throught the array to control for spatial variation in hybridization efficiency.

Mismatch controls can also be used in the array. Mismatch controls are oligonucleotide probes identical to their corresponding test or control probes except for the presence of one or more mismatched bases. A mismatched base is a base selected so that it is not complementary to the corresponding base in the target sequence to which the probe would otherwise specifically hybridize. One or more mismatches are selected such that under appropriate hybridization conditions the control probe would be expected to hybridize with its target sequence, but the mismatch probe would not hybridize (or would hybridize to statistically significant lesser extent). Preferred mismatch probes contain a central mismatch. For example, with a 20-mer probe, a corresponding mismatch probe will have the identical sequence except for a single base mismatch at any of positions 6 to 14.

Sample preparation and amplification controls are probes that are complementary to sequences of control genes or gene subsequences because they do not normally occur in the nucleic acids of the particular biological sample being analyzed. For example, the use of a eukaryotic gene in a sample believed to contain a microorganism. The sample is then spiked with a known amount of the nucleic acid to which the sample preparation/amplification control probe is directed before processing.

### 8.3 Labeling Nucleic Acids

Hybridized nucleic acids can be detected using one or more labels attached to the sample nucleic acids. The labels may be incorporated by any of a number of means known in the art. Preferred embodiments include incorporating the label during the amplification step in preparing the sample nucleic acids.

Alternatively, a label may be added directly to the original nucleic acid sample (e.g., mRNA, polyA mRNA, cDNA, etc.) or to the amplification product after the amplification is completed. Means of attaching labels are well known and include, but are not limited to, nick attachment (ligation) of a nucleic acid linker joining the sample nucleic acid to a label (*e.g,* a fluorophore).

Detection labels suitable for use include any composition detectable by means of spectroscopic, photochemical, biochemical, immunochemical, electrical, optical, or chemical means. Commonly preferred labels include biotin for staining with labeled strepavidin conjugates, magnetic beads *(e.g.,* Dynabeads™, fluorescent dyes *(e.g.,* fluorescein, Texas red, rhodamine, green fluorescent protein and the like), radiolabels *(e.g.,* ³H, ¹²⁵I, ³²P, or ¹⁴C), enzymes *(e.g.,* horseradish peroxidase, alkaline phosphatase and the like), and colorimetric labels such as colloidal gold.

### 8.4 Hybridization and Detection

Hybridization of the probes is preferably at low stringency. Hybridization stringency includes for example, 20°C to about 50°C, more preferably 30°C to about 40°C, and most preferably about 37°C and 6X SSPE-T buffer (0.9 M NaCl, 60 mM Na₂HPO₄, 6 mM EDTA, 0.005% Triton X-100, pH 7.6) or less. Optimally washes can be performed thereafter at high stringency or progressively increasing stringency until a desired level of hybridization specificity is reached.

As the number of probes on a chip can be quite large, preferred chips may include only thoe probes that are needed, *e.g*, those probes relating to 16S or 23S. This will reduce the total number probes and the necessary size of the chip. This may also result in a different tiling strategy used for a particular chip design. For conceptual simplicity, probes are often arranged in order of the sequence in a lane across the chip. However, this tiling strategy is not required. Probes can be randomly placed on the chip. For discussion on tiling, *see* U.S. Pat. No. 6,228,575.

Probe length can vary. Probes usually have a single complementary segment having a length of at least 3 nucleotides, and more usually at least 5, 6, 7, 8, 9, 10, 11, 12, 13, 14, 15, 16, 17, 18, 19, 20, 21, 22, 23, 24, 25, 26, 27, 28, 29, or 30 bases exhibiting complimentarity.

In some chips, it may be optimal to have the probes all the same length, while in others it may be optimal to have probe sets of varying lenth. The probe length may vary by group or may vary by individual probe. For example, some chips may contain gour groups of probes having sizes of 20-mers, 22-mers, 25-mers and 30-mers. Other chips may have different size probes within the same group of four probes. Additional methods of designing chips comprising the distinguishing moieties or of the present invention would be known in the art. *See e.g.,* U.S. Pat. No. 6,228,575.

Additionally, the arrays are also contemplated as having other components than probes, for example linkers attaching the probes to a support.

### 8.5 Microarrays and Kits for Their Use

Many of these microarrays can be used as research tools, as diagnostics to determine the presence of a microorganism in a sample (*e.g.*, food sample or patient sample), as a means of following a patient's response to therapy and so forth. Thus these microarrays can be used in the form of kits. Kits can include an array of immobilized oligonucleotide probes complementary to subsequences of 16S and/or 23S. The kit may also include instructions describing the use of the array for the detectio nand/or quantification of expression levels of these sequences. The kit may optimally contain one or more of the following: buffers, hybridization mix, wash and read solutions, labels, labeling reagents (*e.g.*, enzymes), control nucleic acids, and software for probe selection, array reading or data analysis.

### 9. Spot Tests and Other High Through-Put Testing

Potential high through put testing methods include but are not limited to (a) The Nanosphere Spot Assay (Nanosphere Inc.) that uses proprietary gold nanoparticle probe technology for a colorimetric detection of amplified DNA sequences; (b) a new fluorescence *in situ* hybridization (FISH) method with peptide nucleic acid (PNA) probes for identification of bacteria, which is based on a fluorescein-labeled PNA probe that targets a species-specific sequence of the 16S rRNA of the target species *(e.g., S. aureus)* (Oliveira *et al.,* 2002 *J. Clin. Microbiol.* 40(1): 247-51); and c) a low-density microarray, in which a set of up to 1,000 probes are spotted on an array and tested with PCR products. Modifications of these would be readily apparent to the artisan of ordinary skill.

### 10. 16S Database Management System

Other aspects of the present invention involves a database management system for storing, maintaining, accessing and processing information relating to 16S rDNA sequences, where the information may be used to associate or distinguish moieties or residues from other moieties or residues based, for example, on nucleic acid sequence, location, relative position or any of a number of other characteristics.

Databases have been employed for storing, organizing and accessing biological data for quite some time. A database is a collection of information which has been organized in such a way that retrieval of the information stored therein is relatively quick and easy. In order to retrieve the information, however, a database management system is needed. A database management system is actually a collection of programs (*i. e.*, computer programs) that enables one to enter, organize, select and access the information. For the purposes of the present invention, the term database and database management system are used interchangeably. Also, for purposes of the present invention, the information entered, organized, selected and accessed, as stated above, relates to 16S rDNA sequences, and in particular see Tables 14, 15, 19, 20, and 21.

Databases are traditionally organized into fields, records and files. A field is a space, *e.g.*, a location in a memory device associated with one or more computer systems which make up the database management system, that has been allocated for a particular data item or piece of information. In general, a field is the smallest unit of information of data that one can access. A field may contain, for example, text information, such as a particular nucleic acid sequence; graphical information, such as data defining a three dimensional molecular representation of the nucleic acid sequence; or numeric information, such as the molecular weight of the nucleic acid sequence.

A collection of fields is generally referred to as a record. In the present invention, a single record may pertain to a particular full-length 16S rRNA/rDNA sequence or fragments thereof. The record may, in turn, contain several fields. As suggested above, one field may contain the nucleic acid sequence itself. Another field may contain the molecular weight of the sequence. Still another may contain data that defines a three dimensional molecular representation of the sequence. Yet another may define the classification of the microorganism (*e.g.*, the bacterium) from which the 16S rRNA/rDNA sequence was isolated *(i.e.,* the family, genus, species and/or strain of the microorganism). Still another may contain data that identifies the source of the sequence. In no way, however, is this list of examples intended to be exhaustive. In a relational database management system, records are often called tuples.

Generally speaking, a file is a collection of data or information that has been given a particular name, called a filename. In a database management system, a file may contain a collection of records. The records contained in that file may have a common attribute. For example, each record may contain 16S rRNA/rDNA data pertaining to or associated with a particular strain of bacteria.

The database management system of the present invention may be a relational database management system or, what is referred to as a flat-file database. A relational database is one that comprises multiple tables of data and/or information, where the word *table* simply refers to data arranged in rows and columns, and where the records contained in each table may have a different format. In a flat-file database, the data is contained in a single table. In accordance with exemplary embodiments of the present invention, the 16S rRNA/rDNA sequence data and/or information may be contained in a single table or spread across multiple tables. In one specific instance, one table may contain newly isolated 16S rRNA/rDNA sequences, or fragments thereof, where the sequence data associated with this table may be referred to as an internal database. Another table may contain 16S rRNA/rDNA sequences retrieved from one or more publically available or external databases, such as GenBank or the Ribosomal Database Project (RDP) database.

A more detailed description will now follow, with respect to generating, analyzing, annotating, storing, organizing and utilizing the 16S rRNA/rDNA sequence data and/or information in the database management system of the present invention.
10.1 Generating Raw Sequence Data. Raw sequence data refers to unedited nucleic acid sequence information. Raw sequence data may be obtained by sequencing isolated or amplified 16S rDNA or rRNA that has been subjected to PCR or RT-PCR and made into DNA or cDNA respectively. Raw sequence data may also be obtained through a variety of other methods, including the acquisition of sequence data from external sources. The preferred method for generating raw sequence data from a biological sample includes the steps of: isolating genomic DNA, preparing a template by PCR and, therefrom, generating a corresponding nucleic acid sequence.
10.2 Automated Bioanalysis. Once raw sequence data is generated for a given sample, as described in section 10.1 above, the raw sequence reads are preferably edited and annotated before the information is entered *(i.e.,* stored) in the database. More specifically, the process maybe divided into two levels of processing: 1) editing raw sequence data and 2) annotating and organizing the edited sequences data. These processing steps are collective referred to herein as automated bioanalysis.
10.2.1 Editing. Automated bioanalysis processing of the 16S rRNA/rDNA sequence data prior to annotating and storing the data in the database according to exemplary embodiments of the present invention may involve, for example, sequence editing, sequence masking, clipping portions of sequences, and removing artifacts associated with cloning and sequencing, as would be apparent to one skilled in the art. Furthermore, the editing process may include the functional arrangement of sequences, for instance, through clustering and master clustering. Still further, the editing process may involve the use of existing sequences, that have been previously edited, to extend a sequence and identify other existing sequences related thereto.
10.2.2 Annotating and Organizing. After edited the raw sequences, the additional information related to a given sequence may be introduced by way of annotations. Annotations may include, as suggested above, information relating to the classification of the microorganism (e.g., the bacterium) from which the 16S rRNA/rDNA sequence was isolated *(i.e.,* the family, genus, species and/or strain of the microorganism), the molecular weight of the sequence and the name(s) associated with the group responsible for isolating the sequence. Additionally, annotations may include information that relates a given sequence to other sequences within a given classification or classifications, disease/phenotype or disease relationship, pathology, histology or epidemiology relationships and methods used in editing the sequence.
   The sequence information and the associated annotations pertaining to a given sequence are stored in the database. The annotations, assigned through the automated bioanalysis, may contain information on the bacterium from which the genes encoding the rRNA are expressed, the relationship of the bacterium to other members of the genus, species and/or strain, and preparation techniques. The sequences from rDNA libraries are preferably organized by bacterium category.
10.3 Database Organization. The database of the present system utilizes the capabilities of modem computers by storing genetic information in association with a large amount of related information. In a preferred embodiment, the information on essentially all the steps of isolating and amplifying DNA, and identifying rRNA or rDNA sequences comprising distinguishing moieties are stored in various relational tables. The database can also allow a user to access information pertinent to the sequences.
   Both sequences and information annotating the sequences are stored in a database such as a relational database. Data is stored in the database in a functional arrangement that allows the user to store, track, and manipulate the rRNA or rDNA sequences and annotated information comprising information regarding distinguishing moieties. Users can access one or more databases via an integrated network, *e.g.* an Ethernet network. The workstations are typically computers, preferably personal computers, that include data entry means, output devices, display, CPU, memory (*i.e.*, RAM and ROM) and interfaces to the network.
   In the preferred embodiment of the present invention the relational database is stored at a file server connected to network. Computers are linked, via an integrated network, to a computer that grants access to the storage unit of the internal database of the present invention. The access computer preferably includes CPU, a memory means, interfaces to the network, and input and output devices. Reference databases illustrate sources of data that, for example, may be searched during use of the database. See for example, Table 7, which can be used as a reference database. Such a database is preferably in a relational form with a means, typically computerized, for querying the reference database. Tables 12, 14, 15, 20, and 21 comprise data that can be related within the database
10.4 Access to the Curated Database. The curated database preferably has a user-friendly interface, which is preferably created in HTML for access with Web browsers (e.g., Netscape® or Internet Explorer®).
10.5 Exemplary Full-length Sequences Stored in the Database. The sequences stored within the database provide information useful for designing probes to reveal the identity of various bacteria. Information of this nature is extremely powerful, as it can be utilized in clinical diagnostics, prognostics, patient treatment, etc. For example, certain types of bacterial species and/or strains may be resistant to some antibiotics, but may respond to other antibiotics. Determining this information would be clinically useful in prescribing the correct drug to treat the animal subject.
10.6 Use of the Internal Database. The structure and methods of data entry of the database allow many different types of analysis to be performed, both within the internal database and between sequences in the internal database and sequences in publicly available databases. The automated bioanalysis of the sequences enhances this analysis by masking or removing sequence elements that may hinder meaningful comparisons. The organization of the database facilitates analysis by providing mechanisms by which queries may be done quickly and efficiently, both within the internal database and with other external databases. The relational nature of the internal database thus provides a more comprehensive analysis, without the need to reformulate each search for each separate database.
10.6.1 Query Sequence Comparison. DNA sequence comparisons can involve comparing sequences within the internal database or comparing sequences with those in external databases.
   Data relating to sequence comparison is organized and stored in the sequence comparison portion of the database. This storage area includes tables containing information about the quality of the sequence matches in sequence match logs, as well as tables containing information about other features of compared sequences. The sequence comparison portion also contains information found during accession of external databases (*e.g.*, GenBank, RDP, PathoGenome™ (Genome Therapeutics Corp.), GenSeq (Derwent)). These databases may provide information on similarity, or rRNA domains, of the compared sequences that may be predictive of activity.
   A sequence comparison results in a text file with details and summaries with respect to the possible relationships between the query sequence and any database sequences identified through the comparison. Preferably, comparisons are done with the aid of algorithms and software such as BLAST, FASTA, Boyer-Moore, or Smith-Waterman. Most preferably, parameters are used which permit the identification of highly related sequences with 0, 1, or more mismatches with the query sequence as discussed herein.
10.6.2 Species and Group Specific Oligonucleotide Generation. The full-length gene sequence can be split into various segment sizes with a given overlap length. These segments are then compared by, for example, BLAST against sequence databases. The databases that can be used for the pairwise comparisons of the oligonucleotides include GenBank, Ribosomal Database Project (RDP), PathoGenome™ and the internal reference database as described herein and in the Examples. The reports produced by the BLAST comparisons are then parsed to determine which oligonucleotides are either species or group-specific. The criterion for a species-specific oligonucleotide is that it exhibit only 0 or 1 mismatch when its sequence is aligned with at least one sequence from a single species (or no species if no additional sequences are available for the particular species under study) and exhibit two or more mismatches when its sequence is aligned with those from any other species.. In other words, to be recognized as a "species-specific oligonucleotide" for a first species, an oligonucleotide hit to a second species must contain at least two mismatches. Likewise, a group-specific oligonucleotide is defined using the same mismatch criteria, but permitting 0 or 1 mismatch for five or fewer species. To better determine the validity of the species or groups-specific oligonucleotides, one can use multiple database comparisons because the content of different databases may not overlap entirely. The validity of the group- or species-specific oligonucleotide sequence is improved by comparison to a larger number of sequences from different databases.

Although the present invention has been described in detail with reference to examples below, it is understood that various modifications can be made without departing from the spirit of the invention, and would be readily known to the skilled artisan.

### EXAMPLES

### EXAMPLE 1

### Sequencing of 16S rRNA

Direct sequencing of uncloned PCR generated template for variant sequence discovery is described by, for example, Wrischnik et al., 1987 *Nuc. Acids Res.* 15(2): 529-42; Gibbs *et al.,* 1989 *Proc. Nat'l Acad. Sci. USA* 86(6): 1919-23; and Rogall *et al.,* 1990 *J. Gen. Microbiol.* 136(Pt 9): 1915-20. This method has been applied to both prokaryotic and eukaryotic systems. Software to support this process is widely available (Nickerson *et al.,* 1997 *Nuc. Acids Res.* 25(14): 2745-51). We have adapted this strategy for discovery of 16S RNA variation in numerous bacterial species.

PCR primers for amplification of the 16S rRNA gene were designed using the *E*. *coli* (ATCC11775) and *S. aureus* (ATCC12066) 16S rDNA sequences. Three tiers of amplicons were designed for complete coverage of the 16S gene as demonstrated in Fig. 1. Tier one has three overlapping fragments. Tier two has two fragments, and tier three has a single fragment. Primer sequences are noted in Tables 4-6 below.

**TABLE 4**

| PCR Fragment Defining Primers | |
|---|---|
| **PCR Fragment** | **Defining Primers** |
| Tier 1 #1 | 0008MF and 0522MR |
| Tier 1 #2 | 0514MF and 1073MR |
| Tier 1 #3 | 1062MF and 1540MR |
| Tier 2 #4 | 0008MF and 0800MR |
| Tier 2 #5 | 0775MF and 1540MR |
| Tier 3 #6 | 0008MF and 1540MR |

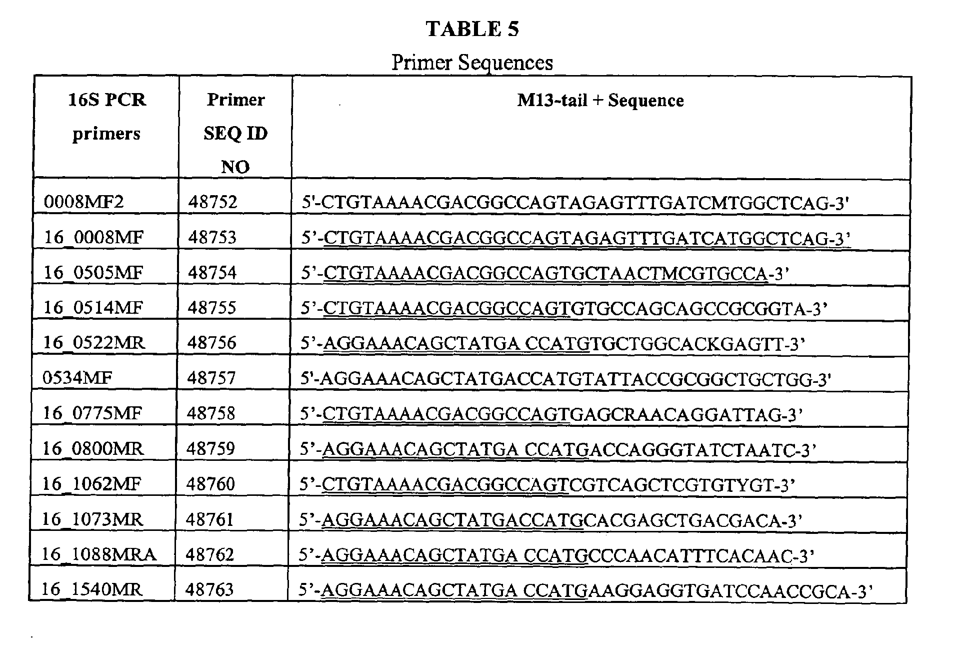

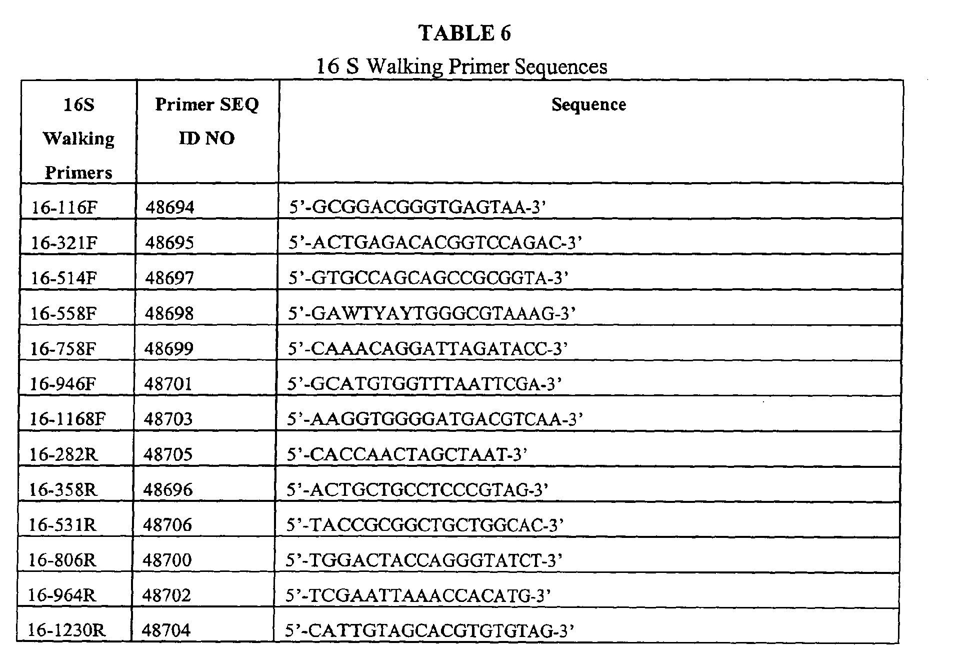

Forward PCR primers are tailed with the M13 -21 sequence and reverse primers with the M13 -28 sequence to facilitate production sequencing. In addition, fourteen walking primers are available for use on the tier three amplicon. These primers are used to generate sequence reads directly from the 1,400 nucleotides (nt) fragment. All sequence reads are generated with BigDye Terminator chemistry (Applied Biosystems, Foster City, CA) run on MegaBACE 1000 instruments (Amersham, Piscataway, NJ). Sequence reads representing the 16S sequence of individual bacterial isolates and passing Phred (Ewing *et al.,* 1998, *Genome Res.* 8: 175-185; and Ewing *et al.,* 1998 *Genome Res.* 8: 186-194) quality criteria of 175 total 30-quality bases, or 75 contiguous 30-quality bases, are assembled using polyphred. The assemblies are considered complete when a consensus sequence of at least 1,380 nt with an average of at least 3.5-fold Phred 20-quality data coverage is achieved.

A summary of all sequences that meet the above criteria was generated, along with information on the origin of the isolate from which each sequence was generated (the "source species"), and is represented in Table 7. Each strain, from which 16S rDNA sequence was obtained, was either a clinical isolate from the bioMérieux bacterial collection or was obtained from a reference collection center such as the ATCC. For all "type strains", a "T" has been added after the strain collection number (*e.g.*, ATCC 25238T).

**TABLE 7**

| **Source Name** | **Assembly ID** | **Collection ID** | **Collection #** |
|---|---|---|---|
| *Klebsiella oxytoca* | 01A01 | ATCC | 43863 |
| *Escherichia coli* | 01A02 | ATCC | 11775T |
| *Citrobacter freundii* | 01A03 | ATCC | 8090T |
| *Klebsiella pneumoniae* | 01A04 | ATCC | 13883T |
| *Enterobacter cloacae* | 01A05 | ATCC | 13047T |
| *Pseudomonas aeruginosa* | 01A06 | ATCC | 10145T |
| *Serratia marcescens* | 01A07 | ATCC | 13880T |
| *Klebsiella pneumoniae* | 01A08 | ATCC | 35657 |
| *Klebsiella pneumoniae* | 01A09 | ATCC | 11296T |
| *Citrobacter koseri* | 01A10 | ATCC | 27156 |
| *Morganella morganii ssp morganii* | 01A11 | ATCC | 25830T |
| *Staphylococcus haemolyticus* | 01B02 | ATCC | 29970T |
| *Citrobacter freundii* | 01B03 | ATCC | 43864 |
| *Streptococcus dysgalactiae* ssp. *Equisimilis* | 01B04 | ATCC | 35666 |
| *Streptococcus agalactiae* | 01B05 | ATCC | 13813T |
| *Achromobacter xylosoxidans* spp. *Denitrificans* | 01B06 | ATCC | 15173T |
| *Achromobacter xylosoxidans* spp.*xylosoxidans* | 01B07 | ATCC | 27061T |
| *Kocuria rosea* | 01B08 | ATCC | 35658 |
| *Streptococcus sanguinis* | 01B09 | ATCC | 10556T |
| *Kocuria rosea* | 01B10 | CCM | 2607 |
| *Streptococcus pneumoniae* | 01B11 | ATCC | 33400T |
| *Enterococcus avium* | 01B12 | ATCC | 14025T |
| *Enterobacter amnigenus* | 01C02 | ATCC | 33072T |
| *Stenotrophomonas maltophilia* | 01C03 | CCM | 2656 |
| *Moraxella osloensis* | 01C04 | ATCC | 19976T |
| *Streptococcus oralis* | 01C05 | ATCC | 35037T |
| *Fusobacterium necrophorum* | 01C06 | ATCC | 25286T |
| *Leclercia adecarboxylata* | 01C07 | ATCC | 23216T |
| *Stenotrophomonas maltophilia* | 01C08 | ATCC | 17666T |
| *Pantoea agglomerans* | 01C09 | ATCC | 27155T |
| *Staphylococcus epidermidis* | 01C10 | ATCC | 14990T |
| *Staphylococcus hominis* ssp. *Hominis* | 01C11 | ATCC | 27844T |
| *Prevotella melaninogenica* | 01C12 | none | none |
| *Staphylococcus haemolyticus* | 01D01 | none | none |
| *Staphylococcus aureus* ssp. *Aureus* | 01D02 | ATCC | 12600T |
| *Klebsiella pneumoniae* | 01D03 | none | none |
| *Campylobacter jejuni* | 01D04 | ATCC | 33560T |
| *Streptococcus iniae* | 01D05 | ATCC | 29178T |
| *Streptococcus ferus* | 01D06 | ATCC | 33477T |
| *Comamonas testosteroni* | 01D07 | ATCC | 11996T |
| *Pseudomonas fluorescens* | 01D08 | none | none |
| *Providencia alcalifaciens* | 01D09 | none | none |
| *Clostridium clostridiiforme* | 01D10 | ATCC | 25537T |
| *Campylobacter jejuni* | 01D11 | ATCC | 29428 |
| *Escherichia hermannii* | 01D12 | none | none |
| *Serratia marcescens* | 01E01 | none | none |
| *Klebsiella oxytoca* | 01E03 | ATCC | 2170 |
| *Klebsiella oxytoca* | 01E04 | ATCC | 2263 |
| *Staphylococcus epidermidis* | 01E05 | CDC | 2007 |
| *Klebsiella oxytoca* | 01E06 | ATCC | 13182T |
| *Proteus mirabilis* | 01E07 | ATCC | 29906T |
| *Providencia stuartii* | 01E08 | ATCC | 29914T |
| *Stenotrophomonas maltophilia* | 01E09 | ATCC | 13637T |
| *Acinetobacter lwoffii* | 01E11 | ATCC | 15309T |
| *Pantoea agglomerans* | 01E12 | none | none |
| *Pseudomonas stutzeri* | 01F01 | ATCC | 17588T |
| *Pasteurella multocida ssp multocida* | 01F02 | ATCC | 43137T |
| *Bacillus cereus* | 01F03 | ATCC | 14579T |
| *Salmonella choleraesuis* | 01F04 | ATCC | 19940 |
| *Acinetobacter haemolyticus* | 01F05 | none | none |
| *Citrobacter freundii* | 01F06 | none | none |
| *Citrobacter youngae* | 01F07 | ATCC | 29935T |
| *Klebsiella pneumoniae* | 01F08 | CDC | 30 |
| *Klebsiella pneumoniae* | 01F09 | CDC | 31 |
| *Klebsiella pneumoniae* | 01F10 | CDC | 40 |
| *Klebsiella oxytoca* | 01F11 | CDC | 44 |
| *Klebsiella oxytoca* | 01F12 | CDC | 46 |
| *Citrobacter freundii* | 01G01 | CDC | 85 |
| *Enterobacter cloacae* | 01G02 | aide diagnostic | none |
| *Enterobacter* sp. | 01G03 | aide diagnostic | none |
| *Pseudomonas aeruginosa* | 01G04 | aide diagnostic | none |
| *Citrobacter freundii* | 01G05 | aide diagnostic | none |
| *Pasteurella* sp. | 01G06 | aide diagnostic | none |
| *Escherichia coli* | 01G07 | aide diagnostic | none |
| *Pasteurella* sp. | 01G08 | aide diagnostic | none |
| *Brevibacillus thermoruber* | 01G09 | aide diagnostic | none |
| *Citrobacter freundii* | 01G10 | aide diagnostic | none |
| *Klebsiella pneumoniae* | 01G11 | aide diagnostic | none |
| *Raoultella planticola* | 01H01 | ATCC | 33531T |
| *Listeria seeligeri* | 01H02 | aide diagnostic | none |
| *Citrobacter freundii* | 01H03 | aide diagnostic | none |
| *Escherichia coli* | 01H04 | aide diagnostic | none |
| *Vibrio hollisae* | 01H05 | aide diagnostic | none |
| *Raoultella planticola* | 01H06 | none | none |
| *Streptococcus* ssp. | 01H07 | aide diagnostic | none |
| *Enterococcus faecalis* | 01H08 | aide diagnostic | none |
| *Staphylococcus aureus ssp.aureus* | 01H09 | aide diagnostic | none |
| *Salmonella enteritidis* | 01H10 | none | none |
| *Salmonella typhimurium* | 01H11 | none | none |
| *Fusobacterium mortiferum* | 01H12 | ATCC | 25557T |
| *Citrobacter freundii* | 03A01 | none | none |
| *Enterobacter aerogenes* | 03A02 | none | none |
| *Escherichia coli* | 03A03 | ATCC | 35421 |
| *Vibrio cholerae* | 03A04 | none | none |
| *Aeromonas caviae* | 03A05 | ATCC | 15468T |
| *Aeromonas hydrophila* | 03A06 | none | none |
| *Aeromonas sobria* | 03A07 | none | none |
| *Enterobacter cloacae* | 03A08 | none | none |
| *Staphylococcus epidermidis* | 03A09 | none | none |
| *Campylobacter jejuni* | 03A10 | none | none |
| *Enterococcus durans* | 03A11 | NCDO | 1724 |
| *Enterococcus hirae* | 03A12 | none | none |
| *Citrobacter koseri* | 03B01 | none | none |
| *Myroides odoratus* | 03B02 | none | none |
| *Pseudomonas aeruginosa* | 03B03 | CDC | none |
| *Vibrio cholerae* | 03B04 | none | none |
| *Aeromonas caviae* | 03B05 | none | none |
| *Aeromonas caviae* | 03B06 | none | none |
| *Aeromonas hydrophila* | 03B07 | none | none |
| *Aeromonas hydrophila* | 03B08 | ATCC | 7966T |
| *Streptococcus bovis* | 03B09 | none | none |
| *Clostridium perfringens* | 03B10 | none | none |
| *Enterococcus faecalis* | 03B11 | none | none |
| *Streptococcus bovis* | 03B12 | ATCC | 33317T |
| *Enterobacter cloacae* | 03C01 | none | none |
| *Citrobacter freundii* | 03C02 | none | none |
| *Citrobacter freundii* | 03C03 | none | none |
| *Citrobacter koseri* | 03C04 | none | none |
| *Citrobacter koseri* | 03C05 | none | none |
| *Enterobacter aerogenes* | 03C06 | ATCC | 13048T |
| *Enterobacter aerogenes* | 03C07 | none | none |
| *Escherichia coli* | 03C08 | CDC | 2039 |
| *Streptococcus equinus* | 03C09 | none | none |
| *Enterococcus durans* | 03C10 | none | none |
| *Enterococcus faecium* | 03C11 | none | none |
| *Corynebacterium jeikeium* | 03C12 | none | none |
| *Escherichia coli* | 03D01 | none | none |
| *Myroides odoratus* | 03D02 | none | none |
| *Pseudomonas aeruginosa* | 03D03 | ATCC | 35422 |
| *Stenotrophomonas maltophilia* | 03D04 | none | none |
| *Acinetobacter baumannii* | 03D05 | none | none |
| *Acinetobacter baumannii* | 03D06 | none | none |
| *Acinetobacter baumannii* | 03D07 | ATCC | 19606T |
| *Aeromonas sobria* | 03D08 | none | none |
| *Enterococcus faecalis* | 03D09 | ATCC | 19433T |
| *Enterococcus faecalis* | 03D10 | none | none |
| *Enterococcus faecium* | 03D11 | none | none |
| *Streptococcus equinus* | 03D12 | ATCC | 9812T |
| *Aeromonas sobria* | 03E01 | ATCC | 43979T |
| *Burkholderia cepacia* | 03E02 | none | none |
| *Burkholderia gladioli* | 03E03 | ATCC | 10248T |
| *Chryseobacterium meningosepticum* | 03E04 | ATCC | 13253T |
| *Myroides odoratus* | 03E05 | ATCC | 4651T |
| *Pseudomonas aeruginosa* | 03E06 | none | none |
| *Pseudomonasfluorescens* | 03E07 | none | none |
| *Stenotrophomonas maltophilia* | 03E08 | none | none |
| *Corynebacterium jeikeium* | 03E09 | none | none |
| *Enterococcus faecium* | 03E10 | ATCC | 19434T |
| *Haemophilus influenzae* | 03E11 | ATCC | 33391T |
| *Streptococcus bovis* | 03E12 | none | none |
| *Stenotrophomonas maltophilia* | 03F01 | none | none |
| *Vibrio cholerae* | 03F02 | ATCC | 14035T |
| *Burkholderia cepacia* | 03F03 | none | none |
| *Burkholderia cepacia* | 03F04 | ATCC | 25416T |
| *Burkholderia gladioli* | 03F05 | none | none |
| *Haemophilus parainfluenzae* | 03F08 | ATCC | 33392T |
| *Corynebacterium jeikeium* | 03F09 | none | none |
| *Enterococcus hirae* | 03F10 | ATCC | 8043T |
| *Haemophilus parainfluenzae* | 03F11 | none | none |
| *Streptococcus equinus* | 03F12 | NCTC | 10386 |
| *Neisseria meningitidis* | 03G01 | none | none |
| *Pseudomonas fluorescens* | 03G02 | none | none |
| *Pseudomonas fluorescens* | 03G03 | ATCC | 13525T |
| *Haemophilus influenzae* | 03G04 | none | none |
| *Haemophilus paraphrophilus* | 03G05 | ATCC | 29241T |
| *Haemophilus paraphrophilus* | 03G06 | none | none |
| *Haemophilus paraphrophilus* | 03G07 | none | none |
| *Neisseria meningitidis* | 03G08 | ATCC | 13077T |
| *Enterococcus hirae* | 03G09 | none | none |
| *Haemophilus influenzae* | 03G10 | none | none |
| *Clostridium perfringens* | 03G11 | ATCC | 13124T |
| *Staphylococcus aureus* ssp. *aureus* | 03H01 | none | none |
| *Staphylococcus aureus* ssp. *aureus* | 03H02 | none | none |
| *Staphylococcus epidermidis* | 03H03 | none | none |
| *Staphylococcus epidermidis* | 03H04 | none | none |
| *Neisseria meningitidis* | 03H05 | ATCC | 13090 |
| *Campylobacter coli* | 03H06 | ATCC | 33559T |
| *Campylobacter coli* | 03H07 | none | none |
| *Campylobacter coli* | 03H08 | none | none |
| *Enterococcus durans* | 03H09 | ATCC | 19432T |
| *Staphylococcus aureus* ssp. *aureus* | 03H10 | none | none |
| *Clostridium perfringens* | 03H11 | none | none |
| *Achromobacter piechaudii* | 04A01 | ATCC | 43552T |
| *Achromobacter xylosoxidans* spp. *denitrificans* | 04A02 | N/A | N/A |
| *Achromobacter xylosoxidans* spp. *xylosoxidans* | 04A03 | N/A | N/A |
| *Acinetobacter calcoaceticus* | 04A04 | ATCC | 14987 |
| *Acinetobacter calcoaceticus* | 04A05 | ATCC | 23055T |
| *Acinetobacter haemolyticus* | 04A06 | ATCC | 17906T |
| *Acinetobacter johnsonii* | 04A07 | N/A | N/A |
| *Acinetobacter johnsonii* | 04A08 | ATCC | 17909T |
| *Acinetobacter junii* | 04A09 | N/A | N/A |
| *Acinetobacter junii* | 04A10 | ATCC | 17908T |
| *Acinetobacter lwoffii* | 04A11 | none | none |
| *Actinobacillus ureae* | 04A12 | ATCC | 25976T |
| *Actinobacillus ureae* | 04B01 | ATCC | 29692 |
| *Aeromonas schubertii* | 04B02 | ATCC | 43700T |
| *Aeromonas schubertii* | 04B03 | ATCC | 43701 |
| *Aeromonas veronii* | 04B04 | ATCC | 35624T |
| *Aeromonas veronii* | 04B05 | N/A | N/A |
| *Alcaligenes faecalis* | 04B06 | ATCC | 8750T |
| *Alcaligenes faecalis* | 04B07 | N/A | N/A |
| *Bordetella avium* | 04B11 | ATCC | 35086T |
| *Bordetella bronchiseptica* | 04B12 | ATCC | 19395T |
| *Bordetella bronchiseptica* | 04C01 | ATCC | 10580 |
| *Bordetella trematum* | 04C02 | LMG | 5894 |
| *Bordetella trematum* | 04C03 | LMG | 13506T |
| *Brevundimonas diminuta* | 04C04 | N/A | N/A |
| *Brevundimonas diminuta* | 04C05 | ATCC | 11568T |
| *Brevundimonas vesicularis* | 04C06 | N/A | N/A |
| *Brevundimonas vesicularis* | 04C07 | ATCC | 11426T |
| *Budvicia aquatica* | 04C08 | ATCC | 51341 |
| *Budvicia aquatica* | 04C09 | ATCC | 35567T |
| *Buttiauxella agrestis* | 04C10 | ATCC | 33320T |
| *Buttiauxella agrestis* | 04C11 | CUETM | 78-27 |
| *Cedecea davisae* | 04C12 | ATCC | 33431T |
| *Cedecea davisae* | 04D01 | ATCC | 43024 |
| *Cedecea lapagei* | 04D02 | ATCC | 33432T |
| *Cedecea lapagei* | 04D03 | ATCC | 43028 |
| *Cedecea neteri* | 04D04 | ATCC | 33856 |
| *Cedecea neteri* | 04D05 | ATCC | 33855T |
| *Chromobacterium violaceum* | 04D06 | ATCC | 12472T |
| *Chromobacterium violaceum* | 04D07 | ATCC | 7461 |
| *Citrobacter amalonaticus* | 04D10 | ATCC | 25405T |
| *Citrobacter amalonaticus* | 04D11 | N/A | N/A |
| *Citrobacter braakii* | 04D12 | N/A | N/A |
| *Citrobacter braakii* | 04E01 | ATCC | 51113T |
| *Citrobacter farmeri* | 04E02 | ATCC | 51112T |
| *Citrobacter farmeri* | 04E03 | CDC | 2604-78 |
| *Citrobacter sedlakii* | 04E04 | ATCC | 51115T |
| *Citrobacter sedlakii* | 04E05 | CDC | 3659-74 |
| *Citrobacter werkmanii* | 04E06 | ATCC | 51114T |
| *Citrobacter werkmanii* | 04E07 | CDC | 631-77 |
| *Citrobacter youngae* | 04E08 | ATCC | 29935T |
| *Citrobacter youngae* | 04E09 | CDC | 6440-59 |
| *Comamonas testosteroni* | 04E10 | N/A | N/A |
| *Delftia acidovorans* | 04E11 | ATCC | 15668T |
| *Delftia acidovorans* | 04E12 | ATCC | 51340 |
| *Edwardsiella hoshinae* | 04F01 | ATCC | 33379T |
| *Edwardsiella hoshinae* | 04F02 | ATCC | 35050 |
| *Edwardsiella tarda* | 04F03 | ATCC | 15947T |
| *Empedobacter brevis* | 04F05 | ATCC | 43319T |
| *Empedobacter brevis* | 04F06 | N/A | N/A |
| *Enterobacter amnigenus* | 04F07 | ATCC | 33072T |
| *Enterobacter amnigenus* | 04F08 | CUETM | 78-70 |
| *Enterobacter asburiae* | 04F09 | N/A | N/A |
| *Enterobacter asburiae* | 04F10 | ATCC | 35953T |
| *Enterobacter cancerogenus* | 04F11 | CDC | 4641-84 |
| *Enterobacter gergoviae* | 04F12 | ATCC | 33028T |
| *Enterobacter gergoviae* | 04G01 | N/A | N/A |
| *Enterobacter intermedius* | 04G02 | ATCC | 33110T |
| *Enterobacter intermedius* | 04G03 | ATCC | 33422 |
| *Moraxella nonliquefaciens* | 04G05 | ATCC | 17975 |
| *Ochrobactrum anthropi* | 04G06 | N/A | N/A |
| *Ochrobactrum anthropi* | 04G07 | ATCC | 49188T |
| *Proteus mirabilis* | 04G08 | ATCC | 35659 |
| *Proteus vulgaris* | 04G09 | ATCC | 13315T |
| *Proteus vulgaris* | 04G10 | ATCC | 6380 |
| *Providencia stuartii* | 04G11 | N/A | N/A |
| *Pseudomonas stutzeri* | 04G12 | N/A | N/A |
| *Ralstonia pickettii* | 04H01 | ATCC | 27511T |
| *Ralstonia pickettii* | 04H02 | N/A | N/A |
| *Salmonella choleraesuis* | 04H03 | ATCC | 13314T |
| *Salmonella choleraesuis* | 04H04 | N/A | N/A |
| *Serratia liquefaciens* | 04H05 | ATCC | 27592T |
| *Serratia liquefaciens* | 04H06 | N/A | N/A |
| *Shewanella algae* | 04H07 | N/A | N/A |
| *Klebsiella pneumoniae* | 04H08 | N/A | N/A |
| *Klebsiella pneumoniae* | 04H09 | N/A | N/A |
| *Klebsiella pneumoniae* | 04H10 | N/A | N/A |
| *Klebsiella pneumoniae* | 04H11 | N/A | N/A |
| *Klebsiella pneumoniae* | 04H12 | N/A | N/A |
| *Staphylococcus arlettae* | 05A01 | ATCC | 43957T |
| *Staphylococcus arlettae* | 05A02 | CCUG | 33610 |
| *Staphylococcus arlettae* | 05A03 | DSM | 20673 |
| *Staphylococcus auricularis* | 05A04 | ATCC | 33753T |
| *Staphylococcus auricularis* | 05A05 | none | none |
| *Staphylococcus auricularis* | 05A06 | ATCC | 33752 |
| *Staphylococcus capitis* ssp. *capitis* | 05A07 | ATCC | 27840T |
| *Staphylococcus capitis* ssp. *capitis* | 05A08 | none | none |
| *Staphylococcus capitis* ssp. *capitis* | 05A09 | ATCC | 27841 |
| *Staphylococcus capitis* ssp. *ureolyticus* | 05A10 | ATCC | 49326T |
| *Staphylococcus capitis* ssp. *ureolyticus* | 05A11 | ATCC | 49325 |
| *Staphylococcus capitis* ssp. *ureolyticus* | 05A12 | none | none |
| *Staphylococcus caprae* | 05B01 | ATCC | 35538T |
| *Staphylococcus caprae* | 05B02 | CCUG | 38378 |
| *Staphylococcus caprae* | 05B03 | none | none |
| *Staphylococcus carnosus* ssp. *carnosus* | 05B04 | ATCC | 51365T |
| *Staphylococcus carnosus* ssp. *carnosus* | 05B05 | LMG | 13567 |
| *Staphylococcus carnosus* ssp. *carnosus* | 05B06 | none | none |
| *Staphylococcus chromogenes* | 05B07 | ATCC | 43764T |
| *Staphylococcus chromogenes* | 05B08 | none | none |
| *Staphylococcus chromogenes* | 05B09 | none | none |
| *Staphylococcus cohnii ssp cohnii* | 05B10 | ATCC | 29974T |
| *Staphylococcus cohnii ssp cohnii* | 05B11 | none | none |
| *Staphylococcus cohnii ssp cohnii* | 05B12 | none | none |
| *Staphylococcus cohnii ssp urealyticum* | 05C01 | ATCC | 49330T |
| *Staphylococcus cohnii ssp urealyticum* | 05C02 | ATCC | 49331 |
| *Staphylococcus cohnii ssp urealyticum* | 05C03 | none | none |
| *Staphylococcus equorum* | 05C04 | ATCC | 43958T |
| *Staphylococcus equorum* | 05C05 | none | none |
| *Staphylococcus equorum* | 05C06 | none | none |
| *Staphylococcus gallinarum* | 05C07 | ATCC | 35539T |
| *Staphylococcus gallinarum* | 05C08 | none | none |
| *Staphylococcus gallinarum* | 05C09 | none | none |
| *Staphylococcus haemolyticus* | 05C10 | CDC | 2233 |
| *Staphylococcus haemolyticus* | 05C11 | none | none |
| *Staphylococcus haemolyticus* | 05C12 | none | none |
| *Staphylococcus hominis ssp hominis* | 05D01 | none | none |
| *Staphylococcus hominis* ssp. *novobiosepticus* | 05D02 | CCUG | 42399T |
| *Staphylococcus hominis* ssp. *novobiosepticus* | 05D03 | none | none |
| *Staphylococcus hominis* ssp. *novobiosepticus* | 05D04 | none | none |
| *Staphylococcus hyicus* | 05D05 | ATCC | 11249T |
| *Staphylococcus hyicus* | 05D06 | none | none |
| *Staphylococcus hyicus* | 05D07 | none | none |
| *Staphylococcus intermedius* | 05D08 | ATCC | 29663T |
| *Staphylococcus intermedius* | 05D09 | none | none |
| *Staphylococcus intermedius* | 05D10 | none | none |
| *Staphylococcus kloosii* | 05D11 | ATCC | 43959T |
| *Staphylococcus kloosii* | 05D12 | none | none |
| *Staphylococcus kloosii* | 05E01 | none | none |
| *Staphylococcus lentus* | 05E02 | ATCC | 29070T |
| *Staphylococcus lentus* | 05E03 | none | none |
| *Staphylococcus lentus* | 05E04 | none | none |
| *Staphylococcus lugdunensis* | 05E05 | ATCC | 43809T |
| *Staphylococcus lugdunensis* | 05E06 | none | none |
| *Staphylococcus lugdunensis* | 05E07 | none | none |
| *Staphylococcus pasteuri* | 05E08 | ATCC | 51129T |
| *Staphylococcus pasteuri* | 05E09 | CCUG | 32422 |
| *Staphylococcus pasteuri* | 05E10 | CCUG | 32421 |
| *Staphylococcus pulvereri* | 05E11 | ATCC | 51698T |
| *Staphylococcus pulvereri* | 05E12 | CCUG | 33939 |
| *Staphylococcus saccharolyticus* | 05F01 | NCDO | 1260 |
| *Staphylococcus saccharolyticus* | 05F02 | none | none |
| *Staphylococcus saccharolyticus* | 05F03 | none | none |
| *Staphylococcus saprophyticus* ssp. *bovis* | 05F04 | CCM | 4410T |
| *Staphylococcus saprophyticus* ssp. *bovis* | 05F05 | CIP | 105264 |
| *Staphylococcus saprophyticus* ssp. *bovis* | 05F06 | none | none |
| *Staphylococcus saprophyticus* ssp. *saprophyticus* | 05F07 | ATCC | 15305T |
| *Staphylococcus saprophyticus* ssp. *saprophyticus* | 05F08 | none | none |
| *Staphylococcus saprophyticus* ssp. *saprophyticus* | 05F09 | none | none |
| *Staphylococcus schleiferi* ssp. *coagulans* | 05F10 | CCUG | 37248T |
| *Staphylococcus schleiferi* ssp. *schleiferi* | 05F11 | ATCC | 43808T |
| *Staphylococcus schleiferi* ssp. *schleiferi* | 05F12 | none | none |
| *Staphylococcus schleiferi* ssp. *schleiferi* | 05G01 | none | none |
| *Staphylococcus sciuri* ssp. *sciuri* | 05G02 | ATCC | 29062T |
| *Staphylococcus sciuri* ssp. *sciuri* | 05G03 | ATCC | 29061 |
| *Staphylococcus sciuri* ssp. *sciuri* | 05G04 | none | none |
| *Staphylococcus simulans* | 05G05 | ATCC | 27848T |
| *Staphylococcus simulans* | 05G06 | none | none |
| *Staphylococcus simulans* | 05G07 | none | none |
| *Staphylococcus vitulinus* | 05G08 | none | none |
| *Staphylococcus vitulinus* | 05G09 | ATCC | 51145T |
| *Staphylococcus warneri* | 05G10 | ATCC | 27836T |
| *Staphylococcus warneri* | 05G11 | none | none |
| *Staphylococcus warneri* | 05G12 | none | none |
| *Staphylococcus xylosus* | 05H01 | ATCC | 29971T |
| *Staphylococcus xylosus* | 05H02 | none | none |
| *Staphylococcus xylosus* | 05H03 | none | none |
| *Staphylococcus capitis* | 05H04 | none | none |
| *Staphylococcus pulvereri* | 05H05 | CCUG | 33940 |
| *Staphylococcus caprae* | 05H06 | none | none |
| *Staphylococcus* spp. | 05H07 | none | none |
| *Staphylococcus* spp. | 05H08 | none | none |
| *Staphylococcus* spp. | 05H09 | none | none |
| *Streptococcus mitis* | 05H10 | none | none |
| *Streptococcus oralis* | 05H11 | none | none |
| *Staphylococcus* spp. | 05H12 | none | none |
| *Bacteroides eggerthii* | 06A01 | none | none |
| *Bacteroides thetaiotaomicron* | 06A02 | none | none |
| *Clostridium histolyticum* | 06A03 | ATCC | 19401T |
| *Clostridium botulinum* | 06A04 | none | none |
| *Clostridium butyricum* | 06A05 | ATCC | 19398T |
| *Clostridium septicum* | 06A06 | ATCC | 12464T |
| *Clostridium subterminale* | 06A07 | ATCC | 25774T |
| *Fusobacterium varium* | 06A08 | ATCC | 8501T |
| *Porphyromonas gingivalis* | 06A09 | ATCC | 33277T |
| *Prevotella melaninogenica* | 06A10 | ATCC | 25845T |
| *Prevotella oris* | 06A11 | ATCC | 33573T |
| *Bacteroides distasonis* | 06A12 | ATCC | 8503T |
| *Actinomyces odontolyticus* | 06B01 | ATCC | 17929T |
| *Actinomyces odontolyticus* | 06B02 | none | none |
| *Bacteroides caccae* | 06B03 | none | none |
| *Bacteroides caccae* | 06B04 | ATCC | 43185T |
| *Bacteroides distasonis* | 06B05 | none | none |
| *Bacteroides eggerthii* | 06B06 | ATCC | 27754T |
| *Bacteroides fragilis* | 06B07 | none | none |
| *Bacteroides merdae* | 06B08 | none | none |
| *Bacteroides ovatus* | 06B09 | none | none |
| *Bacteroides ovatus* | 06B10 | ATCC | 8483T |
| *Bacteroides stercoris* | 06B11 | none | none |
| *Actinomyces bovis* | 06B12 | none | none |
| *Actinomyces meyeri* | 06C01 | none | none |
| *Actinomyces naeslundii* | 06C02 | none | none |
| *Actinomyces viscosus* | 06C03 | ATCC | 15987T |
| *Actinomyces viscosus* | 06C04 | none | none |
| *Bacteroides stercoris* | 06C07 | ATCC | 43183T |
| *Bacteroides thetaiotaomicron* | 06C08 | none | none |
| *Bacteroides uniformis* | 06C09 | none | none |
| *Bacteroides uniformis* | 06C10 | ATCC | 8492T |
| *Bacteroides ureolyticus* | 06C11 | ATCC | 33387T |
| *Bacteroides vulgatus* | 06C12 | none | none |
| *Bacteroides vulgatus* | 06D01 | ATCC | 8482T |
| *Arcanobacterium pyogenes* | 06D10 | ATCC | 19411T |
| *Arcanobacterium pyogenes* | 06D11 | none | none |
| *Clostridium barati* | 06D12 | ATCC | 27638T |
| *Clostridium botulinum* | 06E01 | none | none |
| *Clostridium glycolicum* | 06E02 | none | none |
| *Clostridium histolyticum* | 06E03 | none | none |
| *Clostridium innocuum* | 06E04 | ATCC | 14501T |
| *Clostridium perfringens* | 06E05 | none | none |
| *Clostridium paraputrificum* | 06E06 | none | none |
| *Clostridium ramosum* | 06E07 | none | none |
| *Clostridium tertium* | 06E09 | none | none |
| *Clostridium tetani* | 06E10 | ATCC | 19406T |
| *Clostridium tetani* | 06E11 | none | none |
| *Collinsella aerofaciens* | 06E12 | ATCC | 25986T |
| *Collinsella aerofaciens* | 06F01 | none | none |
| *Eggerthella lenta* | 06F02 | none | none |
| *Eggerthella lenta* | 06F03 | ATCC | 25559T |
| *Eubacterium limosum* | 06F04 | ATCC | 8486T |
| *Lactobacillus acidophilus* | 06F05 | ATCC | 4356T |
| *Lactobacillus casei* | 06F07 | ATCC | 393T |
| *Bacteroides capillosus* | 06F08 | none | none |
| *Bacteroides capillosus* | 06F09 | ATCC | 29799T |
| *Fusobacterium mortiferum* | 06F10 | none | none |
| *Fusobacterium necrophorum* | 06F11 | none | none |
| *Fusobacterium nucleatum ssp nucleatum* | 06F12 | none | none |
| *Fusobacterium varium* | 06G01 | none | none |
| *Leptotrichia buccalis* | 06G02 | none | none |
| *Porphyromonas gingivalis* | 06G04 | none | none |
| *Porphyromonas levii* | 06G05 | ATCC | 29147T |
| *Porphyromonas maccae* | 06G06 | ATCC | 33141T |
| *Clostridium hastiforme* | 06G07 | ATCC | 33268T |
| *Clostridium innocuum* | 06G08 | none | none |
| *Clostridium septicum* | 06G09 | none | none |
| *Clostridium sporogenes* | 06G10 | none | none |
| *Clostridium sporogenes* | 06G11 | ATCC | 3584T |
| *Clostridium subterminale* | 06G12 | none | none |
| *Clostridium difficile* | 06H01 | none | none |
| *Clostridium difficile* | 06H02 | ATCC | 9689T |
| *Capnocytophaga gingivalis* | 06H03 | none | none |
| *Capnocytophaga gingivalis* | 06H04 | ATCC | 33624T |
| *Capnocytophaga ochracea* | 06H05 | none | none |
| *Capnocytophaga* spp. | 06H06 | none | none |
| *Eubacterium limosum* | 06H07 | none | none |
| *Peptoniphilus asaccharolyticus* | 06H08 | none | none |
| *Peptoniphilus asaccharolyticus* | 06H09 | ATCC | 14963T |
| *Peptostreptococcus anaerobius* | 06H10 | none | none |
| *Peptostreptococcus anaerobius* | 06H11 | ATCC | 27337T |
| *Peptoniphilus indolicus* | 06H12 | none | none |
| *Peptoniphilus indolicus* | 07A01 | ATCC | 29427T |
| *Finegoldia magna* | 07A02 | none | none |
| *Micromonas micros* | 07A03 | ATCC | 33270T |
| *Micromonas micros* | 07A04 | none | none |
| *Anaerococcus prevotii* | 07A05 | none | none |
| *Anaerococcus prevotii* | 07A06 | ATCC | 9321T |
| *Anaerococcus tetradius* | 07A07 | none | none |
| *Anaerococcus tetradius* | 07A08 | ATCC | 35098T |
| *Propionibacterium acnes* | 07A09 | none | none |
| *Propionibacterium granulosum* | 07B01 | ATCC | 25564T |
| *Propionibacterium propionicus* | 07B03 | none | none |
| *Rhodococcus equi* | 07B04 | none | none |
| *Porphyromonas asaccharolytica* | 07B06 | none | none |
| *Leptotrichia buccalis* | 07B07 | ATCC | 14201T |
| *Prevotella buccae* | 07B09 | ATCC | 33574T |
| *Prevotella corporis* | 07B10 | none | none |
| *Prevotella corporis* | 07B11 | ATCC | 33547T |
| *Prevotella denticola* | 07B12 | none | none |
| *Prevotella disiens* | 07C03 | ATCC | 29426T |
| *Clostridium clostridiiforme* | 07C05 | none | none |
| *Propionibacterium propionicus* | 07C06 | ATCC | 14157T |
| *Bacteroides ureolyticus* | 07C08 | none | none |
| *Prevotella oralis* | 07C10 | none | none |
| *Prevotella oris* | 07C11 | none | none |
| *Clostridium cadaveris* | 07C12 | none | none |
| *Clostridium butyricum* | 07D01 | none | none |
| *Clostridium sordellii* | 07D02 | none | none |
| *Fusobacterium necrogenes* | 07D03 | none | none |
| *Porphyromonas endodontalis* | 07D04 | ATCC | 35406T |
| *Prevotella intermedia* | 07D05 | ATCC | 25611T |
| *Prevotella oralis* | 07D08 | ATCC | 33269T |
| *Veillonella parvula* | 07D09 | none | none |
| *Veillonella parvula* | 07D10 | ATCC | 10790T |
| *Veillonella spp* | 07D11 | none | none |
| *Prevotella bivia* | 07D12 | none | none |
| *Cardiobacterium hominis* | 07E01 | none | none |
| *Haemophilus actinomycetemcomicans* | 07E02 | none | none |
| *Haemophilus aegyptius* | 07E03 | none | none |
| *Haemophilus aegyptius* | 07E04 | ATCC | 11116T |
| *Haemophilus aphrophilus* | 07E05 | none | none |
| *Haemophilus aphrophilus* | 07E06 | ATCC | 33389T |
| *Haemophilus ducreyi* | 07E07 | none | none |
| *Haemophilus haemolyticus* | 07E08 | none | none |
| *Haemophilus influenzae* | 07E09 | none | none |
| *Haemophilus influenzae* | 07E10 | none | none |
| *Haemophilus influenzae* | 07E11 | none | none |
| *Haemophilus paraphrophilus* | 07E12 | none | none |
| *Haemophilus paraphrophilus* | 07F01 | none | none |
| *Haemophilus parainfluenzae* | 07F02 | none | none |
| *Haemophilus parainfluenzae* | 07F03 | none | none |
| *Haemophilus segnis* | 07F04 | none | none |
| *Haemophilus segnis* | 07F05 | ATCC | 33393T |
| *Kingella denitrificans* | 07F06 | none | none |
| *Kingella denitrificans* | 07F07 | none | none |
| *Kingella kingae* | 07F08 | none | none |
| *Moraxella (Branhamella) catarrhalis* | 07F09 | none | none |
| *Moraxella (Branhamella) catarrhalis* | 07F10 | none | none |
| *Neisseria cinerea* | 07F11 | ATCC | 14685T |
| *Neisseria cinerea* | 07F12 | none | none |
| *Neisseria elongata* | 07G01 | none | none |
| *Neisseria flavescens* | 07G02 | ATCC | 13120T |
| *Neisseria flavescens* | 07G03 | none | none |
| *Neisseria gonorrhoeae* | 07G04 | none | none |
| *Neisseria gonorrhoeae* | 07G05 | ATCC | 19424T |
| *Neisseria lactamica* | 07G06 | ATCC | 23970T |
| *Neisseria lactamica* | 07G07 | none | none |
| *Neisseria meningitidis* | 07G08 | none | none |
| *Neisseria meningitidis* | 07G09 | none | none |
| *Neisseria mucosa* | 07G10 | none | none |
| *Neisseria mucosa* | 07G11 | ATCC | 19696T |
| *Neisseria subflava* | 07G12 | none | none |
| *Neisseria sicca* | 07H01 | none | none |
| *Neisseria weaveri* | 07H02 | none | none |
| *Suttonella indologenes* | 07H03 | none | none |
| *Cardiobacterium hominis* | 07H04 | ATCC | 15826T |
| *Suttonella indologenes* | 07H05 | ATCC | 25869T |
| *Cellulosimicrobium cellulans* | 07H06 | ATCC | 12830T |
| *Corynebacterium amycolatum* | 07H09 | ATCC | 49368T |
| *Corynebacterium amycolatum* | 07H10 | none | none |
| *Achromobacter piechaudii* | 08A01 | LMG | 6002 |
| *Rhizobium radiobacter* | 08A02 | none | none |
| *Proteus penneri* | 08A03 | ATCC | 33519T |
| *Enterobacter hormaechei* | 08A04 | ATCC | 49162T |
| *Escherichia vulneris* | 08A05 | ATCC | 33821T |
| *Klebsiella pneumoniae* | 08A06 | none | none |
| *Kluyvera cryocrescens* | 08A07 | ATCC | 14239 |
| *Moraxella atlantae* | 08A08 | ATCC | 29525T |
| *Morganella morganii ssp. sibonii* | 08A09 | ATCC | 49948T |
| *Enterococcus gallinarum* | 08A10 | ATCC | 49573T |
| *Oligella ureolytica* | 08A11 | ATCC | 43534T |
| *Providencia rettgeri* | 08A12 | CDC | 2163 |
| *Acinetobacter genospecies 3* | 08B01 | ATCC | 17922 |
| *CDC group EF-4B* | 08B02 | none | none |
| *Proteus penneri* | 08B03 | none | none |
| *Enterobacter hormaechei* | 08B04 | none | none |
| *Escherichia vulneris* | 08B05 | CCUG | 23001 |
| *Klebsiella pneumoniae* | 08B06 | ATCC | 13884T |
| *Leclercia adecarboxylata* | 08B07 | none | none |
| *Moraxella atlantae* | 08B08 | none | none |
| *Morganella morganii* ssp. *sibonii* | 08B09 | none | none |
| *Enterococcus gallinarum* | 08B10 | none | none |
| *Oligella ureolytica* | 08B11 | ATCC | 35578 |
| *Pasteurella multocida* ssp. *multocida* | 08B12 | none | none |
| *Acinetobacter genospecies 3* | 08C01 | ATCC | 19004 |
| *CDC group EF-4B* | 08C02 | none | none |
| *Enterobacter sakazakii* | 08C04 | ATCC | 29544T |
| *Ewingella americana* | 08C05 | ATCC | 33852T |
| *Klebsiella pneumoniae* | 08C06 | none | none |
| *Mannheimia haemolytica* | 08C07 | ATCC | 33396T |
| *Moraxella bovis* | 08C08 | ATCC | 10900T |
| *Enterococcus avium* | 08C09 | ATCC | 49602 |
| *Enterococcus raffinosus* | 08C10 | ATCC | 49427T |
| *Oligella urethralis* | 08C11 | ATCC | 17960T |
| *Pasteurella multocida* ssp. *gallicida* | 08C12 | ATCC | 51689T |
| *Acinetobacter radioresistens* | 08D01 | none | none |
| *CDC group EO-2* | 08D02 | none | none |
| *Enterobacter sakazakii* | 08D04 | ATCC | 51329 |
| *Ewingella americana* | 08D05 | CDC | 4048-83 |
| *Raoultella terrigena* | 08D06 | ATCC | 33257T |
| *Mannheimia haemolytica* | 08D07 | none | none |
| *Moraxella bovis* | 08D08 | ATCC | 17947 |
| *Enterococcus casseliflavus* | 08D09 | ATCC | 25788T |
| *Enterococcus raffinosus* | 08D10 | CDC | 2226 |
| *Oligella urethralis* | 08D11 | CCUG | 994 |
| *Pasteurella trehalosi* | 08D12 | ATCC | 29703T |
| *Acinetobacter radioresistens* | 08E01 | none | none |
| *CDC group EO-2* | 08E02 | none | none |
| *Comamonas terrigena* | 08E03 | CCUG | 15848 |
| *Escherichia fergusonii* | 08E04 | ATCC | 35469T |
| *Hafnia alvei* | 08E05 | ATCC | 13337T |
| *Raoultella terrigena* | 08E06 | none | none |
| *Moellerella wisconsensis* | 08E07 | ATCC | 35017T |
| *Moraxella lacunata* | 08E08 | ATCC | 17967T |
| *Enterococcus casseliflavus* | 08E09 | none | none |
| *Enterococcus saccharolyticus* | 08E10 | ATCC | 43076T |
| *Pantoea dispersa* | 08E11 | ATCC | 14589T |
| *Photobacterium damselae* | 08E12 | ATCC | 33539T |
| *Aeromonas salmonicida ssp salmonicida* | 08F01 | ATCC | 33658T |
| *CDC group VB-3* | 08F02 | none | none |
| *Comamonas terrigena* | 08F03 | CCUG | 15850 |
| *Escherichia fergusonii* | 08F04 | none | none |
| *Hafnia alvei* | 08F05 | none | none |
| *Kluyvera ascorbata* | 08F06 | ATCC | 33433T |
| *Moellerella wisconsensis* | 08F07 | ATCC | 35621 |
| *Moraxella lacunata* | 08F08 | none | none |
| *Enterococcus cecorum* | 08F09 | ATCC | 43198T |
| *Enterococcus saccharolyticus* | 08F10 | NCDO | 2614 |
| *Pantoea dispersa* | 08F11 | LMG | 2770 |
| *Photobacterium damselae* | 08F12 | CIP | 100540 |
| *Aeromonas salmonicida* ssp. *salmonicida* | 08G01 | none | none |
| *CDC group VB-3* | 08G02 | none | none |
| *Enterobacter cancerogenus* | 08G03 | ATCC | 35317 |
| *Escherichia hermannii* | 08G04 | ATCC | 33650T |
| *Raoultella ornithinolytica* | 08G05 | ATCC | 31898T |
| *Kluyvera ascorbata* | 08G06 | none | none |
| *Moraxella (Branhamella) catarrhalis* | 08G07 | ATCC | 25238T |
| *Moraxella osloensis* | 08G08 | none | none |
| *Enterococcus cecorum* | 08G09 | none | none |
| *Staphylococcus hominis* ssp. *hominis* | 08G10 | none | none |
| *Pasteurella aerogenes* | 08G11 | ATCC | 27883T |
| *Plesiomonas shigelloides* | 08G12 | ATCC | 14029T |
| *Rhizobium radiobacter* | 08H01 | ATCC | 23308T |
| *CDC group VB-3* | 08H02 | none | none |
| *Providencia alcalifaciens* | 08H03 | ATCC | 9886T |
| *Providencia rettgeri* | 08H04 | ATCC | 29944T |
| *Raoultella ornithinolytica* | 08H05 | none | none |
| *Kluyvera cryocrescens* | 08H06 | ATCC | 33435T |
| *Moraxella (Branhamella) catarrhalis* | 08H07 | none | none |
| *Morganella morganii* ssp. *morganii* | 08H08 | none | none |
| *Enterococcus dispar* | 08H09 | CCUG | 33309T |
| *Ochrobactrum intermedium* | 08H10 | LMG | 3301T |
| *Pasteurella aerogenes* | 08H11 | none | none |
| *Plesiomonas shigelloides* | 08H12 | none | none |
| *Aeromonas salmonicida* ssp. *achromogenes* | 09A01 | ATCC | 33659T |
| *Weeksella virosa* | 09A02 | CIP | 81.91 |
| *Pasteurella multocida* ssp. *septica* | 09A03 | ATCC | 51687T |
| *Pseudomonas luteola* | 09A04 | ATCC | 43273T |
| *Pseudomonas putida* | 09A05 | ATCC | 12633T |
| *Ralstonia paucula* | 09A06 | none | none |
| *Serratia odorifera* | 09A07 | none | none |
| *Serratia rubidaea* | 09A08 | none | none |
| *Yersinia pseudotuberculosis* | 09A09 | none | none |
| *Vibrio alginolyticus* | 09A11 | none | none |
| *Vibrio metschnikovii* | 09A12 | CCUG | 7491 |
| *Aeromonas salmonicida* ssp. *achromogenes* | 09B01 | none | none |
| *Xanthomonas campestris* | 09B02 | ATCC | 33913T |
| *Pasteurella pneumotropica* | 09B03 | ATCC | 35149T |
| *Pseudomonas luteola* | 09B04 | none | none |
| *Pseudomonas putida* | 09B05 | none | none |
| *Serratia ficaria* | 09B06 | ATCC | 33105T |
| *Serratia plymuthica* | 09B07 | ATCC | 183T |
| *Shewanella algae* | 09B08 | ATCC | 51192T |
| *Shigella flexneri* | 09B09 | ATCC | 29903T |
| *Vibrio fluvialis* | 09B11 | ATCC | 33809T |
| *Vibrio mimicus* | 09B12 | ATCC | 33653T |
| *Aeromonas salmonicida* ssp. *masoucida* | 09C01 | ATCC | 27013T |
| *Xanthomonas campestris* | 09C02 | ATCC | 35938 |
| *Pasteurella pneumotropica* | 09C03 | ATCC | 12555 |
| *Pseudomonas mendocina* | 09C04 | ATCC | 25411T |
| *Psychrobacter phenylpyruvicus* | 09C05 | ATCC | 23333T |
| *Serratia ficaria* | 09C06 | ATCC | 4092-83 |
| *Serratia plymuthica* | 09C07 | none | none |
| *Shewanella algae* | 09C08 | none | none |
| *Shigella flexneri* | 09C09 | none | none |
| *Yersinia bercovieri* | 09C10 | ATCC | 43970T |
| *Vibrio fluvialis* | 09C11 | none | none |
| *Vibrio mimicus* | 09C12 | CCUG | 13176 |
| *Aeromonas salmonicida* ssp. *masoucida* | 09D01 | none | none |
| *Yersinia aldovae* | 09D02 | ATCC | 35236T |
| *Pasteurella trehalosi* | 09D03 | none | none |
| *Pseudomonas mendocina* | 09D04 | none | none |
| *Rahnella aquatilis* | 09D05 | ATCC | 33071T |
| *Serratia fonticola* | 09D06 | ATCC | 29844T |
| *Yersinia bercovieri* | 09D07 | CIP | 103327 |
| *Shewanella putrefaciens* | 09D08 | ATCC | 8071T |
| *Shigella sonnei* | 09D09 | ATCC | 29930T |
| *Sphingomonas paucimobilis* | 09D10 | ATCC | 29837T |
| *Vibrio harveyi* | 09D11 | ATCC | 14126T |
| *Vibrio parahaemolyticus* | 09D12 | ATCC | 17802T |
| *Citrobacter gillenii* | 09E01 | ATCC | 51117T |
| *Yersinia aldovae* | 09E02 | CIP | 104234 |
| *Providencia rustigianii* | 09E03 | ATCC | 33673T |
| *Pseudomonas oryzihabitans* | 09E04 | ATCC | 43272T |
| *Rahnella aquatilis* | 09E05 | ATCC | 33392 |
| *Serratia fonticola* | 09E06 | ATCC | 29846 |
| *Yersinia enterocolitica* ssp. *enterocolitica* | 09E07 | ATCC | 9610T |
| *Shewanella putrefaciens* | 09E08 | none | none |
| *Shigella sonnei* | 09E09 | none | none |
| *Sphingomonas paucimobilis* | 09E10 | none | none |
| *Vibrio harveyi* | 09E11 | ATCC | 33867 |
| *Vibrio parahaemolyticus* | 09E12 | none | none |
| *Citrobacter gillenii* | 09F01 | none | none |
| *Myroides odoratimimus* | 09F02 | CCUG | 39352T |
| *Providencia rustigianii* | 09F03 | CIP | A253 |
| *Pseudomonas oryzihabitans* | 09F04 | CCUG | 9468 |
| *Ralstonia gilardii* | 09F05 | LMG | 5886T |
| *Serratia grimesii* | 09F06 | ATCC | 14660T |
| *Yersinia enterocolitica* ssp. *enterocolitica* | 09F07 | none | none |
| *Shigella boydii* | 09F08 | ATCC | 8700T |
| *Sphingobacterium multivorum* | 09F09 | ATCC | 33613T |
| *Tatumella ptyseos* | 09F10 | ATCC | 33301T |
| *Vibrio hollisae* | 09F11 | ATCC | 33564T |
| *Vibrio vulnificus* | 09F12 | ATCC | 27562T |
| *Citrobacter murliniae* | 09G01 | ATCC | 51118T |
| *Myroides odoratimimus* | 09G02 | CCUG | 41717 |
| *Pseudomonas alcaligenes* | 09G03 | ATCC | 14909T |
| *Pseudomonas pseudoalcaligenes* | 09G04 | ATCC | 17440T |
| *Ralstonia gilardii* | 09G05 | LMG | 5888 |
| *Serratia grimesii* | 09G06 | ATCC | 35478 |
| *Yersinia frederiksenii* | 09G07 | ATCC | 33641T |
| *Shigella boydii* | 09G08 | none | none |
| *Sphingobacterium multivorum* | 09G09 | none | none |
| *Tatumella ptyseos* | 09G10 | CCUG | 30112 |
| *Vibrio hollisae* | 09G11 | none | none |
| *Vibrio vulnificus* | 09G12 | CDC | C7184 |
| *Citrobacter murliniae* | 09H01 | none | none |
| *Psychrobacter phenylpyruvicus* | 09H02 | none | none |
| *Pseudomonas alcaligenes* | 09H03 | none | none |
| *Pseudomonas pseudoalcaligenes* | 09H04 | none | none |
| *Yokenella regensburgei* | 09H05 | none | none |
| *Serratia odorifera* | 09H06 | ATCC | 33077T |
| *Serratia rubidaea* | 09H07 | ATCC | 27593T |
| *Yersinia ruckeri* | 09H08 | CUETM | 80-110 |
| *Vibrio alginolyticus* | 09H10 | ATCC | 17749T |
| *Vibrio metschnikovii* | 09H11 | ATCC | 700040T |
| *Corynebacterium cystitidis* | 10A01 | ATCC | 29593T |
| *Corynebacterium diphtheriae* | 10A02 | ATCC | 27010T |
| *Corynebacterium diphtheriae* | 10A03 | none | none |
| *Corynebacterium jeikeium* | 10A05 | ATCC | 43734T |
| *Corynebacterium kutscheri* | 10A06 | ATCC | 15677T |
| *Corynebacterium kutscheri* | 10A07 | none | none |
| *Corynebacterium macginleyi* | 10A08 | none | none |
| *Corynebacterium matruchotii* | 10A09 | none | none |
| *Corynebacterium matruchotii* | 10A10 | ATCC | 14266T |
| *Corynebacterium pilosum* | 10B01 | none | none |
| *Corynebacterium pilosum* | 10B02 | ATCC | 29592T |
| *Corynebacterium propinquum* | 10B03 | none | none |
| *Corynebacterium pseudodiphtheriticum* | 10B04 | ATCC | 10700T |
| *Corynebacterium pseudotuberculosis* | 10B05 | ATCC | 19410T |
| *Corynebacterium pseudotuberculosis* | 10B06 | none | none |
| *Corynebacterium renale* | 10B07 | ATCC | 19412T |
| *Corynebacterium renale* | 10B08 | none | none |
| *Corynebacterium striatum* | 10B09 | ATCC | 6940T |
| *Corynebacterium striatum* | 10B10 | none | none |
| *Corynebacterium urealyticum* | 10B11 | none | none |
| *Corynebacterium urealyticum* | 10B12 | ATCC | 43043 |
| *Corynebacterium xerosis* | 10C01 | none | none |
| *Corynebacterium pseudodiphtheriticum* | 10C02 | none | none |
| *Leifsonia aquatica* | 10C03 | none | none |
| *Leifsonia aquatica* | 10C04 | ATCC | 14665T |
| *Corynebacterium ulcerans* | 10C05 | none | none |
| *Corynebacterium group ANF* | 10C06 | none | none |
| *Corynebacterium cystitidis* | 10C07 | none | none |
| *Corynebacterium group F1* | 10C08 | none | none |
| *Rothia dentocariosa* | 10C09 | none | none |
| *Clostridium bifermentans* | 10C10 | none | none |
| *Clostridium sordellii* | 10C11 | ATCC | 9714T |
| *Clostridium limosum* | 10C12 | none | none |
| *Lactococcus lactis ssp cremoris* | 10D01 | ATCC | 19257T |
| *Leuconostoc mesenteroides ssp mesenteroides* | 10D02 | ATCC | 8293T |
| *Neisseria polysaccharea* | 10D03 | none | none |
| *Haemophilus parahaemolyticus* | 10D04 | none | none |
| *Haemophilus parahaemolyticus* | 10D05 | ATCC | 10014T |
| *Eikenella corrodens* | 10D06 | none | none |
| *Clostridium barati* | 10D08 | none | none |
| *Fusobacterium necrogenes* | 10D09 | ATCC | 25556T |
| *Wolinella succinogenes* | 10D10 | none | none |
| *Eubacterium moniliforme* | 10D11 | ATCC | 25546T |
| *Arcanobacterium haemolyticum* | 10D12 | none | none |
| *Actinomyces naeslundii* | 10E01 | ATCC | 12104T |
| *Arcanobacterium haemolyticum* | 10E02 | ATCC | 9345T |
| *Leuconostoc lactis* | 10E03 | ATCC | 19256T |
| *Streptococcus gallolyticus* | 10E04 | ATCC | 9809 |
| *Streptococcus uberis* | 10E05 | ATCC | 19436T |
| *Streptococcus uberis* | 10E06 | none | none |
| *Aerococcus viridans* | 10E07 | none | none |
| *Streptococcus uberis* | 10E08 | none | none |
| *Aerococcus viridans* | 10E09 | ATCC | 11563T |
| *Streptococcus equi* ssp. *zooepidemicus* | 10F01 | none | none |
| *Macrococcus caseolyticus* | 10F02 | ATCC | 29750 |
| *Streptococcus pyogenes* | 10F03 | ATCC | 19615 |
| *Streptococcus pyogenes* | 10F04 | ATCC | 12344T |
| *Kocuria kristinae* | 10F05 | none | none |
| *Lactococcus lactis* ssp. *lactis* | 10F06 | ATCC | 19435T |
| *Streptococcus vestibularis* | 10F07 | none | none |
| *Streptococcus salivarius* | 10F08 | ATCC | 9758 |
| *Streptococcus mitis* | 10F09 | none | none |
| *Brevibacterium linens* | 10F11 | ATCC | 9174 |
| *Brevibacterium epidermidis* | 10G01 | ATCC | 35514T |
| *Streptococcus equi ssp zooepidemicus* | 10G02 | ATCC | 43079T |
| *Gemella morbillorum* | 10G03 | none | none |
| *Streptococcus acidominimus* | 10G04 | none | none |
| *Rhodococcus equi* | 10G05 | ATCC | 6939T |
| *Streptococcus agalactiae* | 10G06 | none | none |
| *Aerococcus urinae* | 10G07 | none | none |
| *Streptococcus gordonii* | 10G08 | none | none |
| *Lactococcus lactis* ssp. *lactis* | 10G09 | none | none |
| *Leuconostoc lactis* | 10G10 | none | none |
| *Dermabacter hominis* | 10G11 | none | none |
| *Streptococcus constellatus* ssp. *constellatus* | 10G12 | none | none |
| *Staphylococcus saccharolyticus* | 10H01 | ATCC | 14953T |
| *Streptococcus mutans* | 10H02 | none | none |
| *Streptococcus anginosus* | 10H03 | none | none |
| *Streptococcus oralis* | 10H04 | none | none |
| *Streptococcus intermedius* | 10H05 | ATCC | 27335T |
| *Streptococcus intermedius* | 10H07 | none | none |
| *Pseudoramibacter alactolyticus* | 10H08 | ATCC | 23263T |
| *Fusobacterium mortiferum* | 10H09 | none | none |
| *Clostridium innocuum* | 10H10 | none | none |
| *Clostridium botulinum* | 10H11 | ATCC | 25763T |
| *Streptococcus mutans* | 10H12 | ATCC | 25175T |
| *Aerococcus urinae* | 11A01 | ATCC | 51268T |
| *Dolosigranulum pigrum* | 11A02 | CCUG | 31310 |
| *Yersinia mollaretii* | 11A03 | CIP | 103328 |
| *Facklamia hominis* | 11A04 | none | none |
| *Streptococcus thoraltensis* | 11A05 | LMG | 14714 |
| *Helcococcus ovis* | 11A06 | CCUG | 37441T |
| *Lactococcus lactis* ssp. *cremoris* | 11A07 | ATCC | 9596 |
| *Listeria ivanovii* ssp. *ivanovii* | 11A08 | none | none |
| *Listeria welshimeri* | 11A09 | none | none |
| *Streptococcus anginosus* | 11A10 | ATCC | 33397T |
| *Streptococcus gallolyticus* | 11A12 | ACM | 3611T |
| *Aerococcus christensenii* | 11B01 | CCUG | 28831T |
| *Eremococcus coleocola* | 11B02 | CCUG | 38207T |
| *Yersinia pseudotuberculosis* | 11B03 | ATCC | 29833T |
| *Facklamia ignava* | 11B04 | ATCC | 700631T |
| *Methylobacterium extorquens* | 11B05 | ATCC | 14718 |
| *Helcococcus ovis* | 11B06 | CCUG | 39041 |
| *Lactococcus raffinolactis* | 11B07 | ATCC | 43920T |
| *Listeria ivanovii ssp londoniensis* | 11B08 | ATCC | 49954T |
| *Macrococcus caseolyticus* | 11B09 | ATCC | 13548T |
| *Streptococcus canis* | 11B10 | ATCC | 43496T |
| *Streptococcus downei* | 11B11 | none | none |
| *Streptococcus gordonii* | 11B12 | ATCC | 10558T |
| *Aerococcus christensenii* | 11C01 | none | none |
| *Yersinia frederiksenii* | 11C02 | none | none |
| *Yersinia ruckeri* | 11C03 | ATCC | 29473T |
| *Facklamia languida* | 11C04 | CCUG | 37842T |
| *Gemella sanguinis* | 11C05 | ATCC | 700632T |
| *Ignavigranum ruoffiae* | 11C06 | ATCC | 700630T |
| *Lactococcus raffinolactis* | 11C07 | LMG | 14169 |
| *Listeria ivanovii* ssp. *londoniensis* | 11C08 | none | none |
| *Streptococcus canis* | 11C10 | none | none |
| *Methylobacterium mesophilicum* | 11C11 | ATCC | 29983T |
| *Streptococcus hyointestinalis* | 11C12 | ATCC | 49169T |
| *Dermacoccus nishinomiyaensis* | 11D01 | ATCC | 29093T |
| *Yersinia intermedia* | 11D02 | ATCC | 29909T |
| *Yokenella regensburgei* | 11D03 | ATCC | 35313T |
| *Facklamia languida* | 11D04 | none | none |
| *Gemella sanguinis* | 11D05 | CCUG | 37821 |
| *Ignavigranum ruoffiae* | 11D06 | NCIMB | 700637 |
| *Listeria grayi* | 11D07 | ATCC | 19120T |
| *Listeria monocytogenes* | 11D08 | ATCC | 15313T |
| *Streptococcus constellatus* ssp. *constellatus* | 11D10 | ATCC | 27823T |
| *Streptococcus vestibularis* | 11D11 | ATCC | 49124T |
| *Streptococcus hyointestinalis* | 11D12 2 | none | none |
| *Methylobacterium mesophilicum* | 11E01 | none | none |
| *Yersinia intermedia* | 11E02 | none | none |
| *Eremococcus coleocola* | 11E03 | CCUG | 39488 |
| *Facklamia sourekii* | 11E04 | ATCC | 700629T |
| *Globicatella sanguinis* | 11E05 | ATCC | 51173T |
| *Kytococcus sedentarius* | 11E06 | ATCC | 14392T |
| *Listeria grayi* | 11E07 | none | none |
| *Listeria monocytogenes* | 11E08 | ATCC | 19115 |
| *Methylobacterium extorquens* | 11E09 | ATCC | 43645T |
| *Streptococcus criceti* | 11E10 | ATCC | 19642T |
| *Streptococcus dysgalactiae* ssp. *equisimilis* | 11E11 | NCFB | 1356T |
| *Streptococcus hyovaginalis* | 11E12 | ATCC | 700866T |
| *Dolosicoccus paucivorans* | 11F01 | CCUG | 39307T |
| *Yersinia kristensenii* | 11F02 | ATCC | 33638T |
| *Erysipelothrix rhusiopathiae* | 11F03 | ATCC | 19414T |
| *Gemella bergeri* | 11F04 | ATCC | 700627T |
| *Globicatella sanguinis* | 11F05 | CCUG | 33000 |
| *Kytococcus sedentarius* | 11F06 | ATCC | 27573 |
| *Listeria innocua* | 11F07 | ATCC | 33090T |
| *Listeria seeligeri* | 11F08 | ATCC | 35967T |
| *Streptococcus acidominimus* | 11F09 | ATCC | 51725T |
| *Streptococcus criceti* | 11F10 | LMG | 14511 |
| *Streptococcus equi* ssp. *equi* | 11F11 | ATCC | 33398T |
| *Streptococcus hyovaginalis* | 11F12 | LMG | 14843 |
| *Dolosicoccus paucivorans* | 11G01 | CCUG | 41592 |
| *Yersinia kristensenii* | 11G02 | none | none |
| *Erysipelothrix rhusiopathiae* | 11G03 | none | none |
| *Gemella bergeri* | 11G04 | CCUG | 37818 |
| *Helcococcus kunzii* | 11G05 | ATCC | 51366T |
| *Lactococcus garvieae* | 11G06 | ATCC | 43921T |
| *Listeria innocua* | 11G07 | none | none |
| *Listeria seeligeri* | 11G08 | none | none |
| *Streptococcus alactolyticus* | 11G09 | ATCC | 43077T |
| *Streptococcus cristatus* | 11G10 | DSM | 8249T |
| *Streptococcus equi* ssp. *equi* | 11G11 | CCUG | 22971 |
| *Streptococcus infantarius* ssp. *infantarius* | 11G12 | ATCC | BAA-102 |
| *Dolosigranulum pigrum* | 11H01 | ATCC | 51524T |
| *Yersinia mollaretii* | 11H02 | ATCC | 43969T |
| *Facklamia hominis* | 11H03 | ATCC | 700628T |
| *Gemella haemolysans* | 11H04 | ATCC | 10379T |
| *Helcococcus kunzii* | 11H05 | CCUG | 31742 |
| *Lactococcus garvieae* | 11H06 | LMG | 9472 |
| *Listeria ivanovii* ssp. *ivanovii* | 11H07 | ATCC | 19119T |
| *Listeria welshimeri* | 11H08 | ATCC | 35897T |
| *Streptococcus alactolyticus* | 11H09 | NCDO | 2603 |
| *Streptococcus cristatus* | 11H10 | CCUG | 43158 |
| *Streptococcus ferus* | 11H11 | ATCC | 33477 |
| *Streptococcus infantis* | 11H12 | DSM | 12462T |
| *Streptococcus infantis* | 12A01 | CCUG | 39818 |
| *Streptococcus peroris* | 12A02 | DSM | 12493T |
| *Streptococcus sanguinis* | 12A03 | none | none |
| *Aneurinibacillus aneurinilyticus* | 12A04 | LMG | 17164 |
| *Bacillus atrophaeus* | 12A05 | none | none |
| *Bacillus circulans* | 12A06 | none | none |
| *Bacillus halodurans* | 12A07 | ATCC | 27557T |
| *Brevibacillus agri* | 12A08 | none | none |
| *Brevibacillus borstelensis* | 12A09 | LMG | 16103 |
| *Bacillus sphaericus* | 12A10 | ATCC | 14577T |
| *Paenibacillus azotofixans* | 12A11 | ATCC | 35681T |
| *Brevibacillus formosus* | 12A12 | ATCC | 51669T |
| *Streptococcus iniae* | 12B01 | none | none |
| *Streptococcus peroris* | 12B02 | CCUG | 39815 |
| *Streptococcus sobrinus* | 12B03 | ATCC | 33478T |
| *Aneurinibacillus migulanus* | 12B04 | ATCC | 9999T |
| *Geobacillus stearothermophilus* | 12B05 | ATCC | 7953 |
| *Bacillus coagulans* | 12B06 | ATCC | 7050T |
| *Brevibacillus agri* | 12B07 | ATCC | 51663T |
| *Bacillus megaterium* | 12B08 | ATCC | 14581T |
| *Brevibacillus centrosporus* | 12B09 | ATCC | 51661T |
| *Bacillus sphaericus* | 12B10 | ATCC | 10208 |
| *Paenibacillus glucanolyticus* | 12B11 | DSM | 5162T |
| *Brevibacillus formosus* | 12B12 | LMG | 16101 |
| *Paenibacillus macerans* | 12C01 | ATCC | 8244T |
| *Streptococcus pneumoniae* | 12C02 | none | none |
| *Streptococcus sobrinus* | 12C03 | CCUG | 27644 |
| *Aneurinibacillus migulanus* | 12C04 | LMG | 16098 |
| *Bacillus azotoformans* | 12C05 | LMG | 15444 |
| *Bacillus coagulans* | 12C06 | none | none |
| *Bacillus laevolacticus* | 12C07 | ATCC | 23492T |
| *Bacillus megaterium* | 12C08 | ATCC | 11562 |
| *Bacillus pumilus* | 12C09 | ATCC | 7061T |
| *Geobacillus stearothermophilus* | 12C10 | ATCC | 12980T |
| *Paenibacillus pabuli* | 12C11 | LMG | 14016 |
| *Brevibacillus laterosporus* | 12C12 | ATCC | 64T |
| *Streptococcus macacae* | 12D01 | none | none |
| *Streptococcus porcinus* | 12D02 | ATCC | 43138T |
| *Paenibacillus polymyxa* | 12D03 | ATCC | 842T |
| *Bacillus alcalophilus* | 12D04 | ATCC | 27647T |
| *Bacillus badius* | 12D05 | ATCC | 14574T |
| *Bacillus firmus* | 12D06 | ATCC | 14575T |
| *Paenibacillus alvei* | 12D07 | ATCC | 6344T |
| *Bacillus mycoides* | 12D08 | ATCC | 6462T |
| *Bacillus pumilus* | 12D09 | none | none |
| *Paenibacillus thiaminolyticus* | 12D10 | LMG | 16908 |
| *Brevibacillus brevis* | 12D11 | ATCC | 8246T |
| *Brevibacillus laterosporus* | 12D12 | ATCC | 6457 |
| *Streptococcus mitis* | 12E01 | ATCC | 49456T |
| *Streptococcus porcinus* | 12E02 | none | none |
| *Streptococcus thermophilus* | 12E03 | none | none |
| *Bacillus alcalophilus* | 12E04 | none | none |
| *Bacillus badius* | 12E05 | none | none |
| *Bacillus firmus* | 12E06 | ATCC | 8247 |
| *Bacillus lentus* | 12E07 | ATCC | 10840T |
| *Bacillus mycoides* | 12E08 | ATCC | 21929 |
| *Brevibacillus centrosporus* | 12E09 | LMG | 15602 |
| *Geobacillus thermoglucosidasius* | 12E10 | ATCC | 43742T |
| *Brevibacillus brevis* | 12E11 | none | none |
| *Brevibacillus parabrevis* | 12E12 | ATCC | 10027T |
| *Streptococcus parasanguinis* | 12F01 | ATCC | 15912T |
| *Streptococcus ratti* | 12F02 | ATCC | 19645T |
| *Streptococcus thoraltensis* | 12F03 | DSM | 12221T |
| *Bacillus amyloliquefaciens* | 12F04 | ATCC | 23350T |
| *Bacillus cereus* | 12F05 | ATCC | 14579T |
| *Bacillus flexus* | 12F06 | ATCC | 49095T |
| *Bacillus lentus* | 12F07 | ATCC | 10841 |
| *Bacillus niacini* | 12F08 | DSM | 2923T |
| *Bacillus simplex* | 12F09 | ATCC | 49097T |
| *Bacillus thuringiensis* | 12F10 | ATCC | 10792T |
| *Paenibacillus pabuli* | 12F11 | ATCC | 43899T |
| *Brevibacillus parabrevis* | 12F12 | none | none |
| *Streptococcus parasanguinis* | 12G01 | none | none |
| *Streptococcus ratti* | 12G02 | none | none |
| *Virgibacillus pantothenticus* | 12G03 | ATCC | 14576T |
| *Bacillus amyloliquefaciens* | 12G04 | none | none |
| *Bacillus cereus* | 12G05 | none | none |
| *Bacillus fusiformis* | 12G06 | ATCC | 7055T |
| *Bacillus licheniformis* | 12G07 | ATCC | 14580T |
| *Bacillus subtilis* | 12G08 | ATCC | 9372 |
| *Paenibacillus peoriae* | 12G09 | LMG | 16108 |
| *Paenibacillus amylolyticus* | 12G10 | ATCC | 9995T |
| *Brevibacillus choshinensis* | 12G11 | ATCC | 51359T |
| *Brevibacillus reuszeri* | 12G12 | ATCC | 51665T |
| *Streptococcus parauberis* | 12H01 | DSM | 6631T |
| *Streptococcus salivarius* | 12H02 | ATCC | 7073T |
| *Aneurinibacillus aneurinilyticus* | 12H03 | ATCC | 12856T |
| *Bacillus atrophaeus* | 12H04 | ATCC | 49337T |
| *Bacillus circulans* | 12H05 | ATCC | 4513T |
| *Bacillus fusiformis* | 12H06 | LMG | 17347 |
| *Bacillus licheniformis* | 12H07 | none | none |
| *Brevibacillus borstelensis* | 12H08 | ATCC | 51668T |
| *Paenibacillus polymyxa* | 12H09 | ATCC | 21551 |
| *Paenibacillus apiarius* | 12H10 | ATCC | 29575T |
| *Brevibacillus choshinensis* | 12H11 | LMG | 106096 |
| *Brevibacillus reuszeri* | 12H12 | LMG | 16105 |
| *Streptococcus macacae* | 13A01 | ATCC | 35911T |
| *Abiotrophia defectiva* | 13A02 | ATCC | 49176T |
| *Staphylococcus delphini* | 13A03 | ATCC | 49171T |
| *Staphylococcus piscifermentans* | 13A04 | ATCC | 51136T |
| *Gemella morbillorum* | 13A06 | ATCC | 27824T |
| *Bacillus smithii* | 13A07 | DSM | 4216T |
| *Leuconostoc gelidum* | 13A08 | ATCC | 49366T |
| *Neisseria elongata* | 13A09 | ATCC | 25295T |
| *Paenibacillus validus* | 13A10 | ATCC | 43897T |
| *Pediococcus pentosaceus* | 13A11 | LMG | 10478 |
| *Salmonella paratyphi B* | 13A12 | none | none |
| *Paenibacillus alvei* | 13B01 | ATCC | 10871 |
| *Paenibacillus macerans* | 13B02 | none | none |
| *Staphylococcus delphini* | 13B03 | ATCC | 49172 |
| *Staphylococcus piscifermentans* | 13B04 | ATCC | 51137 |
| *Corynebacterium group ANF* | 13B05 | none | none |
| *Kocuria rosea* | 13B07 | ATCC | 186T |
| *Leuconostoc mesenteroides* ssp. *cremoris* | 13B08 | ATCC | 19254T |
| *Neisseria sicca* | 13B09 | ATCC | 29256T |
| *Paenibacillus validus* | 13B10 | LMG | 14019 |
| *Prevotella bivia* | 13B11 | ATCC | 29303T |
| *Salmonella paratyphi B* | 13B12 | none | none |
| *Paenibacillus amylolyticus* | 13C01 | none | none |
| *Geobacillus thermoglucosidasius* | 13C02 | none | none |
| *Staphylococcus felis* | 13C03 | ATCC | 49168T |
| *Staphylococcus piscifermentans* | 13C04 | ATCC | 51138 |
| *Bacillus azotoformans* | 13C05 | ATCC | 29788T |
| *Gracilibacillus dipsosauri* | 13C06 | ATCC | 700347T |
| *Kocuria varians* | 13C07 | CCM | 1414 |
| *Leuconostoc mesenteroides* ssp. *cremoris* | 13C08 | NCDO | 1388 |
| *Neisseria subflava* | 13C09 | ATCC | 49275T |
| *Pediococcus acidilactici* | 13C10 | ATCC | 33314T |
| *Salmonella typhimurium* | 13C12 | ATCC | 13311T |
| *Lactobacillus acidophilus* | 13D07 | none | none |
| *Leuconostoc mesenteroides* ssp. *dextranicum* | 13D08 | ATCC | 19255T |
| *Campylobacter fetus ssp. fetus* | 13D09 | ATCC | 27374T |
| *Pediococcus acidilactici* | 13D10 | LMG | 10632 |
| *Campylobacter lari* | 13D11 | none | none |
| *Clostridium hastiforme* | 13D12 | none | none |
| *Alloiococcus otitis* | 13E01 | ATCC | 51267T |
| *Staphylococcus aureus* ssp. *anaerobius* | 13E02 | ATCC | 35844T |
| *Staphylococcus lutrae* | 13E03 | ATCC | 700374 |
| *Staphylococcus schleiferi* ssp. *coagulans* | 13E04 | CIP | 104366 |
| *Corynebacterium propinquum* | 13E05 | ATCC | 51488T |
| *Granulicatella adiacens* | 13E06 | CCUG | 35135 |
| *Bacillus smithii* | 13E07 | LMG | 6327 |
| *Leuconostoc mesenteroides* ssp. *dextranicum* | 13E08 | none | none |
| *Paenibacillus apiarius* | 13E09 | LMG | 17434 |
| *Clostridium glycolicum* | 13E11 | ATCC | 14880T |
| *Corynebacterium accolens* | 13E12 | CDC | F3883 |
| *Paenibacillus lautus* | 13F01 | ATCC | 43898T |
| *Staphylococcus carnosus* ssp. *utilis* | 13F02 | DSM | 11676T |
| *Staphylococcus muscae* | 13F03 | ATCC | 49910T |
| *Staphylococcus sciuri* ssp. *rodentium* | 13F04 | ATCC | 700061T |
| *Corynebacterium seminale* | 13F05 | none | none |
| *Granulicatella elegans* | 13F06 | DSM | 11693T |
| *Streptococcus parauberis* | 13F07 | DSM | 6631 |
| *Leuconostoc mesenteroides* ssp. *mesenteroides* | 13F08 | CCUG | 39992 |
| *Paenibacillus glucanolyticus* | 13F09 | none | none |
| *Pediococcus dextrinicus* | 13F10 | ATCC | 33087T |
| *Salmonella choleraesuis* | 13F11 | ATCC | 13312T |
| *Paenibacillus azotofixans* | 13G01 | none | none |
| *Staphylococcus carnosus* ssp. *utilis* | 13G02 | DSM | 11677 |
| *Staphylococcus muscae* | 13G03 | ATCC | 49912 |
| *Staphylococcus sciuri* ssp. *rodentium* | 13G04 | ATCC | 700063 |
| *Dermacoccus nishinomiyaensis* | 13G05 | CCUG | 33029 |
| *Granulicatella elegans* | 13G06 | CCUG | 27554 |
| *Leuconostoc citreum* | 13G07 | DSM | 5577T |
| *Leuconostoc pseudomesenteroides* | 13G08 | DSM | 20193T |
| *Paenibacillus lautus* | 13G09 | none | none |
| *Pediococcus inopinatus* | 13G10 | ATCC | 49902T |
| *Salmonella paratyphi A* | 13G11 | none | none |
| *Streptococcus infantarius* ssp. *coli* | 13G12 | none | none |
| *Leuconostoc citreum* | 13H07 | LMG | 9824 |
| *Leuconostoc pseudomesenteroides* | 13H08 | CCUG | 27119 |
| *Pediococcus pentosaceus* | 13H10 | ATCC | 33316T |
| *Salmonella paratyphi A* | 13H11 | none | none |
| *Staphylococcus capitis* ssp. *capitis* | 14A01 | none | none |
| *Pseudoramibacter alactolyticus* | 14A02 | none | none |
| *Staphylococcus cohnii* ssp. *urealyticum* | 14A03 | none | none |
| *Streptococcus suis I* | 14A04 | none | none |
| *Virgibacillus pantothenticus* | 14A05 | LMG | 17342 |
| *Shigella dysenteriae* | 14A06 | none | none |
| *Alloiococcus otitis* | 14A08 | none | none |
| *Staphylococcus saprophyticus* ssp. *bovis* | 14A09 | CIP | 105261 |
| *Streptococcus dysgalactiae* ssp. *dysgalactiae* | 14A10 | ATCC | 43078T |
| *Streptococcus urinalis* | 14A11 | CCUG | 41590T |
| *Corynebacterium macginleyi* | 14A12 | DSM | 44284T |
| *Staphylococcus cohnii* ssp. *urealyticum* | 14B03 | none | none |
| *Streptococcus suis I* | 14B04 | none | none |
| *Weissella paramesenteroides* | 14B05 | ATCC | 33313T |
| *Bacillus thuringiensis* | 14B07 | none | none |
| *Corynebacterium group F1* | 14B08 | none | none |
| *Staphylococcus saprophyticus* ssp. *bovis* | 14B09 | CIP | 105264 |
| *Streptococcus dysgalactiae* ssp. *dysgalactiae* | 14B10 | none | none |
| *Clostridium cadaveris* | 14B11 | DSM | 1284T |
| *Actinomyces neuii* ssp. *anitratus* | 14B12 | CCUG | 43734 |
| *Corynebacterium coyleae* | 14C01 | none | none |
| *Brevibacterium casei* | 14C02 | none | none |
| *Staphylococcus gallinarum* | 14C03 | CCM | 4506 |
| *Streptococcus suis II* | 14C04 | ATCC | 43765T |
| *Weissella paramesenteroides* | 14C05 | NCDO | 1567 |
| *Paenibacillus peoriae* | 14C07 | ATCC | 51925T |
| *Gemella haemolysans* | 14C08 | none | none |
| *Staphylococcus capitis* ssp. *ureolyticus* | 14C09 | none | none |
| *Bacillus subtilis* | 14C10 | none | none |
| *Clostridium ramosum* | 14C11 | DSM | 1402T |
| *Burkholderia multivorans* | 14C12 | CCUG | 43127 |
| *Haemophilus haemolyticus* | 14D01 | ATCC | 33390T |
| *Staphylococcus carnosus* ssp. *carnosus* | 14D02 | none | none |
| *Staphylococcus gallinarum* | 14D03 | CCUG | 28809 |
| *Streptococcus suis II* | 14D04 | none | none |
| *Brevibacterium epidermidis* | 14D05 | none | none |
| *Paenibacillus thiaminolyticus* | 14D07 | DSM | 7262T |
| *Granulicatella adiacens* | 14D08 | ATCC | 49175T |
| *Staphylococcus capitis ssp ureolyticus* | 14D09 | none | none |
| *Staphylococcus condimenti* | 14D10 | DSM | 11675 |
| *Clostridium bifermentans* | 14D12 | CCUG | 36626T |
| *Leuconostoc argentinum* | 14E01 | DSM | 8581T |
| *Staphylococcus hominis* ssp. *novobiosepticus* | 14E03 | none | none |
| *Streptococcus thermophilus* | 14E04 | ATCC | 19258T |
| *Cellulosimicrobium cellulans* | 14E05 | none | none |
| *Staphylococcus condimenti* | 14E07 | DSM | 11674T |
| *Bacillus psychrosaccharolyticus* | 14E08 | ATCC | 23296T |
| *Serratia proteamaculans* ssp. *proteamaculans* | 14E09 | ATCC | 19323T |
| *Pediococcus dextrinicus* | 14E10 | DSM | 20293 |
| *Clostridium tertium* | 14E11 | DSM | 2485T |
| *Corynebacterium auris* | 14E12 | CCUG | 33428 |
| *Leuconostoc carnosum* | 14F01 | ATCC | 49367T |
| *Abiotrophia defectiva* | 14F02 | CCUG | 27805 |
| *Staphylococcus hominis* ssp. *novobiosepticus* | 14F03 | none | none |
| *Tetragenococcus halophilus* | 14F04 | ATCC | 33315T |
| *Salmonella typhi* | 14F05 | ATCC | 19430T |
| *Staphylococcus lutrae* | 14F07 | ATCC | 700373T |
| *Serratia proteamaculans* ssp. *proteamaculans* | 14F09 | none | none |
| *Tetragenococcus halophilus* | 14F10 | DSM | 20338 |
| *Clostridium paraputrificum* | 14F11 | DSM | 2630T |
| *Actinomyces meyeri* | 14F12 | LMG | 16161T |
| *Staphylococcus simulans* | 14G01 | none | none |
| *Corynebacterium urealyticum* | 14G02 | ATCC | 43042T |
| *Staphylococcus hominis* ssp. *novobiosepticus* | 14G03 | CIP | 105720 |
| *Vagococcus fluvialis* | 14G04 | ATCC | 49515T |
| *Salmonella typhi* | 14G05 | none | none |
| *Staphylococcus muscae* | 14G07 | ATCC | 49911 |
| *Staphylococcus hyicus* | 14G08 | none | none |
| *Serratia proteamaculans* ssp. *quinovora* | 14G09 | ATCC | 33765T |
| *Pasteurella multocida* ssp. *septica* | 14G10 | CCUG | 38669 |
| *Burkholderia vietnamiensis* | 14G12 | LMG | 10929T |
| *Nocardia asteroides* | 14H01 | none | none |
| *Staphylococcus cohnii* ssp. *cohnii* | 14H02 | none | none |
| *Staphylococcus hyicus* | 14H03 | none | none |
| *Vagococcus fluvialis* | 14H04 | LMG | 11735 |
| *Shigella dysenteriae* | 14H05 | ATCC | 13313T |
| *Staphylococcus schleiferi* ssp. *coagulans* | 14H07 | CCUG | 37249 |
| *Staphylococcus lugdunensis* | 14H08 | none | none |
| *Serratia proteamaculans* ssp. *quinovora* | 14H09 | CCL | 4705 |
| *Pediococcus parvulus* | 14H10 | CCUG | 41976 |
| *Arcanobacterium bernardiae* | 14H11 | DSM | 9151 |
| *Burkholderia vietnamiensis* | 14H12 | LMG | 11347 |
| *Pasteurella multocida ssp gallicida* | 15A01 | CCUG | 26980 |
| *Comamonas terrigena* | 15A02 | CCM | 2409T |
| *Turicella otitidis* | 15A03 | CCUG | 39347 |
| *Brochothrix campestris* | 15A04 | CCM | 4218T |
| *Arcanobacterium bernardiae* | 15A05 | CCM | 4571T |
| *Corynebacterium confusum* | 15A06 | CCUG | 38268 |
| *Actinomyces turicensis* | 15A07 | LMG | 14329 |
| *Paenibacillus larvae ssp. pulvifaciens* | 15A08 | LMG | 15974T |
| *Clostridium limosum* | 15A09 | DSM | 1400T |
| *Microbacterium testaceum* | 15A10 | DSM | 20526 |
| *Dietzia maris* | 15A11 | DSM | 43984 |
| *Corynebacterium group F1* | 15A12 | N/A | N/A |
| *Facklamia ignava* | 15B01 | CCUG | 43733 |
| *Enterobacter cancerogenus* | 15B02 | CCM | 2421T |
| *Campylobacter lari* | 15B03 | LMG | 8846T |
| *Staphylococcus sciuri* ssp. *sciuri* | 15B04 | CCM | 4232 |
| *Neisseria weaveri* | 15B05 | CCM | 4587T |
| *Corynebacterium glucuronolyticum* | 15B06 | CCUG | 44228 |
| *Arthrobacter globiformis* | 15B07 | LMG | 3813T |
| *Tissierella praeacuta* | 15B08 | LMG | 8203T |
| *Bacillus psychrosaccharolyticus* | 15B09 | DSM | 2270 |
| *Corynebacterium argentoratense* | 15B11 | DSM | 44202T |
| *Aneurinibacillus thermoaerophilus* | 15B12 | LMG | 17166 |
| *Burkholderia stabilis* | 15C01 | LMG | 14294T |
| *Citrobacter koseri* | 15C02 | CCM | 2537T |
| *Staphylococcus saprophyticus* ssp. *saprophyticus* | 15C03 | CCM | 2204 |
| *Leuconostoc fallax* | 15C04 | CCM | 4303T |
| *Actinomyces radingae* | 15C05 | CCM | 4741T |
| *Corynebacterium hoagii* | 15C06 | CCUG | 37875 |
| *Bacillus oleronius* | 15C07 | LMG | 17882 |
| *Virgibacillus proomii* | 15C08 | LMG | 12370T |
| *Corynebacterium mycetoides* | 15C10 | DSM | 20632T |
| *Corynebacterium seminale* | 15C11 | DSM | 44288T |
| *Cellulomonas turbata* | 15C12 | CCM | 4094 |
| *Burkholderia stabilis* | 15D01 | LMG | 14295 |
| *Kytococcus sedentarius* | 15D02 | CCM | 314T |
| *Microbacterium testaceum* | 15D03 | CCM | 2299T |
| *Paenibacillus larvae* ssp. *larvae* | 15D04 | CCM | 4483 |
| *Actinomyces turicensis* | 15D05 | CCM | 4742T |
| *Eubacterium moniliforme* | 15D06 | CCUG | 37327A |
| *Bacillus pallidus* | 15D07 | LMG | 19006T |
| *Virgibacillus proomii* | 15D08 | LMG | 17368 |
| *Corynebacterium mycetoides* | 15D09 | DSM | 20148 |
| *Veillonella dispar* | 15D10 | DSM | 20735T |
| *Corynebacterium falsenii* | 15D11 | DSM | 44352 |
| *Aneurinibacillus thermoaerophilus* | 15D12 | CCM | 4597T |
| *Eikenella corrodens* | 15E01 | LMG | 15557T |
| *Pediococcus parvulus* | 15E02 | CCM | 3450T |
| *Leuconostoc fallax* | 15E03 | DSM | 10614 |
| *Kingella kingae* | 15E05 | CCM | 5679T |
| *Corynebacterium coyleae* | 15E06 | DSM | 44185 |
| *Corynebacterium xerosis* | 15E07 | CCUG | 27544T |
| *Marinibacillus marinus* | 15E08 | DSM | 1298 |
| *Corynebacterium hoagii* | 15E09 | DSM | 20295T |
| *Veillonella atypica* | 15E10 | DSM | 20739T |
| *Corynebacterium falsenii* | 15E11 | DSM | 44353T |
| *Burkholderia multivorans* | 15E12 | CCM | 4863T |
| *Enterococcus dispar* | 15F01 | LMG | 13590 |
| *Pediococcus damnosus* | 15F02 | CCM | 3454 |
| *Paenibacillus larvae* ssp. *pulvifaciens* | 15F03 | CCM | 38 |
| *Corynebacterium glucuronolyticum* | 15F04 | CCM | 4567T |
| *Sporosarcina ureae* | 15F05 | CCM | 684T |
| *Fusobacterium nucleatum* ssp. *animalis* | 15F06 | CCUG | 32879T |
| *Dietzia maris* | 15F07 | LMG | 5361T |
| *Bacillus flexus* | 15F08 | DSM | 1316 |
| *Atopobium parvulum* | 15F09 | DSM | 20469T |
| *Staphylococcus carnosus* ssp. *utilis* | 15F10 | CCM | 4752 |
| *Corynebacterium confusum* | 15F11 | DSM | 44384T |
| *Ralstonia paucula* | 15F12 | CCM | 4867T |
| *Pseudomonas citronellolis* | 15G01 | LMG | 18378T |
| *Acinetobacter radioresistens* | 15G02 | CCM | 3588T |
| *Bacillus subtilis* | 15G03 | ATCC | 6051T |
| *Actinomyces neuii* ssp. *anitratus* | 15G04 | CCM | 4569T |
| *Sporosarcina ureae* | 15G05 | CCM | 860 |
| *Fusobacterium nucleatum ssp. fusiforme* | 15G06 | CCUG | 32880T |
| *Leuconostoc carnosum* | 15G07 | LMG | 18865 |
| *Bacillus simplex* | 15G08 | DSM | 1317 |
| *Finegoldia magna* | 15G09 | DSM | 20470T |
| *Gordonia bronchialis* | 15G10 | DSM | 43341 |
| *Corynebacterium ulcerans* | 15G11 | DSM | 46325T |
| *Corynebacterium accolens* | 15G12 | ATCC | 49725T |
| *Staphylococcus felis* | 15H02 | CCM | 4197 |
| *Brevibacterium casei* | 15H03 | CCM | 4100T |
| *Actinomyces neuii ssp neuii* | 15H04 | CCM | 4570T |
| *Fusobacterium nucleatum* ssp. *fusiforme* | 15H05 | CIP | 60.39 |
| *Actinomyces radingae* | 15H06 | LMG | 15955 |
| *Paenibacillus larvae* ssp. *larvae* | 15H07 | LMG | 9820T |
| *Veillonella atypica* | 15H08 | DSM | 1399 |
| *Fusobacterium nucleatum* ssp. *polymorphum* | 15H09 | DSM | 20482T |
| *Rothia dentocariosa* | 15H10 | DSM | 43762T |
| *Propionibacterium freudenreichii* | 15H11 | CCM | 1857T |
| *Bacillus halodurans* | 15H12 | N/A | N/A |
| *Dermacoccus nishinomiyaensis* | 16A01 | CCM | 2140T |
| *Campylobacter sputorum* ssp. *sputorum* | 16A02 | CCM | 3960T |
| *Anaerobiospirillum succiniciproducens* | 16A03 | CCUG | 24194T |
| *Facklamia sourekii* | 16A04 | CCUG | 28783T |
| *Campylobacter curvus* | 16A05 | CCUG | 11644 |
| *Anaerococcus prevotii* | 16A06 | none | none |
| *Kocuria kristinae* | 16A07 | ATCC | 27570T |
| *Paenibacillus popilliae* | 16A08 | CCUG | 28881T |
| *Leclercia adecarboxylata* | 16A09 | ATCC | 23216T |
| *Staphylococcus succinus* | 16A10 | ATCC | 700337T |
| *Prevotella intermedia* | 16A11 | none | none |
| *Bacillus lentus* | 16A12 | none | none |
| *Streptococcus constellatus* ssp. *pharyngis* | 16B01 | NCTC | 13122T |
| *Arthrobacter globiformis* | 16B02 | none | none |
| *Alicyclobacillus acidocaldarius* | 16B03 | CCM | 2855 |
| *Neisseria polysaccharea* | 16B04 | CCUG | 18030T |
| *Campylobacter concisus* | 16B05 | LMG | 7968 |
| *Enterobacter hormaechei* | 16B09 | ATCC | 49162T |
| *Proteus vulgaris* | 16B10 | CIP | 104989T |
| *Prevotella melaninogenica* | 16B11 | none | none |
| *Brevibacillus brevis* | 16B12 | none | none |
| *Streptococcus infantarius* ssp. *infantarius* | 16C01 | NCIMB | 700599T |
| *Streptococcus infantarius* ssp. *infantarius* | 16C02 | none | none |
| *Sporosarcina pasteurii* | 16C03 | CCM | 2056T |
| *Bacillus schlegellii* | 16C05 | LMG | 7133T |
| *Micrococcus luteus* | 16C07 | none | none |
| *Streptococcus equi* ssp. *equi* | 16C08 | ATCC | 33398T |
| *Escherichia vulneris* | 16C09 | ATCC | 33821T |
| *Campylobacter rectus* | 16C10 | DSM | 3260T |
| *Propionibacterium avidum* | 16C11 | none | none |
| *Bacillus* sp. | 16C12 | none | none |
| *Streptococcus infantarius* ssp. *coli* | 16D01 | NCIMB | 702620T |
| *Marinibacillus marinus* | 16D02 | ATCC | 29841T |
| *Sporosarcina pasteurii* | 16D03 | CCM | 2879 |
| *Raoultella ornithinolytica* | 16D04 | CIP | 103364T |
| *Bacillus schlegellii* | 16D05 | DSM | 9129 |
| *Ochrobactrum intermedium* | 16D06 | CCUG | 43465 |
| *Nocardia asteroides* | 16D07 | CCM | 2754T |
| *Prevotella bivia* | 16D08 | ATCC | 29303T |
| *Morganella morganii* ssp. *sibonii* | 16D09 | ATCC | 49948T |
| *Bacteroides forsythus* | 16D10 | ATCC | 43037T |
| *Bacillus* sp. | 16D12 | none | none |
| *Microbacterium terregens* | 16E01 | CCM | 2634 |
| *Streptococcus parauberis* | 16E02 | ATCC | 13386 |
| *Anaerobiospirillum succiniciproducens* | 16E03 | CCUG | 37578 |
| *Haemophilus ducreyi* | 16E04 | DSM | 8925T |
| *Actinomyces bovis* | 16E05 | none | none |
| *Bacteroides thetaiotaomicron* | 16E06 | DSM | 2079T |
| *Prevotella denticola* | 16E07 | ATCC | 35308T |
| *Paenibacillus glucanolyticus* | 16E08 | DSM | 5162T |
| *Providencia alcalifaciens* | 16E09 | ATCC | 9886T |
| *Bifidobacterium adolescentis* | 16E10 | none | none |
| *Prevotella loescheii* | 16E11 | ATCC | 15930T |
| *Brevibacillus brevis* | 16E12 | none | none |
| *Pediococcus inopinatus* | 16F01 | CCM | 3452 |
| *Dermabacter hominis* | 16F02 | CCM | 4122T |
| *Campylobacter concisus* | 16F03 | LMG | 7788T |
| *Bacillus oleronius* | 16F04 | LMG | 17952T |
| *Clostridium subterminale* | 16F05 | none | none |
| *Rothia mucilaginosa* | 16F07 | ATCC | 25296T |
| *Paenibacillus popilliae* | 16F08 | ATCC | 53256 |
| *Staphylococcus sciuri* ssp. *carnaticus* | 16F09 | ATCC | 700058T |
| *Propionibacterium acnes* | 16F11 | ATCC | 6919T |
| *Paenibacillus macerans* | 16F12 | none | none |
| *Kingella denitrificans* | 16G01 | CCUG | 9125 |
| *Campylobacter curvus* | 16G02 | LMG | 7609T |
| *Haemophilus actinemycetemcomitans* | 16G03 | CCUG | 13227T |
| *Actinomyces neuii* ssp. *neuii* | 16G04 | LMG | 14790 |
| *Morganella morganii* ssp. *morganii* | 16G08 | ATCC | 25830T |
| *Staphylococcus sciuri* ssp. *carnaticus* | 16G09 | ATCC | 700059 |
| *Corynebacterium afermentans* | 16G10 | none | none |
| *Bacteroides capillosus* | 16G11 | ATCC | 29799T |
| *Bacillus coagulans* | 16G12 | none | none |
| *Staphylococcus succinus* | 16H01 | CCUG | 43571 |
| *Microbacterium hominis* | 16H02 | DSM | 12509T |
| *Campylobacter sputorum* ssp. *bubulus* | 16H03 | LMG | 6447 |
| *Campylobacter gracilis* | 16H04 | LMG | 7616 |
| *Clostridium clostridiiforme* | 16H05 | none | none |
| *Corynebacterium auris* | 16H06 | CCM | 4566T |
| *Enterobacter amnigenus* | 16H08 | ATCC | 33072T |
| *Staphylococcus sciuri* ssp. *carnaticus* | 16H09 | ATCC | 700060 |
| *Bacillus* sp. | 16H11 | none | none |
| *Bacillus* sp. | 16H12 | none | none |

### EXAMPLE 2

### Identification of Species-specific Oligonucleotide Sequences Computationally Using 30-mers with 15 Nucleotide Overlaps

DNA sequences of the 16S ribosomal loci of 1,214 bacterial samples representing 545 different species were generated and stored in an internal database. As outlined in the schematic diagram of Fig. 2, these sequences were processed *in silico* to yield 132,325 fragments of 30 nucleotides (nt) in length with a 15 nt (n=15) overlap. These fragments were compared against sequence databases, and the BLAST reports were parsed to discover oligonucleotides that matched a portion of the 16S rDNA sequence of a bacterial species with a criterion of permitting no more than 1 mismatch. Fragments that met this criterion and only matched one or zero species (or unidentified/uncultured/unknown entries) within that criterion were called "species-specific oligos". This examination was conducted on three databases, the internal database described above, GenBank, and RDP. The results indicated that 2,935 oligonucleotides were species-specific (*i.e.*, SSOs) with respect to all three databases examined. Of these species-specific oligonucleotides, 2,723 were unique and specific for 325 different species, including 38 out of the 81 priority species of Table 1, above.

In order to identify conserved sequences upstream and downstream from the 30-mer species-specific oligonucleotide sequences, each 30-mer oligonucleotide was extended by 60 bases on each side to generate a 150-mer oligonucleotide. A multiple sequence alignment using CLUSTALW was generated using the particular 150-mer oligonucleotide to be tested and the 20 and 40 closest sequences in the GenBank database and in the internal database described above, respectively. This information was used to design primers for TaqMan®.

Superimposition of the species-specific regions on the structure of 16S revealed that they fall in non-base-paired loops with considerable tolerance for nucleotide differences. These hypervariable regions are known, but no systematic examination of 16S segments to identify those that are species specific has been reported.

### EXAMPLE 3

### Identification of Species-specific Oligonucleotide Sequences Computationally Using 20-mers with 19 Nucleotide Overlaps

DNA sequences of the 16S ribosomal loci of 1,324 bacterial samples representing 585 different species were processed *in silico* to yield 1,859,805 fragments of 20 nucleotide (nt) each, with an overlap of 19 nt (n=19). A pair-wise comparison was conducted using BLAST on the fragments against the Ribosomal Database Project (RDP) database. The BLAST reports generated from the comparisons were parsed using PERL programming language to identify oligonucleotides that matched a portion of the 16S rDNA sequence of a bacterial species with a criterion of permitting no more than 1 mismatch. Fragments that met this criterion and only matched one or zero species (or unidentified/uncultured/unknown entries) within that criterion were called "species-specific oligos". The analysis discovered 90,079 fragments that met this criterion in RDP database. These 90,079 fragments were then blasted against the GenBank database to further qualify these oligonucleotides as being species-specific, *i.e.,* to identify those which matched one or zero species (or unidentified/uncultured/unknown entries) by this criterion. Of these, only 37,072 remained species-specific after the comparison with GenBank. Of these species-specific oligonucleotides, 25,346 were unique and specific for 648 different species, including 68 out of the 81 priority species denoted in Table 1.

In order to identify conserved sequences upstream and downstream from the 20-mer species-specific oligonucleotide sequences, each 20-mer oligonucleotide was extended by 60 bases on each side to generate a 140-mer oligonucleotide. A multiple sequence alignment using CLUSTALW was generated using the particular 140-mer oligonucleotide to be tested and the 20 and 40 closest sequences in the GenBank database and the internal database, respectively.

Overlapping species specific oligonucleotides (SSO's) were converted into species specific regions (SSR's) as follows. Apparently species-specific oligonucleotide (20-mers) that were consecutively overlapping (*e.g.*, nucleotides 1-20 and nucleotides 2-21, etc. of a 16S rDNA sequence) were combined into a large segment (i.e., 1-21 nucleotides) and termed a "species-specific region" ("SSR"). Any 20-mer oligonucleotide sequence selected from such an SSR will itself be species-specific for the particular species.

### EXAMPLE 4

### Further Selection of Species-Specific Oligonucleotides for Specificity and Robustness

In order to distinguish strain- and species-specific oligonucleotides, a curated database was constructed from Ribosomal Database Project (RDP) 16S rDNA sequences. The RDP database consisted of aprroximatedly 30,000 16S rDNA sequences, but the majority of these sequences *(i.e.,* ~20,000) were not full length. Accordingly the RDP sequences were mapped to the same *E. coli* rDNA sequence as used to assign nucleotide numbers to the SSOs and the GSOs. Once the sequences were mapped, a comparison was performed to determine if each species-specific oligonucleotide was present at the same nucleotide coordinate positions in all entries for the species. The results indicated that 9,879 SSOs representing 232 species were species-specific using the prioritization ranking of 1-25. The other 18,191 SSOs were in the last ranking of 25-30, therefore being strain specific. The resulting curated RDP database (cRDP) was used to further predict the degree of species-specificity of probes.

A count of the number of entries for each species was conducted and the number stored in a table. Oligonucleotides that had been identified as being species-specific using the methods disclosed herein were compared using BLASTN2 (Washington University, St. Louis, MO) (default parameters except that "expectation value" = 1000) against the sequences in this database. The results obtained using the BLAST algorithm were then parsed and examined for the number of species hit and compared with the entries for a given species represented. If a putative species-specific oligonucleotide hit all entries for a given species, then it was designated a species-specific oligonucleotide (SSO); but if the oligonucleotide did not hit all of the entries, then it was designated as a putative strain-specific oligonucleotide and placed on lower priority for further analysis. Species-specific oligonucleotides that are identified can then be used in, for example, diagnostic testing of bacterial infection or bacterial contamination. For example, if the number of *E*. *coli* entries in the database were five, then to be a species-specific oligonucleotide for the species *E. coli,* an oligonucleotide would have "hit" all 5 *E*. *coli* entries in the database by using BLAST or using a similar algorithm. Alternatively, if the oligonucleotide hit only 4 of the 5 entries, then the oligonucleotide would be a putative strain-specific oligonucleotide. Further analysis would be required to verify whether or not the oligonucleotide might be species-specific. For example, sequence errors in the database sequences in some cases could result in artificial designation of an oligonucleotide as a putative strain-specific oligonucleotide. 16S rDNA sequences would have to be obtained from additional strains of the species in question and then compared by BLAST or similar algorithm to the putative species-specific oligonucleotide in order to determine whether it is truly species-specific.

Species Specific Oligonucleotide "Robustness". To prioritize the species-specific oligonucleotides according to their expected utility in a diagnostic test, BLAST reports from comparisons of the species-specific oligonucleotides with the database sequences were parsed, and a table was constructed indicating the number of mismatched nucleotides (*i.e.*, distinguishing moieties) observed (up to a total of 5) in the BLAST alignment with 16S rDNA sequences from other bacterial species. The number of nucleotide changes required for a species-specific oligonucleotide to perfectly match the 16S rDNA sequence of another species is defined as its "robustness" in a diagnostic test. The larger the number of nucleotide changes required for a probe to perfectly align with a different species, the more robust the resulting diagnostic test. In other words, there is a greater margin of error or more tolerance in such a test. For example, hybridization conditions need not be as rigidly defined for an SSO with a high robustness number as for a species-specific oligonucleotide whose sequence is only 1 nucleotide change away from a perfect match with a second species.

Species Specific Oligonucleotide Quality Score. To further prioritize the species specific-oligonucleotides, a quality score was generated using information from both the "robustness" number and the predicted species-specificity. A scoring rating system was created based on 5 different robustness scores combined with 6 levels of predicted species-specificity. This generated a scoring rating system of scores ranging from 1 to 30, with 1 being the most preferred species-specific oligonucleotide and 30 being the least preferred. The 6 levels of predicted species-specificity are calculated based on the total number of entries and the total hits percentage, which is the percentage of the total full length cRDP database entries for a species which are "hit" *(i.e.,* align, permitting no more than 1 mismatch) by the probe in question. The first specificity level represents oligonucleotides with greater than two full-length entries and a 100% total hits percentage. The second level represents oligonucleotides with only one full-length entry and a 100% total hits percentage. The third level contains oligonucleotides lacking any full-length entries; therefore, their species-specificity cannot be predicted at this time. Level four represents oligonucleotides with more than 10 entries and a total hits percentage of at least 85%. The fifth level contains entries from 4 to 9 and a total hits percentage of at least 75%. The sixth level represents oligonucleotides that are most likely strain-specific rather than species-specific and do not meet the above criteria. The "robustness" score indicates the number of nucleotide changes required for the probe to match another species sequence in either RDP or GenBank (*i.e.* MM2 is a mismatch of two nucleotides between the probe and the rDNA sequence of the next best species match, *i.e.*, two nucleotide changes would be required for a perfect match to the next best species; MM>5 indicates that more than 5 nucleotide changes are required for the probe to match another species). In general, a probe with a robustness score ofMM>5 is more preferred than a probe with a score of MM2. Therefore, a species-specific oligonucleotide with a quality score of 1 would exhibit a "robustness" score ofMM>5 and be at level one in predicted species-specificity. Table 8 summarizes the different quality scores according to "robustness" and predicted species-specificity, and Table 9 shows specific examples for each quality score. Table 14 provides the quality score information for all of the species-specific markers (attached to the specification as a separate document). Table 15 (attached to the specification as a separate document) provides the number and identity of the species that each group-specific oligonucleotide marker identifies. This data was further refined a discussed in Example 17 below. All group-specific markers were chosen to match no more than five different species in either the GenBank or RDP databases, with the criterion that each marker exhibits no more than one mismatch when its sequence is aligned with the 16S rDNA sequence of each matching species. Table 15 lists the species that each group-specific oligonucleotide marker identifies. All group-specific markers were chosen to match no more than five different species in GenBank or RDP databases, with the criterion that each marker exhibits no more than one mismatch when its sequence is aligned with the 16S rDNA sequence of each matching species. Shown in Table 15 are the names of the species, which each group-specific marker matches as compared to 16S rDNA sequences in GenBank and RDP.

**TABLE 8**

| **Quality Score** | **Robustness** | **Total Hits Percentage in cRDP** | **Total Full Length Entries in cRDP** |
|---|---|---|---|
| 1 | MM>5 | 100% | >=2 |
| 2 | MM5 | 100% | >=2 |
| 3 | MM4 | 100% | >=2 |
| 4 | MM3 | 100% | >=2 |
| 5 | MM2 | 100% | >=2 |
| 6 | MM>5 | 100% | 1 |
| 7 | MM5 | 100% | 1 |
| 8 | MM4 | 100% | 1 |
| 9 | MM3 | 100% | 1 |
| 10 | MM2 | 100% | 1 |
| 11 | MM>5 | N/A | N/A |
| 12 | MM5 | N/A | N/A |
| 13 | MM4 | N/A | N/A |
| 14 | MM3 | N/A | N/A |
| 15 | MM2 | N/A | N/A |
| 16 | MM>5 | >=85% | >=10 |
| 17 | MM5 | >=85% | >=10 |
| 18 | MM4 | >=85% | >=10 |
| 19 | MM3 | >=85% | >=10 |
| 20 | MM2 | >=85% | >=10 |
| 21 | MM>5 | >=75% | 4-9 |
| 22 | MM5 | >=75% | 4-9 |
| 23 | MM4 | >=75% | 4-9 |
| 24 | MM3 | >=75% | 4-9 |
| 25 | MM2 | >=75% | 4-9 |
| 26 | MM>5 | <75% | >=1 |
| 27 | MM5 | <75% | >=1 |
| 28 | MM4 | <75% | >=1 |
| 29 | MM3 | <75% | >=1 |
| 30 | MM2 | <75% | >=1 |

**TABLE 9**

| Quality Score 1-5: | | | | | | | |
|---|---|---|---|---|---|---|---|
| **SEQ ID NO** | **Species Name** | **GenBank Best Hit** | **RDP Best Hit** | **Robutstness in GenBank and RDP** | **Total Full Length Entries in cRDP** | **Total Hits Percentage in cRDP** | **Quality Score** |
| *7* | *Enterococcus faecalis* | *Enterococcus faecalis* | *Enterococcus faecalis* | MM>5 | 5 | 100% | 1 |
| 27 | *Haemophilus ducreyi* | *Haemophilus ducreyi* | *Haemophilus ducreyi* | MM4 | 4 | 100% | 3 |
| 2836 | *Streptococcus dysgalactiae* | *Streptococcus dysgalactiae* | *Streptococcus dysgalactiae* | MM2 | 32 | 100% | 5 |

| Quality Score 6-10: | | | | | | | |
|---|---|---|---|---|---|---|---|
| 3564 | *Streptococcus thoraltensis* | *Streptococcus thoraltensis* | *Streptococcus thoraltensis* | MM>5 | 1 | 100% | 6 |
| 3648 | *Prevotella disiens* | *Prevotella disiens* | *Prevotella disiens* | MM4 | 1 | 100% | 8 |
| 4293 | *Atopobium parvulum* | *Atopobium parvulum* | *Atopobium parvulum* | MM3 | 1 | 100% | 9 |

| Quality Score 11-15: | | | | | | | |
|---|---|---|---|---|---|---|---|
| 11987 | *Corynebacterium renale* | *No Hits* | *No Hits* | MM>5 | 0 | 0% | 11 |
| 13192 | *Plesiomonas shigelloides* | *Plesiomonas shigelloides* | *Plesiomonas shigelloides* | MM3 | 0 | 0% | 14 |
| 14499 | *Bacteroides stercoris* | *Bacteroides stercoris* | *Bacteroides stercoris* | MM2 | 0 | 0% | 15 |

| Quality Score 16-20: | | | | | | | |
|---|---|---|---|---|---|---|---|
| 25330 | *Streptococcus dysgalactiae* | *Streptococcus dysgalactiae* | *Streptococcus dysgalactiae* | MM3 | 32 | 94% | 19 |
| 25341 | *Streptococcus suis* | *Streptococcus suis* | *Streptococcus suis* | MM2 | 34 | 85% | 20 |
| 25350 | *Streptococcus dysgalactiae* | *Streptococcus dysgalactiae* | *Streptococcus dysgalactiae* | MM2 | 32 | 94% | 20 |

| Quality Score 21-25: | | | | | | | |
|---|---|---|---|---|---|---|---|
| 25352 | *Enterococcus saccharolyticus* | *Enterococcus saccharolytic us* | *Enterococcus saccharolyticus* | MM>5 | 4 | 75% | 21 |
| 25359 | *Streptococcus suis* | *Streptococcus suis* | *Streptococcus suis* | MM3 | 34 | 82% | 24 |
| 25424 | *Aerococcus urinae* | *Aerococcus urinae* | *Aerococcus urinae* | MM2 | 9 | 78% | 25 |

| Quality Score 26-30: | | | | | | | |
|---|---|---|---|---|---|---|---|
| 25686 | *Paenibacillus* | *Paenibacillus* | *Paenibacillus* | MM>5 | 3 | 67% | 26 |
| | *azotofixans* | *azotofixans* | *azotofixans* | | | | |
| 25793 | *Streptococcus dysgalactiae* | *Streptococcus dysgalactiae* | *Streptococcus dysgalactiae* | MM3 | 32 | 31% | 29 |
| 27568 | *Campylobacter sputorum* | *Campylobact er sputorum* | *Campylobacter sputorum* | MM2 | 9 | 11% | 30 |

The system of naming species represented by species-specific oligonucleotides was done as follows. To further evaluate the species for which the oligonucleotide probe was specific, BLAST searches were run against GenBank and RDP for each oligonucleotide probe, and the name of the species exhibiting the best BLAST hit was compared to the species name of the isolate from which the oligonucleotide probe was derived, (*i.e.*, the species name of this isolate in the internal database, SNID). In most cases, the same species name was found in the internal database, the RDP database, and the GenBank database. However, the species name for an rDNA sequence may vary in various databases for several reasons including, for example, systematic nomenclature changes as well as identification errors. In case of discrepancies in the species names between the three databases, the following criteria were used to select the species name for the purposes of this invention. The following definitions are helpful for describing the set of criteria: (1) let "SNIDseq" and "SNIDprobe" be the species name in the internal database (*i.e.*, that assigned by diagnostic tests to the isolate from which the 16S rDNA sequence was determined and the probe was derived) for the 16S rDNA sequence and the probe, respectively, (2) let "BLASTseq" and "BLASTprobe" be the names of the species among the top 10 GenBank and RDP BLAST hits for the 16S rDNA sequence and the probe, respectively, and (3) let "bestBLASTseq" and "bestBLASTprobe" be the name of the species which is the best GenBank and RDP BLAST hits for the 16S rDNA sequence and the probe, respectively; and (4) let a single isolate be designated as "A" and multiple probes derived from the 16S rDNA sequence of isolate "A" be designated as "A1", "A2", etc. The following criteria serve as useful guides for assigning a species name to each probe, but one skilled in the art will recognize that confirmation must be obtained experimentally by use of the probes for hybridization-based testing. This naming criteria was also utilized in preparation of the attached Sequence Listing for the Source Identifier
1) If no BLAST hits were obtained for the oligonucleotide probe in GenBank and RDP, the species name of the internal database was selected.
2) If BLAST hits were obtained for the oligonucleotide probe in only one public database (GenBank or RDP), the species name of the internal database was selected.
3) If the oligonucleotide probe was obtained from a species not represented in GenBank or RDP, the species name of the internal database was selected.
4) If the discrepancy in species names was caused by nomenclature changes, the most up-to-date species name to our knowledge was selected
5) If the best BLAST hits in Genbank and RDP for the oligonucleotide probe were obtained with the same species, but that species name did not match the species name in the internal database and:
   a) If SNIDseq is identical to bestBLASTseq, then the species name of the internal database was selected.
   b) If SNIDseq is within BLASTseq but SNIDprobe is not within BLASTseq, then the species name of the internal database was selected.
   c) If SNIDseqA is not within BLASTseqA, but bestBLASTprobeA1 is not the same as bestBLASTprobeA2, then the species name of the internal database was selected.
   d) If SNIDseq is not within BLASTseq at the species level, but is within BLASTseq at the Genus level and SNIDprobe is not within BLASTseq, then the species name of the internal database was selected.
   e) If only one or few 16S rDNA sequences of short length or poor quality for SNIDseq were found in GenBank, then the species name of the internal database was selected
   f) If SNIDseq is within BLASTseq, but bestBLASTprobe is a different but higher quality hit in BLASTseq, then the species name of GenBank and RDP was selected.
   g) SNIDseq is not within BLASTseq (and the strain was obviously mistyped), then the species name of GenBank and RDP was selected.
   h) If the species name in the internal database was poorly described (at the genus level for instance), the name obtained in GenBank and RDP was selected.

### EXAMPLE 5

### Selection of TagMan® Probes and Primers

TaqMan® probes and primers were designed from the 150-mer oligonucleotide sequence using PrimerExpress software (version 1.5, Applied Biosystems, Foster City, CA). Probes and primers were designed with the following characteristics (as recommended by Applied Biosystems):
- The choice of probe is made first; then the primers are designed to be as close as possible to the probe without overlapping the probe.
- G+C content of TaqMan® probes should be between 30 and 80 %.
- Probe melting temperature (Tₘ) should be approximately 68°C to approximately 70°C.
- TaqMan® probes should not begin with G's at the 5' end as it tends to quench the fluorescence.
- The probes should not have runs of identical nucleotides.
- Strands that give the probe more Cs than Gs should be selected to get a better signal.
- Primers should have a melting temperature (Tₘ) of 58-60°C. Probes need to have a Tₘ value of 10°C higher. The Tₘ difference between the forward and reverse primers should not exceed 2°C.
- Primer optimal length is 20 nucleotides.
- The G+C content of primers should be between 30 and 80%.
- The same nucleotide (G, A, T, or C) should not be repeated several times in a row in the sequence.
- The total number of Gs and Cs in the last five nucleotides at the 3' end of the primer should not exceed two.
- Maximum amplicon size should be 150 bp (ideally 50-150 bases).

The choice of TaqMan® probes can be done using PrimerExpress software or manually by the user; in which case, the user indicates to the software the TaqMan® probe sequence location, and the software searches for primers fitting the characteristics described above. If no primers corresponding to the criteria described above are found, the PrimerExpress software parameters are adjusted. For instance, the appropriate Tₘ may not be found surrounding some TaqMan® probe unless the requirements for Tₘ are adjusted (preferably, using a Tₘ between 53°C and 60°C and more than 2°C difference between the Tₘs of the forward and reverse primers).

Using this method, twenty sets of probes and primers were designed for the following species: *Bacteroides merdae, Bacteroides stercoris, Bacteroides thetaiotaomicron, Bacteroides vulgatus, Cardiobacterium hominis, Clostridium botulinum, Clostridium difficile, Clostridium septicum, Clostridium tetani, Eikenella corrodens, Enterococcus faecalis, Haemophilus ducreyi, Kingella denitrificans, Morganella morganii, Neisseria gonorrhoeae, Oligella urethralis, Proteus mirabilis, Providencia stuartii,* and *Streptococcus agalactiae.*

### EXAMPLE 6

### Species-specificity in a set of GSOs

Using sets of GSOs, rDNA sequences were identified to the species level as described further below.

Number of species covered by SSOs, GSOs or sets of GSOs. For species that were lacking a SSO *(i.e.,* 76 species), the 20,594 GSOs were analyzed to determine if sets of GSOs could identify a particular species. The following table represents the 46 species in which species-specificity could be determined by using sets of GSOs.

**TABLE 10:**

| Species using combinations of GSOs for species identity | | |
|---|---|---|
| **Species** | **GSO SEQ ID NOS** | **Number of GSOs** |
| *Acinetobacter baumannii* | 36686,30934,48435,33884 | 4 |
| *Acinetobacter calcoaceticus* | 39848,36113,30934,44459 | 4 |
| *Acinetobacter junii* | 29479,30108,42845 | 3 |
| *Aeromonas veronii* | 47193,42352 | 2 |
| *Aneurinibacillus migulanus* | 44900,38378,32010 | 3 |
| *Arcanobacterium bernardiae* | 39338,46134,44988 | 3 |
| *Bacillus amyloliquefaciens* | 33959,42090 | 2 |
| *Bacillus atrophaeus* | 35155,44448 | 2 |
| *Bacillus halodurans* | 43844,31450,43257,28281 | 4 |
| *Bacillus pallidus* | 46080,47414,30578 | 3 |
| *Bacillus pumilus* | 45259,32582 | 2 |
| *Bacillus simplex* | 34319,34826,42979 | 3 |
| *Bacillus sphaericus* | 28338,34282,37717 | 3 |
| *Bacillus subtilis* | 45687,42090,44487 | 3 |
| *Brevibacillus borstelensis* | 35138,47275,34276,46030 | 4 |
| *Brevibacillus parabrevis* | 31941,28198 | 2 |
| *Brevundimonas diminuta* | 31958,29543,29067,28720 | 4 |
| *Burkholderia multivorans* | 37454,37447 | 2 |
| *Burkholderia vietnamiensis* | 37447,30138,37454 | 3 |
| *Campylobacter fetus ssp. fetus* | 36610,47208 | 2 |
| *Campylobacter gracilis* | 34520,40897,41548 | 3 |
| *Chryseobacterium meningosepticum* | 35999,48268,38975,46309 | 4 |
| *Clostridium paraputrificum* | 35968,33115 | 2 |
| *Clostridium sporogenes* | 36468,45611,43243,32088 | 4 |
| *Corynebacterium amycolatum* | 31320,46952,29055 | 3 |
| *Gemella bergeri* | 36461,47995,47328 | 3 |
| *Moraxella nonliquefaciens* | 43598,32706 | 2 |
| *Porphyromonas levii* | 35301,47513,34027,36791 | 4 |
| *Pseudomonas aeruginosa* | 41019,46306,30201,34876 | 4 |
| *Raoultella planticola* | 43294,37670,43050 | 3 |
| *Rhodococcus equi* | 37186,28975,45409 | 3 |
| *Salmonella enteritidis* | 45117,41157,31333 | 3 |
| *Salmonella typhimurium* | 32964,32896,41157 | 3 |
| *Staphylococcus caprae* | 36775,35000,34734 | 3 |
| *Staphylococcus gallinarum* | 28303,47437 | 2 |
| *Staphylococcus hominis* | 37181,29913,37005 | 3 |
| *Staphylococcus pasteuri* | 33774,37181 | 2 |
| *Staphylococcus pulvereri* | 47140,42760,39045 | 3 |
| *Staphylococcus vitulinus* | 39045,47140 | 2 |
| *Staphylococcus xylosus* | 47173,29349,38773 | 3 |
| *Streptococcus downei* | 43822,34565,30088 | 3 |
| *Streptococcus hyovaginalis* | 34828,36951 | 2 |
| *Streptococcus intermedius* | 29499,34917,33594 | 3 |
| *Streptococcus parauberis* | 36715,31995,40921 | 3 |
| *Xanthomonas campestris* | 33160,46608,45341 | 3 |
| *Yersinia intermedia* | 39077,33902 | 2 |

Of the 567 species represented by the 1,324 completed 16S rDNA, 491 of these species had at least one SSO. Another 46 species were identified at the species level using a combination of two or more GSOs, as reflected in the third column of Table 10. Twenty species could only be characterized as belonging to a subset containing multiple species. Only 10 species were not able to be determined using the above analysis: *Cedecea neteri, Citrobacter gillenii, Citrobacter murliniae, Enterobacter asburiae, Enterobacter intermedius, Kluyvera ascorbata, Kluyvera cryocrescens, Moraxella (Branhamella) catarrhalis, Pantoea dispersa,* and *Pseudomonas alcaligenes.* Thus the probes utilized were able to discriminate 557 out of 567 species tested.

### EXAMPLE 7

### Verification of the Species Specificity and Robustness of Species-Specific Oligonucleotides by TaqMan® Assay

The species-specificity and robustness of probes was evaluated by bioinformatic analysis. Each 30-mer oligonucleotide was compared by BLASTN2 (Washington University, St. Louis, MO) (default parameters except that "expectation value" = 1000) to sequences present in proprietary or public databases. The number of mismatches was derived from the BLAST report (see Example 4). As discussed, the robustness of the 30-mer increases as the number of mismatches increases. Table 11 below illustrates the number of mismatches needed for the probes to hit the next closest species in RDP database.

**TABLE 11.**

| Number of mismatches required to eliminate species-specificity of a probe. The probe sequences were compared to those available in Ribosomal Database Project (RDP) and in GenBank. | | | | | |
|---|---|---|---|---|---|
| Species | Probe SEQ ID NO | OligolD | Probe Name | Primer SEQ ID NO | # of mismatches needed to eliminate species-specificity (according to RDP & GenBank databases) |
| *Bacteroides merdae* | 48727 | 06B08-0095 | PB01 | 48707, 48732 | 3 |
| *Bacteroides stercoris* | 48729 | 06C07-0095 | PB03 | 48708, 48733 | 3 |
| *Bacteroides thetaiotaomicron* | 26757 | 06A02-033 | PB14 | 48717,48742 | 2 |
| *Bacteroides vulgatus* | 48728 | 06C12-0051 | PB02 | 48709, 48734 | 5 |
| *Cardiobacterium hominis* | 7089 | 07E01-071 | PB15 | 48718,48743 | 2 |
| *Clostridium botulinum* | 26916 | 06A04-065 | PB17 | 48719,48744 | 2 |
| *Clostridium difficile* | 273 | 06H02-0037 | PB12 | 48716,48741 | 3 |
| *Clostridium septicum* | 5711 | 06A06-008 | PB18 | 48720, 48745 | 2 |
| *Clostridium tetani* | 15516 | 06E10-00065 | PB07 | 48711,48736 | 2 |
| *Eikenella corrodens* | 25477 | 10D06-0086 | PB08 | 48712, 48737 | 2 |
| *Enterococcus faecalis* | 388 | 03D09-00030 | PB05 | 48710,48735 | 3 |
| *Haemophilus ducreyi* | 2414 | 07E07-0071 | PB11 | 48715,48740 | 3 |
| *Haemophilus ducreyi* | 3308 | 07E07-034 | PB19 | 48721,48746 | 2 |
| *Kingella denitrificans* | 36 | 07F07-0065 | PB09 | 48713,48738 | 4 |
| *Morganella morganii* | 19649 | 01A11-017 | PB20 | 48722, 48747 | 2 |
| *Neisseria gonorrhoeae* | 495 | 07G04-0067 | PB10 | 48714,48739 | 3 |
| *Oligella urethralis* | 23485 | 08C11-059 | PB21 | 48723, 48748 | 2 |
| *Proteus mirabilis* | 15260 | 01E07-070 | PB22 | 48724, 48749 | 2 |
| *Providencia stuartii* | 15473 | 01E08-032 | PB23 | 48724, 48750 | 2 |
| *Streptococcus agalactiae* | 3454 | 01B05-071 | PB24 | 48725,48751 | 3 |
| *Staphylococcus chromognes* | 6024 | 05B07-11 | PB25 | 48764,48770 | 2 |
| *Prevotella oralis* | 3935 | 07D08-09 | PB26 | 48765,48771 | 3 |
| *Staphylococcus simulans* | 4333 | 05G07-12 | PB27 | 48766,48772 | 3 |
| *Moraxella atlantae* | 9336 | 08B08-46 | PB28 | 48767, 48773 | 2 |
| *Kocuria rosea* | 24018 | 01B08-11 | PB29 | 48768, 48774 | 2 |

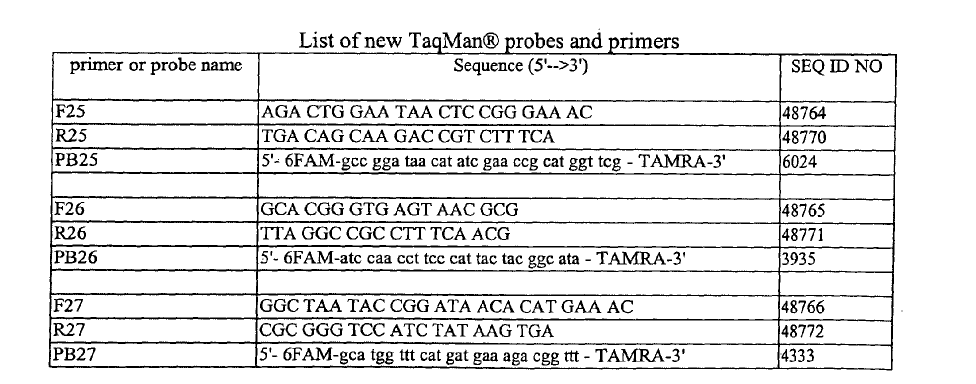

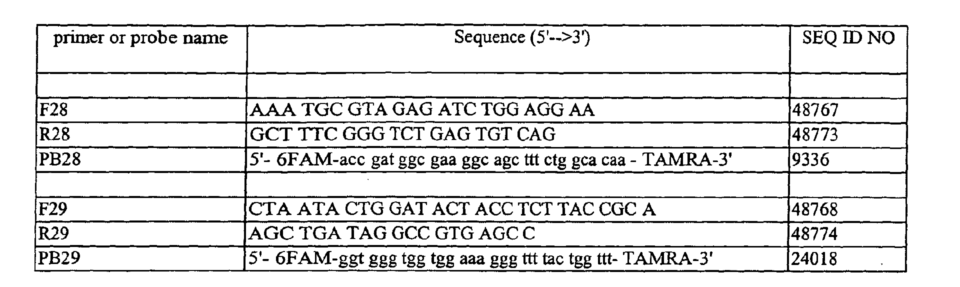

The species-specificity of probes was also verified at the experimental level using the TaqMan® method.

For each probe, one or several isolates from the target species and several isolates of related species were tested. The isolates tested in TaqMan® assays were obtained from the internal database described above. These isolates represented clinical strains obtained from different clinical laboratories and collection strains obtained from the American Type Culture Collection (ATCC) or other reference institutes. Identification of these strains was done by phenotypic methods, which included classical biochemical reference tests and/or commercial tests. The TaqMan® results, number of isolates and species tested for each probe are shown in Table 12.

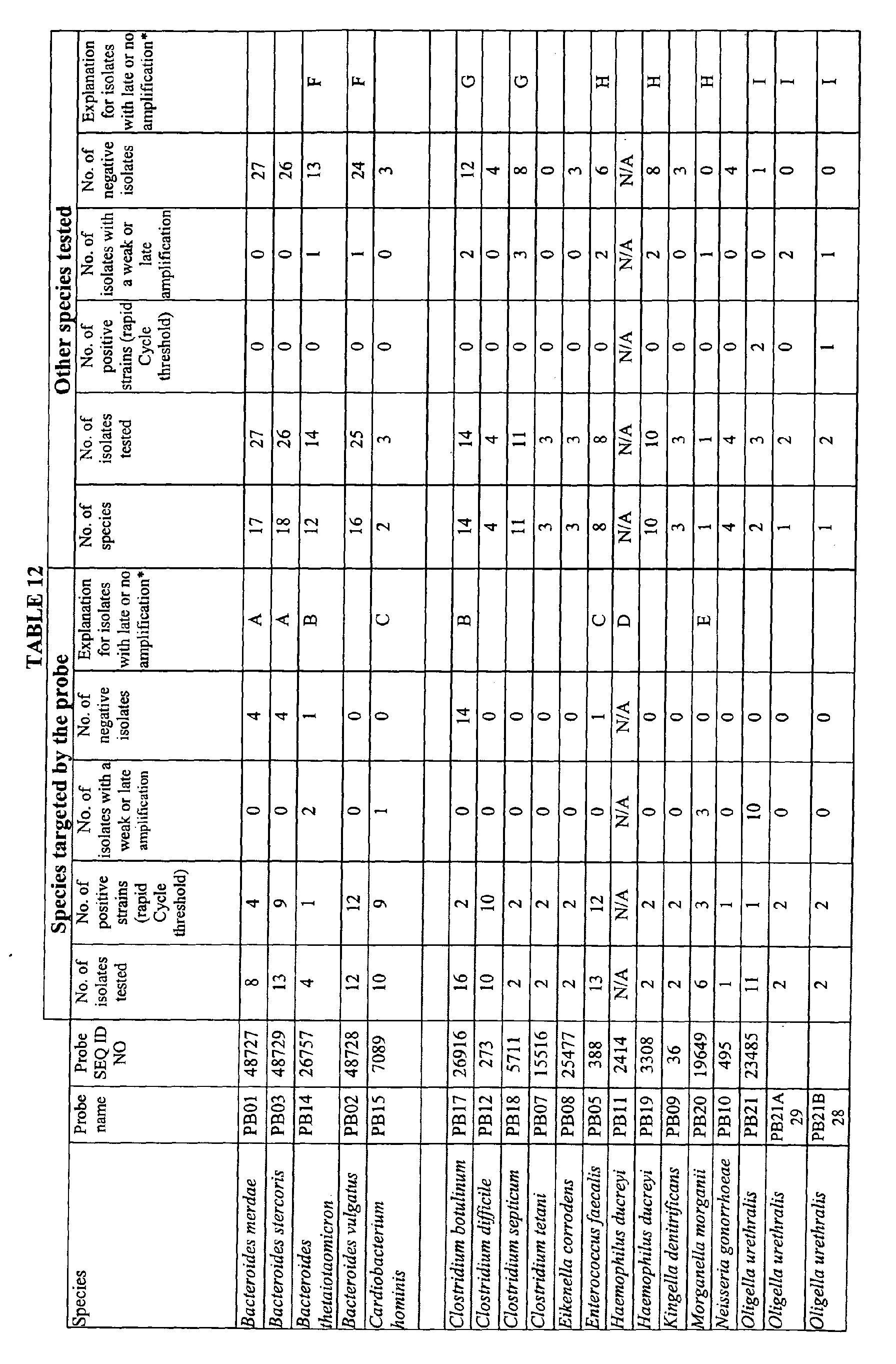

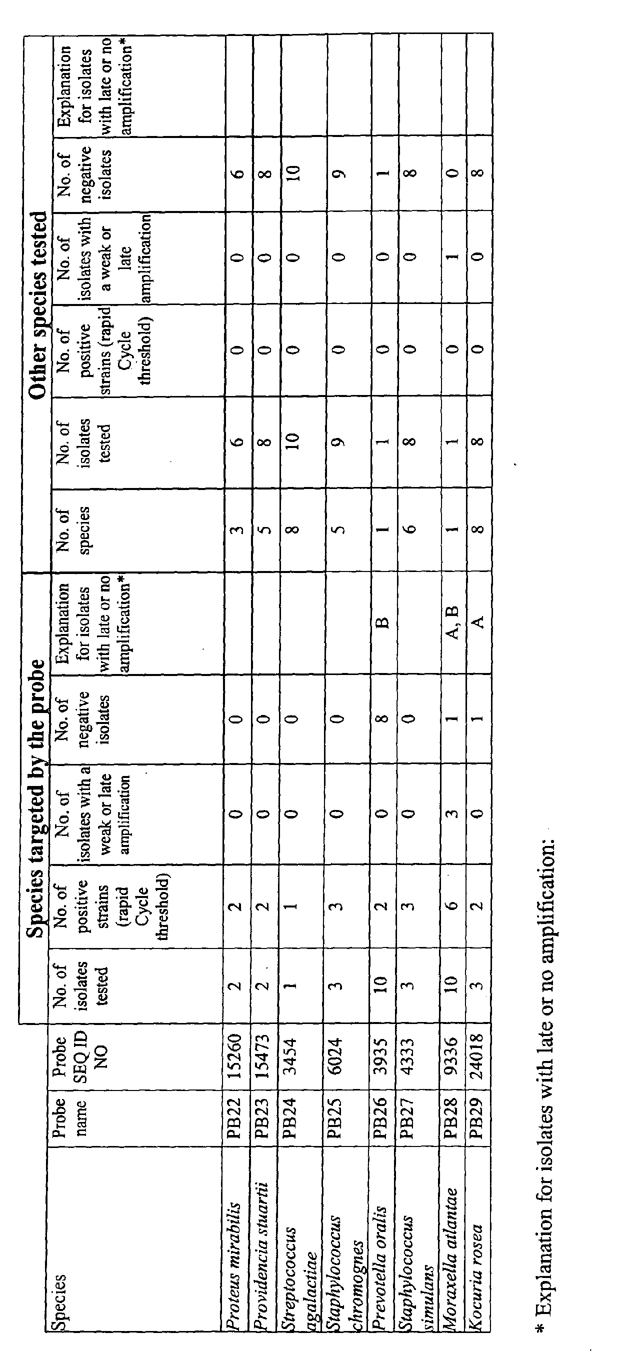

Reason A: The 16S sequence of negative isolates does not match the probe sequence suggesting that these strains might have been mistyped by phenotypic methods.
Reason B: The Probe is strain-specific rather than species-specific (few mismatches with other isolates from the same species or other species).
Reason C: Mistyped strain.
Reason D: No PCR product because of a TaqMan® probe that does not fit the TaqMan® probe design guidelines.
Reason E: Possible split of isolates at sub-species level.
Reason F: Non-specific late amplification for isolates with >6 mismatches for the probe sequence.
Reason G: Non-specific late amplification for isolates with 1-3 mismatches for the probe sequence.
Reason H: Non-specific late amplification for isolates with 3-5 mismatches for the probe sequence.
Reason I: The probe PB21B28 is the same as PB21 minus the 2 bases in 5' that gives the species specificity. PB21A29 is the same as PB21 minus 1 base. The location of critical nucleotides for species-specificity at the 5' end might not be optimal.

Some of the discrepancies between TaqMan® results and the expected identification *(i.e.* the species the isolate belongs to) were further investigated by sequencing of 16S rDNA. For instance, all isolates labeled as *Bacteroides vulgatus* were positive in a TaqMan® assay using a probe specific to *B. vulgatus* (see Fig. 6). By contrast, some isolates identified phenotypically as *B. merdae* or B. *stercoris were* negative when tested with the appropriate TaqMan® probes. Four out of eight strains labeled *B. merdae* and 4 of 13 strains labeled *B. stercoris* did not have a positive reaction in the TaqMan® assay. To determine whether these results were due to failure of the TaqMan® assay, intra-species strain variation, or a wrong identification call based on phenotypic testing, the 16S rDNA of the strains labeled as *B. vulgatus* or *B. merdae* or *B. stercoris* were sequenced. The sequence alignment was obtained using ClustalX. The samples that were negative by TaqMan® assay did not contain the TaqMan® probe sequence in their 16S rDNA. Thus, the TaqMan® results were consistent with the DNA sequence of those isolates, and the species identity of the isolates was apparently mis-typed using phenotypic methods. These results emphasize the need to develop rapid diagnostic tests based on molecular methods to complement and/or replace some phenotypic tests in order to improve the diagnostics of bacterial infections and move towards more appropriate and efficient antibiotic therapy.

### EXAMPLE 8

### TaqMan® Probes Specific to Clostridium difficile

TaqMan® reactions were conducted in 50 µL volumes in 96-well optical-grade plate (PE Applied Biosystems) using a ABI7700 sequence detector (PE Applied Biosystems). Each 50 µL reaction mix contained 900 nM forward primer (Seq ID 48716), 900 nM reverse primer (Seq ID 48741), 250 nM TaqMan® probe (SeqID 273) labeled with FAM in the 5' direction and TAMRA in the 3' direction, and 25 µL of TaqMan® universal PCR master mix at a concentration of 2X (PE Applied Biosystems) that contained MgCl₂, AmpErase® uracil-N-glycosylase (UNG), AmpliTaq Gold® DNA polymerase, reference dye ROX, and a mix of dNTP including dUTP, and 10 ng of DNA sample.

A typical TaqMan® reaction consisted of three steps: (1) 2 min. at 50°C to activate the UNG enzyme that prevents the reamplification of carryover-PCR products by removing any uracyl present in double-stranded DNA; (2) 10 min at 95°C to activate the AmpliTaq Gold® enzyme; and (3) 40 cycles of 15 sec at 95°C (denaturation) and 1 min at 60°C (annealing and extension).

Data analysis was conducted using the ABI 7900 Prism software (Applied Biosystems). The baseline fluorescence was determined by the software between the 3^{rd} and 15^{th} PCR cycles. The baseline can be recalculated between different cycles (3 and 12 for example) if the first positive samples have a Ct of less than 15. Once the baseline is set correctly, the software automatically sets the threshold at 10 standard deviations above the mean baseline fluorescence. The threshold can also be adjusted manually by the user. The cycle threshold (Ct) represents the cycle number at which there is a significant increase of fluorescence above threshold.

The software examines the fluorescence intensity of reporter and quencher dyes and calculates the increase in normalized reporter emission intensity over the course of the amplification. The ΔRn is calculated as follows:
- Rn⁺ is the Rn value of a reaction containing all components
- Rn⁻ is the Rn value of an unreacted sample (baseline value or the value detected in non-template control).
- ΔRn is the difference between Rn⁺ and Rn⁻. It is an indicator of the magnitude of the signal generated by the PCR.
The results expressed as ΔRn are then plotted versus time, represented by cycle number, to produce a continuous measure of PCR amplification.

A non-template control was used as a negative control.

Fig. 5 illustrates results from the TaqMan® real-time PCR test of five bacterial strains, two *C. difficile* strains and three strains representing other species from the *Clostridium* genus. The test clearly distinguished between the *C. difficile* strains (#06H01 and 06H02) and the non *C. difficile* isolates, since only the isolates belonging to C. *difficile* species had a positive amplification signal detected after 18 PCR cycles (CT value of about 18 cycles).

### EXAMPLE 9

### Probes Specific to Clostridium septicum

TaqMan® reactions were conducted in 50 µL volumes in 96-well optical-grade plate (PE Applied Biosystems) using an ABI7700 sequence detector (PE Applied Biosystems). Each 50 µL reaction mix contained 900 nM forward primer (Seq ID 48720), 900 nM reverse primer (Seq ID 48745), 250 nM TaqMan® probe (Seq ID 5711) labeled with FAM in the 5' direction and TAMRA in the 3' direction, 25 µL of TaqMan® universal PCR master mix at a concentration of 2X (PE Applied Biosystems) that contained MgCl₂, AmpErase® uracil-N-glycosylase (UNG), AmpliTaq Gold® DNA polymerase, reference dye ROX, and a mix of dNTP including dUTP, and 10 ng of DNA sample. The results expressed as ΔRn (indicator of the magnitude of the signal generated by the PCR) were then plotted versus time, represented by cycle number, to produce a continuous measure of PCR amplification.

A non-template (no DNA) control was used as a negative control. As shown in Figure 4, the two isolates of *Clostridium septicum* gave the expected positive results with the fastest Ct (∼15). A partial multiple sequence alignment of 16S sequences of all samples tested in this assay is presented in Table 13. The region containing the probe for the *C. septicum* species is shaded; the nucleotides that differ from the probe sequence for the other *Clostridium* species tested are underlined. Interestingly, three samples belonging to other species gave a delayed Ct with a value of 17 to 19. Although this TaqMan® assay was able to clearly differentiate the C. *septicum* isolates from other samples, it also suggests that other DNA-based tests more robust and specific than TaqMan® assays need to be developed to reach the *in vitro* diagnostic market.

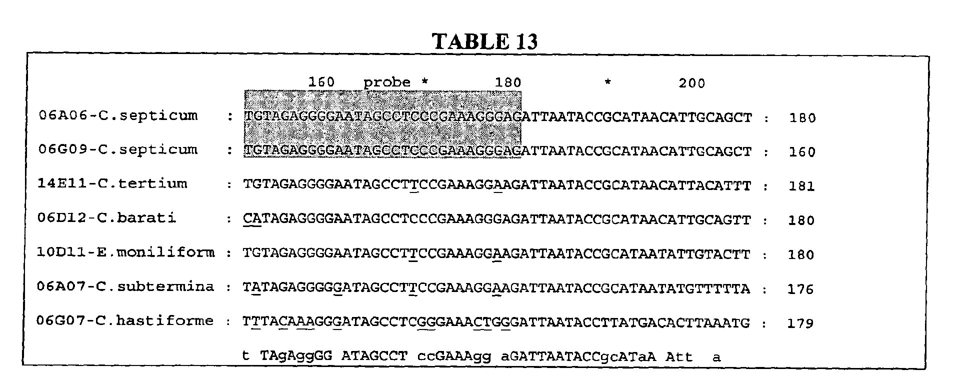

### EXAMPLE 10

### Optimization of DNA Concentration to Use in TaqMan® Assay

The TaqMan® assay for detecting DNA sequence differences is based on real-time PCR which can be also be used to quantify the amount of target DNA originally present in a sample to test. Therefore, it is important to determine the optimal DNA concentration to use in each test, and different isolates should be tested at the same DNA concentration in order to make an accurate comparison of their threshold cycles (Ct). The threshold cycle (Ct) is defined as the PCR cycle at which a fluorescence signal passes a preset value (threshold). The concentration of DNA stock solutions was first determined using a spectrophotomer, reading DNA absorbance at 260 nm. Serial dilutions of genomic DNA from one *Bacteroides stercoris* isolate were used as templates for real-time PCR. Fig. 3 shows the relative fluorescence obtained for each PCR cycles and for each DNA concentration tested. The fastest Ct was obtained with the highest DNA amount used (50 ng). However, a concentration of 10 ng represents a suitable compromise between optimizing Ct and conserving DNA since the Ct had a similar value (approximately 18) to the Ct obtained with DNA tested at 25 and 50 ng. Furthermore, the slope of the curves was also similar. It thus may be preferred to test the DNA samples at about 10 ng per reaction.

### EXAMPLE 11

### Correlation Between the Identification Obtained by Phenotypic Testing with Identification Based on 16S rDNA Sequence

Each of 1,241 16S sequences has been subjected to a search for homologous sequences in the GenBank database using the BLAST algorithm. In order to determine if the phenotypic identification was supported by the 16S rDNA sequence information, the species represented by the first ten BLAST hits were compared to the species name assigned to the samples as a result of phenotypic characterization. The following categories: CORRECT TYPE, PROBABLY CORRECT, CLOSE MATCH, MISTYPED, POSSIBLE MISTYPE, NOVEL SEQUENCE, and VAGUE SAMPLE NAME were defined based on the presence of 16S rDNA sequences for the expected species in GenBank and/or RDP and the Blast hit position obtained for this given species (Table 16A).

**TABLE 16A**

| **TYPING** | **Percent of isolates** | **GenBank and RDP hits** |
|---|---|---|
| CORRECT TYPE | 69 | Hit # 1, 2, or 3 in GenBank and/or RDP |
| PROBABLY CORRECT | 7 | No hit in top 10 in one database but hit #1 to #4 in the other database |
| CLOSE MATCH | 4 | Hit # 4 to # 10 in GenBank and RDP. |
| MISTYPED | 10 | No hit in top 10 in GenBank and RDP, but species represented in both databases. |
| POSSIBLE MISTYPE | 4 | No hit in top 10 in GenBank and/or RDP, but species represented in GenBank or RDP. |
| NOVEL SEQUENCE | 4 | No hit in top 10 in GenBank and RDP because of "novel sequences". |
| VAGUE SAMPLE NAME | 2 | Unclear name |

BLAST result hits 4 to 10 were further investigated to determine whether or not a clear assignment can be made from the additional information obtained.

Only 69% of the isolates had a 16S sequence that gave the best sequence homology with the 16S sequence of the expected species. In other words, the identification of the isolate by 16S rDNA sequencing agreed with the identification results obtained by phenotyping for about two-thirds of all the isolates tested. For as many as 31 % of the isolates, the species name defined by phenotypic methods did not match the information obtained using a BLAST analysis, at least at the level of the top three hits obtained.

There are several possible explanations for these findings including (1) the species not represented in public databases, (2) sequencing errors, (3) some 16S sequences are too short in the public databases, (4) nomenclature changes not integrated, (5) closely related species not differentiated by 16S rDNA, (6) species not claimed as identifiable with the phenotypic identification products used, (7) unclear identification based on phenotypic analysis, and/or (8) wrong phenotypic identification.

These results underscore the need to check a reference database for accuracy. These results also emphasize the need to develop rapid diagnostic tests based on molecular method in complement and/or in replacement of some phenotypic tests in order to improve the diagnostic of bacterial infections and orient towards an appropriate and efficient antibiotherapy.

### EXAMPLE 12

### An Apibio Low Density Chip

The 16S rDNA probes described in this patent can serve as the basis for the design of research and commercial kits for bacterial identification. The probes are suitable to be tested in low-density microarray systems, such as those developed by Apibio (Grenoble, France). Several probes (up to 1,000) can be spotted on a solid substrate. The substrate or support actually contains several wells and several probes (up to 16) can be spotted in each well of the support. Depending on the number of probes specific to a particular species, one well can be dedicated to a particular bacterial species. Biotinylated PCR products will be prepared for each of the bacterial strains to be identified. The resulting PCR products will then be hybridized to the species-specific oligonucleotide (SSO) probes in each well of the solid support. The probe-target hybridization will be evaluated by a streptavidin-coupled detection with colorimetry or fluorescence. A positive signal is expected when the DNA target tested and the probes spotted on the support belong to the same species. Negative and positive controls preferably are both adhered or spotted on each DNA chip or chip substrate.

### EXAMPLE 13

### Use of Microarray for the detection of Streptococcus agalactiae

The 16S rDNA probes described in this application can be used on a high-density DNA chip system, such as GeneChip® (Affymetrix). Since the length of probes, the Tₘ and secondary structures of probes are important for preparing a GeneChip® array, the probes can be derived from 20-mer, 30-mer and species-specific regions (SSR), such as those disclosed herein. The species-specific oligonucleotides (SSOs) and group-specific oligonucleotides (GSOs) covering clinically relevant species were used to build a GeneChip® (Affymetrix) chip. One of these species was *Streptococcus agalactiae,* which is responsible for severe infections in neonates. For a given species-specific oligonucleotide or group-specific oligonucleotide, the original 20-mer sequence and four 17-mer probes derived from the 20-mer were included onto the chip. Each probe called a perfect match (PM) cell was associated to another probe called a mismatch (MM) cell that differs from the PM by just one nucleotide (probe substitution position). The pair of PM and MM cells defines an atom on the chip. For *Streptococcus agalactiae* species, twenty 20-mer species-specific oligonucleotides (SEQ ID NOS: 274, 393, 665, 703, 1076, 1173, 1409, 1508, 1576, 1614, 1838, 2199, 2213, 2541, 2839, 2857, 2914, 3078, 3157, and 3232) and thirty-two 20-mer group-specific oligonucleotides (SEQ ID NOS: 28304, 28306, 29325, 29448, 29632, 30338, 30633, 32212, 33065, 33324, 33370, 34524, 34870, 35688, 35949, 36303, 36583, 36647, 37293, 37551, 39226, 39998, 41902, 42485, 42884, 43386, 43488, 43620, 44569, 45263, 45776, 46945, 47112, and 47975) and their 17-mer derivatives were used on the chip.

The assay was conducted as follows. A *Streptococcus agalactiae* isolate was cultured on a blood agar plate overnight. After culture, cells (600 µl of a 0.5 McFarland suspension) were mechanically lysed with beads. Cell lysate (2 µL) was used to amplify 16S rDNA gene by PCR. Each 50 µL reaction mix contained 300 nM forward primer (5'-AGA GTT TGA TCA TGG CTC AG-3'), 300 nM reverse primer (5'-GAA GGA GGT GAT CCA ACC GCA-3'), 1X PCR buffer (Expand High Fidelity Buffer Roche) that contained 1.5 mM MgCl₂, a mix of 200 nM dNTP, 1.75 unit of Taq DNA polymerase (Expand High Fidelity Taq DNA Polymerase, Roche), and 2 µL of DNA lysate. The 16S rDNA amplified fragment with a size of about 1,500 bp was then labeled with a biotinylated compound (bioPMDAM, bioMerieux), and cleaved with 0.1N HCl in order to obtain small fragments *(i.e.,* less than 100 bp) to hybridize with the probes on the chip.

Labeled and cleaved PCR samples were then hybridized on the Affymetrix chip at 45°C for 30 min in a hybridization buffer containing 7X saline-sodium phosphate-EDTA (SSPE), 3 M betaine, 0.01 M dodecyltri-methylammonium bromide (DTAB), and antifoam. Hybridized chips were then washed in 4X SSPE buffer containing 0.01 % Triton, followed by washes in 6X SSPE buffer with 0.01% Triton. The hybridized chip was then stained with Streptavidine-phycoerythrin (DAKO) and laser scanned at 570 nm on a scanner (Hewlet Packard). The scan was recorded as a pixel image.

Data were analyzed using a bioMerieux software similar to GeneChip Analysis Suite, version 3.3 (Affymetrix). Average background noise was substracted from all the cells. A probe was scored as positive when 1) the intensity of the hybridization signal of the perfectly matched probe cell (PM) was greater than or equal to 1.2 times the intensity of the mismatch control cell (MM); and 2) the intensity of the PM was 8 times higher than the noise value. Data analysis was based on the comparaison of the hybridization signals. The full length 16S rDNA sequences of the species represented on the chip are stored into a reference database. Since each probe on the chip is derived from one 16S rDNA sequence of this database, it can easily be matched to the relevant 16S rDNA sequence and species.

In the present example, the reference database used contained 40 full-length 16S rDNAs sequences from 20 species *(i.e., Burkholderia cepacia, Burkholderia gladioli, Burkholderia multivorans, Cardiobacterium hominis, Enterobacter sakazakii, Enterococcus faecalis, Gemella morbillorum, Granulicatella adiacens, Kocuria rosea, Mannheimia haemolytica, Pantoea agglomerans, Proteus penneri, Providencia stuartii, Pseudomonas aeruginosa, Ralstonia pickettii, Sphingobacterium multivorum, Staphylococcus chromogenes, Staphylococcus simulans, Streptococcus agalactiae,* and *Vibrio cholerae).* All the positive hybridization signals of the test samples are then correlated to species that contain the probes on the chip with the highest sequence homology. These sequences are full length 16S rDNA sequences without SEQ ID NO and are included in Table 16B. Each species is represented on the chip by its different species-specific and group-specific oligonucleotides. Since some species have a higher number of species-specific oligonucleotides than others and some species lack group-specific oligonucleotides, the number of nucleotide positions tested on the chip varies from one species to species. Below is the percent homology ranking obtained for the type strain of *Streptococcus agalactiae* (ATCC 13813T).

**TABLE 16B**

| Species | % Homology | probe* |
|---|---|---|
| *Streptococcus agalactiae* | 100 | SSO |
| *Streptococcus agalactiae* | 94.29 | GSO+SSO |
| *Streptococcus agalactiae* | 87.50 | GSO |
| *Burkholderia cepacia* | 84.62 | SSO |
| *Burkholderia cepacia* | 84.62 | GSO+SSO |
| *Ralstonia pickettii* | 80.00 | SSO |
| *Ralstonia pickettii* | 80.00 | GSO+SSO |
| *Staphylococcus chromogenes* | 70.00 | SSO |
| *Staphylococcus chromogenes* | 70.00 | GSO+SSO |
| *Mannheimia haemolytica* | 66.67 | SSO |
| *Mannheimia haemolytica* | 66.67 | GSO+SSO |
| *Sphingobacterium multivorum* | 52.63 | SSO |
| *Granulicatella adiacens* | 50.00 | GSO |
| *Sphingobacterium multivorum* | 50.00 | GSO |
| *Staphylococcus simulans* | 49.32 | SSO |
| *Staphylococcus simulans* | 49.32 | GSO+SSO |
| *Burkholderia gladioli* | 47.83 | GSO |
| *Sphingobacterium multivorum* | 47.37 | GSO+SSO |
| *Kocuria rosea* | 47.19 | SSO |
| *Kocuria rosea* | 47.19 | GSO+SSO |
| *Enterococcus faecalis* | 43.68 | SSO |
| *Enterococcus faecalis* | 43.68 | GSO+SSO |
| *Enterobacter sakazakii* | 42.86 | SSO |
| *Gemella morbillorum* | 42.17 | GSO |
| *Burkholderia gladioli* | 41.76 | GSO+SSO |
| *Gemella morbillorum* | 41.71 | GSO+SSO |
| *Proteus penneri* | 41.67 | SSO |
| *Granulicatella adiacens* | 40.96 | GSO+SSO |
| *Granulicatella adiacens* | 39.44 | SSO |
| *Proteus penneri* | 39.13 | GSO |
| *Gemella morbillorum* | 38.89 | SSO |
| *Cardiobacterium hominis* | 38.24 | SSO |
| *Vibrio cholerae* | 37.21 | GSO |
| *Burkholderia gladioli* | 36.73 | SSO |
| *Proteus penneri* | 36.36 | GSO+SSO |
| *Providencia stuartii* | 35.71 | SSO |
| *Vibrio cholerae* | 35.51 | GSO+SSO |
| *Pantoea agglomerans* | 35.29 | SSO |
| *Cardiobacterium hominis* | 31.71 | GSO+SSO |
| *Pantoea agglomerans* | 28.00 | GSO+SSO |
| *Enterobacter sakazakii* | 25.00 | GSO+SSO |
| *Pantoea agglomerans* | 25.00 | GSO |
| *Providencia stuartii* | 22.22 | GSO+SSO |
| *Vibrio cholerae* | 11.11 | SSO |
| *Enterobacter sakazakii* | 11.11 | GSO |
| *Kocuria rosea* | 11.11 | GSO |
| *Burkholderia multivorans* | 0.00 | GSO+SSO |
| *Burkholderia multivorans* | 0.00 | GSO |
| *Cardiobacterium hominis* | 0.00 | GSO |
| *Providencia stuartii* | 0.00 | GSO |

| | | |
|---|---|---|
| *"SSO": species-specific oligonucleotide; "GSO": group-specific oligonucleotide | | |

Species-specific probes and group-specific probes were analyzed separately as well as in combination. In all cases, the best homology score was obtained for *Streptococcus agalactiae.* These results confirm that the 52 probes cited above and specific to *Streptococcus agalactiae* or to a group of species including *S. agalactiae* can be used on a microarray for the detection of *Streptococcus agalactiae* in a sample. This methodology can be applied to a large range of species. Such microarrays can be used to diagnose the cause of a bacterial infection.

### EXAMPLE 14

### Verification of the Species Specificity and Robustness of Species-Specific Oligonucleotides by Light Cycler Assay

The LightCycler method is a real-time PCR method. It uses two different species-specific oligonucleotide (SSO) probes that are labeled with different dyes. These two hybridization probes hybridize to their complementary DNA template in a head-to-tail arrangement; *i.e.*, the two probes are just separated by one or two nucleotides. Because of this close proximity and after excitation by a blue light source, the donor dye (fluorescein) located on the first probe transfers its energy to the acceptor dye (Red640) located on the second probe. The transfer of energy causes fluorescent light to be emitted. When the probes cannot find their complementary sequence on the DNA template, no hybridization occurs such that no fluorescent signal is produced. The intensity of the fluorescent signal is proportional to the amount of PCR product generated during the cycle. Similar to the TaqMan® assay, a cycle threshold (Ct) is obtained.

In Example 13, the 16S rDNA sequence of *Staphylococus simulans* strains was used to select two species-specific hybridiztion probes spaced by 1 nucleotide. At the same time, two non-species-specific PCR primers were designed as controls and used in a LightCycler assay with the two hybridization probes. Sequences of these probes and primers are presented in Table 17.

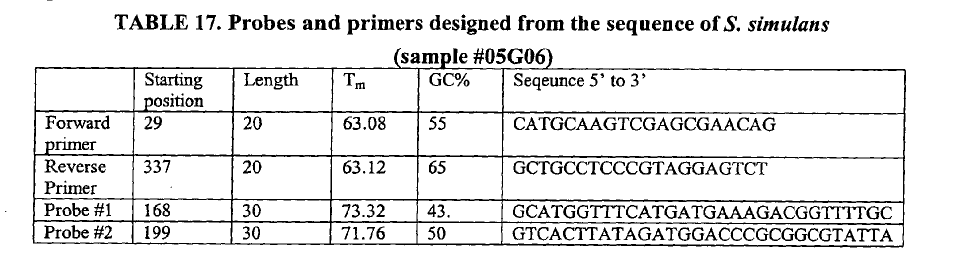

Real-time PCR data obtained with this combination of probes and primers on different *Staphylococcal* species are presented in Figure 7. The results clearly show that only strains belonging to *Staphylococcus simulans* species gave a positive signal with a Ct of around 16.

### EXAMPLE 15

### Verification of the Species Specificity of Species-Specific Oligonucleotides by Pyrosequencing

The pyrosequencing method is a simple and easy-to-use sequencing method by synthesis. This method uses a mixture of four enzymes and specific substrates. At first, a sequencing primer is hybridized to a single-stranded PCR product (*e.g.*, DNA template). With an enzyme cascade system, light is produced whenever a nucleotide is incorporated and forms a base pair with a complementary nucleotide located on the DNA template (as discussed in Example 13 above). Each peak represents the incorporation of the nucleotide mentioned on the X-axis during the sequencing reaction. The size of the peak determines the number of nucleotides incorporated. The software also gives a schematic representation of the pyrogram for each possibility of the single nucleotide polymorphism (SNP).

In this example, six species *of Enterococcus (i.e., Enterococccus faecalis, E. durans, E. faecium, E. avium, E. gallinarum,* and *E. saccharolyticus)* were selected to determine if pyrosequencing was a suitable method to validate (at the experimental level) the species specificity of some SSOs (signature sequences). A multiple sequence alignment of 16S rDNA sequences of the six different species of Enteroccci was performed using ClustalX and GeneDoc softwares. Variable regions that contained SSOs were identified. Variation at one nucleotide position between the different species defines a distinguishing moiety also known as a single nucleotide polymorphism (SNP). Four SNP positions were selected to test the pyrosequencing method.

PCR primers and sequencing primers were designed to amplify and sequence the 16S rDNA areas that contain a SNP. The size of PCR product should not exceed 500 bp in order to minimize the risk of mispriming for the sequencing primer. The pyrosequencing primer needs to be localized just upstream of the SNP site as the maximum length of the sequence obtained by pyrosequencing is about 40-50 bases. Pyrosequencing was done on a Pyrosequencer using the SNP detection software.
Two isolates per species for the six different species of Enterococci were tested. An example of pyrogram is presented in Fig. 8. Table 18 illustrates the results obtained for 4 SNP for 6 different species of Enterococci.

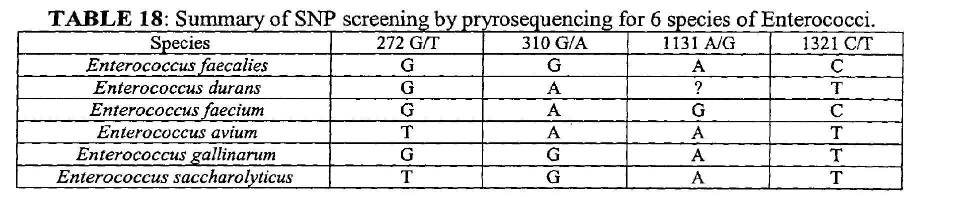

Interestingly, by testing only 4 SNPs, it was possible to differentiate six species of Enterococci. These results underscore the high potential of pyrosequencing for bacterial typing and rapid evaluation of signature sequences, provided that adequate PCR and sequencing primers are designed and used for the group of species to screen.

### EXAMPLE 16

### Identification of nucleotide positions comprising "distinguishing moieties"

To identify nucleotides that are critical for distinguishing species (*i.e.*, distinguishing moieties), comparisons with the GenBank sequence database were made using BLAST for each of the 28,070 Species Specific Oligonucleotides. For each SSO, the top GenBank BLAST hits were compared with the SSO sequence, and the number of variations at each nucleotide position counted. The higher the count at any position of the 30-mer (*e.g.*, Position 1 being represented by the column entitled "pos _01"; Postion 2 being represented in the column entitled "pos_02" and so forth), the more critical th distinguishing moiety is in discriminating other species. These counts are represented in Table 19, which is attached to the specification as a separate document. For certain sequences, only a 20-mer was used and analyzed, consequently no information is provided for certain sequences at postions 21-30.

The results acquired through this method may change over time due to the entry of new sequence information into the public domain. Thus, one skilled in the art will recognize that the assignment of robustness of distinguishing moieties is a process that should be updated periodically by methods such as the one described here. Even if the status of some of the distinguishing moieties described here changes from species-specific to genus-specific or group-specific, a sufficient number of probes containing distinguishing moieties are provided to maintain the diagnostic utility of the invention.

### EXAMPLE 17

### Further Analysis of the SSOs and GSOs

The data from Example 4 was analyzed further in view of additional sequence information released to the public. Sequences from a total of 567 different bacterial species were examined. No sequence could be obtained for 7 isolates representing 4 species: *Corynebacterium minutissimum* (2 isolates), *Kocuria kristinae* (1 isolate), *Micrococcus lylae* (3 isolates), and *Sphingobacterium spiritivorum* (1 isolate). The 16 S loci of these 7 isolates could not be amplified using the 16S universial primers as discussed above.

From the collection of 1,325 16S rDNA sequences, 2,724 30-mer SSOs, 25,346 20-mer SSOs, and 20,954 20-mer GSOs were identified *in silico* using the methods discussed above. The 30-mer and 20-mer SSos were specific for 325 and 648 different species respectively. At least one SSO was identified for each of 491 of the 567 total species examined. Another 46 species were identified as the species level using a combination of two or more GSOs. Tweny species could only be characterized by using GSOs as belonging to a subset containing multiple sepces. Only 10 species were not identifiable at all using any combination of the SSOs and GSOs *(i. e., Cedecea neteri, Citrobacter gillenii, Citrobacter murliniae, Enterobacter asburiae, Enterobacter intermedius, Kluvera ascorbata, Kluyvera cryocrescens, Moraxella (Branbamella) catarrhalis, Pantoea dispersa,* and *Pseudomonas alcaligenes.).* The SSO and GSO probes developed were capable of discriminating 557 out of the 567 species.

To ensure the elimination of SSOs that were merely strain specific, further comparisons using all sequences for a given species in both public and properiteary 16S databases was performed. A total of 15,127 SSOs were found to be species-specific for 271 species after comparison to the proprietary sequence data and to publicaly available 16S rDNA sequences in GenBank. When the analysis was extended to include partial 16S rDNA sequences in RDP, a total of 9,879 SSOs representing 232 species were species specific. The information based on this further analysis is provided in Tables 20 and 21, which are similar to Tables 14 and 15 respectively.

All references cited herein as well as U.S.S.N. 60/464,955 filed April 24, 2003 are herein incorporated in their entirety for all purposes.

## Claims

1. A plurality of 16S polynucleotides immobilized to a solid support, wherein the plurality of 16S polynucleotides are subsequences of 16S rDNA and each 16S polynucleotide individually comprises at least one distinguishing moiety, which differentiates between microorganisms by genus, group, species, strain, and/or isolate.

2. The plurality of 16S polynucleotides of Claim 1, wherein the 16S polynucleotide is an oligomer of 11 to 45 nucleotides.

3. The plurality of 16S polynucleotides of Claim 2, wherein the 16S polynucleotide is an oligomer of 15, 16, 17,18, 19, 20, 21, 22, 23, 24, 25, 26, 27, 28, 29, or 30 nucleotides.

4. The plurality of 16S polynucleotides of Claim 3, wherein the solid support is a bead, slide, chip, microtube, or plate.

5. The plurality of 16S polynucleotides of Claim 4, wherein the bead is glass or plastic.

6. The plurality of 16S polynucleotides of Claim 1, wherein at least 5 to 1x10⁶ 16S polynucleotides are immobilized to the solid support.

7. The plurality of 16S polynucleotides of Claim 1, wherein at least 5 to 100 16S polynucleotides are immobilized to the solid support.

8. A method of detecting the presence of a microorganism and determining an isolate, a strain, a species, a group, or a genus of a microorganism in a sample suspected of containing the microorganism comprising the steps of:
(A) selecting at least one primer pair to amplify at least a portion of a 16S rDNA of the sample;
(B) amplifying the 16S rDNA of the sample with the at least one primer pair;
(C) contacting the amplified rDNA with at least one isolated nucleic acid comprising at least one distinguishing moiety;
(D) incubating the amplified rDNA and the isolated nucleic acid under hybridizing conditions which allow hybridization in a sequence-specific manner between the sample and the at least one isolated nucleic acid to form a hybridization product;
(E) detecting the hybridization product and thereby one or more distinguishing moieties of the microorganism; and
(F) determining the isolate, strain, species, group, and/or genus of the microorganism by the presence of the one or more distinguishing moieties.

9. The method of Claim 8, wherein the sample is a food, a biological sample taken from a subject, an environmental sample, or a plant.

10. The method of Claim 9, wherein the subject is an agricultural animal or a mammal.

11. A kit for the detection and identification of at least one microorganism by genus, group, species, strain, and/or isolate in a sample comprising:
(A) at least one primer pair for amplification of at least a portion of a 16S rRNA of the microorganism;
(B) two or more nucleic acids comprising at least two critical residues of a 16S rDNA which distinguish the microorganism by genus, group, species, strain, or isolate;
(C) a hybridization buffer to allow sequence-specific hybridization between the probes and the nucleic acids present in the sample, or to allow sequence-specific hybridization between the probes and the nucleic acids of amplified products of the sample; and
(D) a detection moiety.

12. The kit of Claim 11, wherein the kit further comprises a detection means, instructions for use of the kit, a wash buffer, and/or a hybridization buffer or any combination thereof.

13. The kit of Claim 11, wherein the sample is a plant sample, an environmental sample, a food, or a biological sample obtained from a subject.

14. The kit of Claim 11, wherein the 16S rDNA distinguishing moiety is a distinguishing moiety of Table 20 and/or Table 21.

15. A composition comprising a plurality of probes of Table 20 and/or Table 21, wherein each probe comprises at least one distinguishing moiety, and wherein the plurality of probes are immobilized on a substrate.

16. The composition of Claim 15, wherein the substrate is a bead, a microarray plate, or a microarray slide.

17. The composition of Claim 16, wherein the bead is glass or plastic.

18. The composition of Claim 16, wherein the composition comprises a plurality of beads forming a matrix and the matrix is in the form of an affinity column.

19. The composition of Claim 15, wherein the plurality of distinguishing moieties are genus specific, group specific, species specific, strain specific, isolate specific, or any combination thereof.

20. The composition of Claim 15 further comprising a linker bound to each probe.

21. The composition of Claim 15, wherein the plurality of probes are individually at least 15 nucleotides to 30 nucleotides.

22. A database comprising a plurality of distinguishing moieties which differentiate a microorganism by genus, group, species, strain, or isolate, and wherein the database comprises at least two distinguishing moieties from Tables 2, 20, or 21.

23. The database of Claim 22, wherein the database further differentiates the microorganism by genus and species.

24. A reference database comprising a plurality of distinguishing moieties, wherein the distinguishing moieties include distinguishing moieties of Table 20 and/or Table 21 in relational form with a means for querying the reference database.

25. A computerized storage and retrieval system of critical residues comprising: a data entry means; a display means; a programmable central processing unit; and a data storage means having 16S rRNA distinguishing moieties and annotated information on attributes of the 16S rRNA distinguishing moieties electronically stored in a relational database.

26. The computerized storage and retrieval system of Claim 25, wherein the stored 16S rRNA critical residues are selected from Tables 2, 20, and 21.

27. A method of identifying distinguishing moieties in a 16S bacterial rRNA or rDNA comprising the steps of:
(A) obtaining a nucleotide sequence of a genetic locus shared by two or more different bacterial strains, species, or genera;
(B) dividing the nucleotide sequence into a set of oligomers of length "n" which overlap by "x" nucleotides, wherein "x" is at least one nucleotide less than "n" and wherein said overlapping oligomers span the length of the sequence of the genetic locus;
(C) comparing an oligomer using a comparative algorithm against at least one database of nucleotide sequences for that locus from a plurality of bacterial strains, species, or genera, wherein the nucleotide sequences are stored in at least one database; and
(D) determining whether the oligomer has a nucleotide sequence which matches, or has no more than one mismatch with, a portion of all available nucleotide sequences for the locus of the strain, species, or genus of origin, or whether the nucleotide sequence has at least two mismatches when aligned with any other strain, species, or genus, wherein the at least two mismatches when aligned correspond to distinguishing moieties which differentiate between strain, species or genus.

28. A method of diagnosing a subject and determining the microorganism causing an infection in the subject comprising the steps of:
(A) obtaining a sample from the subject; and
(B) screening the sample for the microorganism using the kit of Claim 11.
